# EUROPEAN PATENT SPECIFICATION

(11) **EP 4 181 920 B1**
(45) Date of publication and mention of the grant of the patent: **10.09.2025**
(21) Application number: 21749550.6
(22) Date of filing: 15.07.2021
(51) Int. Cl.: A61K 31/4196, A61K 31/423, A61K 31/506, A61K 31/519, A61K 31/565, A61K 31/7064, A61K 31/18, A61K 45/06, A61P 35/00

(54) **KAT6 INHIBITOR AND COMBINATIONS FOR BREAST CANCER TREATMENT**
KAT6-INHIBITOR UND KOMBINATIONEN ZUR BRUSTKREBSBEHANDLUNG
INHIBITEURS DE KAT6 ET COMBINAISONS POUR LE TRAITEMENT DU CANCER DU SEIN

(30) Priority: 15.07.2020 US 202063052215 P; 16.06.2021 US 202163211044 P
(43) Date of publication of application: 24.05.2023
(73) Proprietor: Pfizer Inc., New York, NY 10001-2192 (US); CTXT PTY LTD, Melbourne, VIC 3000 (AU)
(72) Inventor: STUPPLE, Paul Anthony, Melbourne, Victoria 3000 (AU); MAZUREK, Anthony, Flemington, NJ 08822 (US); ARNDT, Kim Timothy, Chesterfield, MO 63017 (US); CHEN, Lei, NY 10530 (US); FOLLETTIE, Maximillian Todd, Stoneham, MA 02180 (US); FRUHLING, David Scott, NY 10924 (US); KUNG, Pei-Pei, San Diego, CA 92130 (US); ZHONG, Wenyan, NY 10956 (US); TEDESCHI, Philip Michael, Gladstone, NJ 07934 (US)
(74) Representative: Pfizer
(86) International application number: PCT/EP2021/069787
(87) International publication number: WO 2022/013369

(56) References cited:
- WO-A1-2016/198507
- WO-A1-2019/207463
- WO-A1-2020/216701
- WO-A1-2020/254946
- YU L ET AL: "Identification of MYST3 as a novel epigenetic activator of ER[alpha] frequently amplified in breast cancer", ONCOGENE, vol. 36, no. 20, 1 May 2017 (2017-05-01), London, pages 2910 - 2918, XP055855359, ISSN: 0950-9232, Retrieved from the Internet <URL:https://www.ncbi.nlm.nih.gov/pmc/articles/PMC5436938/pdf/onc2016433a.pdf> DOI: 10.1038/onc.2016.433
- BAELL JONATHAN B ET AL: "Inhibitors of histone acetyltransferases KAT6A/B induce senescence and arrest tumour growth", NATURE, MACMILLAN JOURNALS LTD., ETC, LONDON, vol. 560, no. 7717, 1 August 2018 (2018-08-01), pages 253 - 257, XP036563466, ISSN: 0028-0836, [retrieved on 20180801], DOI: 10.1038/S41586-018-0387-5
- PRIEBBENOW DANIEL L. ET AL: "Discovery of Acylsulfonohydrazide-Derived Inhibitors of the Lysine Acetyltransferase, KAT6A, as Potent Senescence-Inducing Anti-Cancer Agents", JOURNAL OF MEDICINAL CHEMISTRY, vol. 63, no. 9, 14 May 2020 (2020-05-14), US, pages 4655 - 4684, XP055854966, ISSN: 0022-2623, Retrieved from the Internet <URL:https://pubs.acs.org/doi/pdf/10.1021/acs.jmedchem.9b02071> DOI: 10.1021/acs.jmedchem.9b02071
- KIM ESTHER S ET AL: "Palbociclib: A Review in HR-Positive, HER2-Negative, Advanced or Metastatic Breast Cancer", TARGETED ONCOLOGY, SPRINGER PARIS, PARIS, vol. 12, no. 3, 9 May 2017 (2017-05-09), pages 373 - 383, XP036247517, ISSN: 1776-2596, [retrieved on 20170509], DOI: 10.1007/S11523-017-0492-7
- COSTEL CHIRILA ET AL: "Comparison of palbociclib in combination with letrozole or fulvestrant with endocrine therapies for advanced/metastatic breast cancer: network meta-analysis", CURRENT MEDICAL RESEARCH AND OPINION, 16 May 2017 (2017-05-16), GB, pages 1 - 10, XP055375435, ISSN: 0300-7995, DOI: 10.1080/03007995.2017.1325730
- BEN O'LEARY ET AL: "Treating cancer with selective CDK4/6 inhibitors", NATURE REVIEWS CLINICAL ONCOLOGY, VL. 13, N. 7, 31 March 2016 (2016-03-31), pages 417 - 430, XP055581045, Retrieved from the Internet <URL:https://www.nature.com/articles/nrclinonc.2016.26.pdf> [retrieved on 20190415], DOI: 10.1038/nrclinonc.2016.26
- SHARMA SHIKHAR ET AL: "Abstract 1130: First-in-class KAT6A/KAT6B inhibitor CTx-648 (PF-9363) demonstrates potent anti-tumor activity in ER+ breast cancer with KAT6A dysregulation", 1 July 2021 (2021-07-01), XP055854862, Retrieved from the Internet <URL:https://cancerres.aacrjournals.org/content/81/13_Supplement/1130> [retrieved on 20211026]
- ANONYMOUS: "Study of PF-07248144 in Advanced or Metastatic Solid Tumors", 28 October 2020 (2020-10-28), XP055854636, Retrieved from the Internet <URL:https://clinicaltrials.gov/ct2/show/NCT04606446> [retrieved on 20211025]

## Description

### Field of the Invention

The present invention relates to a KAT6 inhibitor for use in a method of treating breast cancer, in combination with a CDK4 inhibitor and/or an antiestrogen. Furthermore, this invention relates to a KAT6 inhibitor as a single agent or in combination for use in a method of overcoming clinical resistance to endocrine therapy, wherein the endocrine therapy is used for treating breast cancer. Pharmaceutical uses of the methods and combinations of the present invention are also described.

### Background

Histone acetylation is a reversible protein modification essential for chromatin organization and function (Lee, K. K., et al., Histone acetyltransferase complexes: one size doesn't fit all. Nat. Rev. Mol. Cell Biol. 2007, 8, 284-295). The acetylation of histones is catalyzed by histone lysine acetyltransferases (HATs or KATs) using acetyl-CoA (AcCoA) as the co-factor (Roth, S. Y., et al., Histone acetyltransferases. Annu. Rev. Biochem. 2001, 70, 81-120; Furdas, S. D., et al., Small molecule inhibitors of histone acetyltransferases as epigenetic tools and drug candidates. Arch. Pharm. 2012, 345, 7-21). Lysine acetyltransferase 6A (KAT6A, also known as MOZ or MYST3) and lysine acetyltransferase 6B (KAT6B, also known as MORF) are paralog genes whose protein products form a complex with ING5, EAF6, and BRPF1, -2, or -3 to acetylate H3K23Ac (Doyon, Y., et al., ING tumor suppressor proteins are critical regulators of chromatin acetylation required for genome expression and perpetuation. Mol. Cell. 2006, 21(1):51-64; Mullah, M., et al., Molecular architexture of quartet MOZ/MORF histone acetyltransferase complexes. Mol. Cell. Biol. 2008, 28(22): 6828-6843). KAT6A and KAT6B acetyltransferase function is involved in fundamental cellular processes, including gene transcription, cellular senescence, tissue development, and maintenance of normal hematopoietic stem cells (Huang, F., et al., Regulation of KAT6 acetyltransferase and their roles in cell cycle progression, stem cell maintenance, and human disease. Mol. Cell. Biol. 2016, 36, 1900-1907).

KAT enzymes perform important regulatory functions in cancer and are therefore frequently targeted by mutations, translocations, and amplifications (Hu, Z., et al., Genomic characterization of genes encoding histone acetylation modulator proteins identifies therapeutic targets for cancer treatment. Nat Commun. 2019 Feb 13;10(1):733). KAT6A was identified in 1996 as part of a chromosomal translocation t(8;16)(p11;p13) with CREBBP (CREB-binding protein) in a subtype of acute myeloid leukemia (Borrow, J., et al, The translocation t(8; 16)(p11;p13) of acute myeloid leukaemia fuses a putative acetyltransferase to the CREB-binding protein. Nat. Genet. 1996, 14, 33-41). Additional KAT6A and KAT6B translocations were subsequently identified in more AML patients, generating fusions with other HATs such as EP300 (adenoviral EIA-associated protein p300), NCOA2 (nuclear receptor coactivator 2), and NCOA3 (Huang, et al.).

In human carcinomas, especially breast cancer, KAT6A was identified as a part of the recurrently amplified region of 8p11-12 found in 10-15% of breast cancers (Adélaïde J., et al, Chromosome region 8p11-p21: refined mapping and molecular alterations in breast cancer. Genes Chromosomes Cancer. 1998 Jul;22(3):186-99). Breast cancer cell lines harboring KAT6A amplifications over-express KAT6A, implicating KAT6A as a putative breast cancer susceptibility gene. Turner-Ivey et al. utilized a genome-scale shRNA screening strategy to identify KAT6A as a significant dependency in 8p11 amplified breast cancer cell lines harboring overexpression of KAT6A (Turner-Ivey B, et al., KAT6A, a chromatin modifier from the 8p11-p12 amplicon is a candidate oncogene in luminal breast cancer. Neoplasia. 2014 Aug;16(8):644-55). Yu et al. subsequently demonstrated in 8p11 amplified breast cancer cells that KAT6A localized to the estrogen receptor promoter and that shRNA-mediated knockdown of KAT6A reduced ESR1 mRNA and protein levels of ERα (Yu, L., et al., Identification of MYST3 as a novel epigenetic activator of ERα frequently amplified in breast cancer. Oncogene 2017, 36, 2910-2918). Moreover, they showed that the growth defect resulting from KAT6A depletion in 8p11 amplified breast cancer cells was partially rescued by re-expression of *ESR1.* These findings indicate an important role of KAT6A in gene regulation of ERα required for growth of ER+ breast cancer cells.

Chromosome 8p11-12 amplifications and KAT6A over-expression exists in additional tumor types including ovarian cancer, uterine cervix cancer, lung adenocarcinoma, colon and rectal adenocarcinomas, and medulloblastoma (Zack TI, et al., Pan-cancer patterns of somatic copy number alteration. Nat Genet 2013 45:1134-1140; Northcott PA, et al., Multiple recurrent genetic events converge on control of histone lysine methylation in medulloblastoma. Nat Genet 2009 41:465-472). Additional KAT6A tumor dependencies including prostate cancer have been identified (Yu C, et al., High-throughput identification of genotype-specific cancer vulnerabilities in mixtures of barcoded tumor cell lines. Nat Biotechnol. 2016; Meyers RM, et al. Computational correction of copy number effect improves specificity of CRISPR-Cas9 essentiality screens in cancer cells. Nat Genet. 2017; and Tsherniak A, et al., Defining a cancer dependency map. Cell. 2017). Overall, these data demonstrate the broader therapeutic opportunity for targeting KAT6A in additional tumor types.

In addition to its catalytic function mediated by the histone acetyltransferase (HAT) domain the KAT6A protein includes additional domains such as PHD domains, an acidic domain, and a serine/methionine-rich domain. KAT6A regulation of gene expression independent of its catalytic activity has been reported (Kitabayashi, I., et al., Activation of AML1 mediated transcription by MOZ and inhibition by the MOZ-CBP fusion protein. EMBO J. 2001, 20(24): 7184-7196). The dependence of ER+ breast cancer cells on KAT6A was demonstrated using RNA interference to knockdown KAT6A protein level (Turner-Ivey B., et al. and Yu, L., et al.) However, the requirement of KAT6A catalytic activity for ERα expression and ER+ breast cancer cell proliferation is unclear. Two advanced KAT6A/6B inhibitors were reported in the literature by researchers at Monash University to effectively kill MYC-driven lymphoma cells, suggesting that KAT6A/6B enzymatic activity is required for proliferation of such cancers (Kitabayashi, I., et al). However, these two reported KAT6A/6B inhibitors were found to have high human liver microsome clearance, strong DDI potential, and acid instability due to a key pharmacophore (acyl sulfonyl hydrazide moiety) present in their chemical structures. To date, no KAT6A/6B enzymatic inhibitors are reported in the literature to be effective in treating solid tumors including HR+ breast cancers and AR+ prostate cancers. There are also no known KAT6A/6B catalytic inhibitors currently being tested in human clinical trials.

The compound, N'-(4-fluoro-5-methyl-[1,1'-biphenyl]-3-carbonyl)benzenesulfonohydrazide (referred to herein as "COMPOUND A"), is a inhibitor of KAT6A, having the structure:

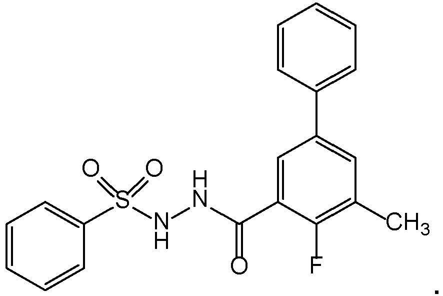

COMPOUND A and pharmaceutically acceptable salts thereof are disclosed in International Publication No. WO 2016/198507.

The compound, N'-(5-chloro-4-fluoro-[1,1'-biphenyl]-3-carbonyl)benzenesulfonohydrazide (referred to herein as "COMPOUND B"), is an inhibitor of KAT6A, having the structure:

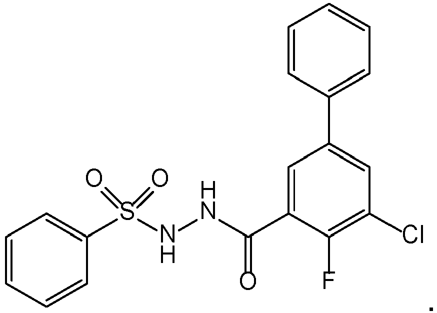

COMPOUND B and pharmaceutically acceptable salts thereof are disclosed in International Publication No. WO 2016/198507.

The compound, 2,6-dimethoxy-*N*-{4-methoxy-6-[(1*H*-pyrazol-1-yl)methyl]-1,2-benzoxazol-3-yl}benzene-1-sulfonamide (referred to herein as "COMPOUND C"), is a potent and selective catalytic inhibitor of KAT6 histone acetyltransferases, KAT6A and KAT6B, having the structure:

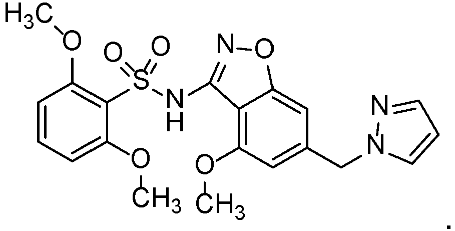

COMPOUND C and pharmaceutically acceptable salts thereof are disclosed in U.S. patent application serial number 16/902,515.

The compound, 2-fluoro-N'-(3-fluoro-5-(pyridin-2-yl)benzoyl)benzenesulfonohydrazide (referred to herein as "COMPOUND D"), is an inhibitor of KAT6A, having the structure:

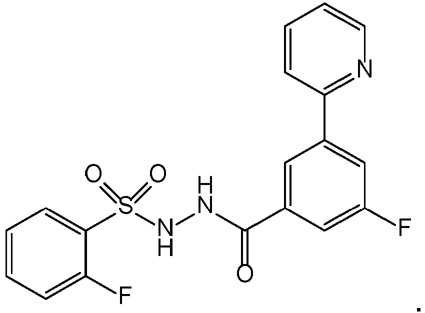

COMPOUND D and pharmaceutically acceptable salts thereof are disclosed in International Publication No. WO 2016/198507.

The compound, 2-methoxy-*N*-{4-methoxy-6-[(1*H*-pyrazol-1-yl)methyl]-1,2-benzoxazol-3-yl}benzene-1-sulfonamide (referred to herein as "COMPOUND E"), is a a potent and selective catalytic inhibitor of KAT6 histone acetyltransferases, KAT6A and KAT6B, having the structure:

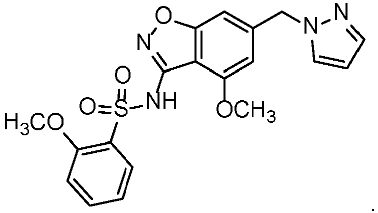

COMPOUND E and pharmaceutically acceptable salts thereof are disclosed in U.S. patent application serial number 16/902,515.

Cyclin-dependent kinases (CDKs) and related serine/threonine protein kinases are important cellular enzymes that perform essential functions in regulating eukaryotic cell division and proliferation. The CDK catalytic units are activated by regulatory subunits known as cyclins. At least sixteen mammalian cyclins have been identified (Johnson DG, Walker CL. Cyclins and Cell Cycle Checkpoints. Annu. Rev. Pharmacol. Toxicol. (1999) 39:295-312). Cyclin B/CDK1, cyclin A/CDK2, cyclin E/CDK2, cyclin D/CDK4, cyclin D/CDK6, and likely other heterodynes are important regulators of cell cycle progression. Additional functions of cyclin/CDK heterodynes include regulation of transcription, DNA repair, differentiation and apoptosis (Morgan DO, Cyclin-dependent kinases: engines, clocks, and microprocessors. Annu. Rev. Cell. Dev. Biol. (1997) 13:261-291).

CDK inhibitors have been demonstrated to be useful in treating cancer. Increased activity or temporally abnormal activation of cyclin-dependent kinases has been shown to result in the development of human tumors, and human tumor development is commonly associated with alterations in either the CDK proteins themselves or their regulators (Cordon-Cardo C. Mutations of cell cycle regulators: biological and clinical implications for human neoplasia. Am. J. Pathol. (1995) 147:545-560; Karp JE, Broder S. Molecular foundations of cancer: new targets for intervention. Nat. Med. (1995) 1:309-320; Hall M, Peters G. Genetic alterations of cyclins, cyclin-dependent kinases, and Cdk inhibitors in human cancer. Adv. Cancer Res. (1996) 68:67-108).

CDK4 and CDK6 are important regulators of cell cycle progression at the G1-S checkpoint, which are controlled by D-type cyclins and INK4 endogenous CDK inhibitors, such as p16^{INK4a} (CDKN2A). Dysregulation of the cyclin D-CDK4/6-INK4-retinoblastoma (Rb) pathway has been reported to be associated with development of endocrine therapy resistance. Furthermore, CDK4 has been identified as the singular oncogenic driver in many breast cancers and emerging data suggest that cyclin D3-CDK6 inhibition may be linked to hematologic toxicity, suggesting a role for CDK4 selective inhibitors.

Clinical trials for the CDK4/6 inhibitors palbociclib, ribociclib and abemaciclib are ongoing for breast and other cancers, as single agents or in combination with other therapeutics. The use of CDK4/6 inhibitors in combination with endocrine therapy has demonstrated significant efficacy in the treatment of hormone receptor (HR)-positive, human epidermal growth factor 2 (HER2)-negative advanced or metastatic breast cancers, and CDK4/6 inhibitors, including palbociclib, ribociclib and abemaciclib, have been approved in combination with endocrine therapy in a first-or second-line setting. Palbociclib, ribociclib and abemaciclib have been approved for treatment of hormone receptor (HR)-positive, human epidermal growth factor receptor 2 (HER2)-negative advanced or metastatic breast cancer in combination with aromatase inhibitors, such as letrozole, in a first line setting and with fulvestrant in second or later lines of therapy in certain patients. (O'Leary et al. Treating cancer with selective CDK4/6 inhbitors. Nature Reviews (2016) 13:417-430).

Palbociclib, or 6-acetyl-8-cyclopentyl-5-methyl-2-(5-piperazin-1-yl-pyridin-2-ylamino)-8*H*-pyrido[2,3-d]pyrimidin-7-one (also referred to as PD-0332991) is a potent and selective inhibitor of CDK4 and CDK6, having the structure:

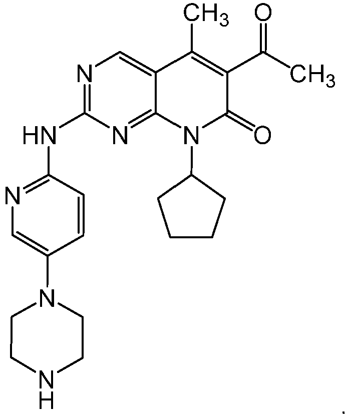

Palbociclib is described in WHO Drug Information, Vol. 27, No. 2, page 172 (2013). Palbociclib and pharmaceutically acceptable salts thereof are disclosed in International Publication No. WO 2003/062236 and U.S. Patent Nos. 6,936,612, 7,456,168 and RE47,739; International Publication No. WO 2005/005426 and U.S. Patent Nos. 7,345,171 and 7,863,278; International Publication No. WO 2008/032157 and U.S. Patent No. 7,781,583; and International Publication No. WO 2014/128588.

The compound, 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol (referred to herein as "COMPOUND F"), is a potent and selective inhibitor of CDK4, having the structure:

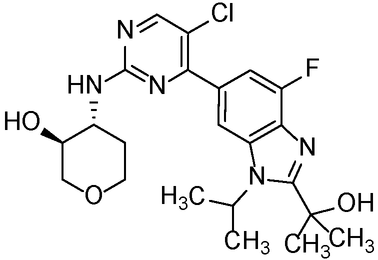

COMPOUND F and pharmaceutically acceptable salts thereof, are disclosed in International Publication No. WO 2019/207463 published October 31, 2019.

While CDK4/6 inhibitors have shown significant clinical efficacy in ER-positive metastatic breast cancer, as with other kinases their effects may be limited over time by the development of primary or acquired resistance. The selective CDK4/6 inhibitor palbociclib has proven to be clinically efficacious in breast cancer (DeMichele A, Clark AS, Tan KS, et al. CDK4/6 inhibitor palbociclib (PD-0332991) in Rb+ advanced breast cancer: phase II activity, safety, and predictive biomarker assessment. Clin Cancer Res 2015; 21(5):995-1001; Finn RS, Martin M, Rugo HS, et al. Palbociclib and Letrozole in Advanced Breast Cancer. New Engl J Med 2016; 375(20):1925-36; Cristofanilli M, Turner NC, Bondarenko I, et al. Fulvestrant plus palbociclib versus fulvestrant plus placebo for treatment of hormone-receptor-positive, HER2-negative metastatic breast cancer that progressed on previous endocrine therapy (PALOMA-3): final analysis of the multicentre, double-blind, phase 3 randomised controlled trial. Lancet Oncol 2016; 17(4):425-39), however, after initial clinical benefit, acquired resistance to palbociclib may occur (Knudsen Erik S., Witkiewicz Agnieszka K., The Strange Case of CDK4/6 Inhibitors: Mechanisms, Resistance, and Combination Strategies. Trends Cancer 2017; 3(1):39-55).

Accordingly, there remains a need for improved therapies for the treatment of cancers. The combinations and methods of the present invention are believed to have one or more advantages, such as greater efficacy; potential to reduce side effects; potential to reduce drug-drug interactions; or potential to overcome resistance mechanisms, such as breast cancers resistant to endocrine therapy; and the like.

### Summary of the Invention

Each of the embodiments described herein envisions within its scope pharmaceutically acceptable salts of the compounds described herein. Accordingly, the phrase "or a pharmaceutically acceptable salt thereof" is implicit in the description of all compounds described herein.

The invention relates to a lysine acetyltransferase 6 (KAT6) inhibitor for use in a method for treating cancer comprising administering to a patient in need thereof an amount of a KAT6 inhibitor and a) an amount of a CDK4 inhibitor; b) an amount of an antiestrogen; or c) an amount of a CDK4 inhibitor and an amount of an antiestrogen; wherein the amounts together are therapeutically effective in treating cancer, wherein the cancer is breast cancer; wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; wherein the antiestrogen is fulvestrant; and wherein the CDK4 inhibitor is either 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof.

In an embodiment of the invention the KAT6 inhibitor is administered with an amount of the CDK4 inhibitor, wherein the amounts together are therapeutically effective in treating the breast cancer.

In an embodiment of the invention the KAT6 inhibitor is administered with an amount of the antiestrogen, wherein the amounts together are therapeutically effective in treating the breast cancer.

In an embodiment of the invention the KAT6 inhibitor is administered with an amount of the CDK4 inhibitor, and an amount of the antiestrogen, wherein the amounts together are therapeutically effective in treating the breast cancer.

In an embodiment of any of the methods of the invention, the patient is human.

In an embodiment of the invention an amount of 2-methoxy-*N*-{4-methoxy-6-[(1*H-*pyrazol-1-yl)methyl]-1,2-benzoxazol-3-yl}benzene-1-sulfonamide or a pharmaceutically acceptable salt thereof, is administered with an amount of palbociclib, wherein the amounts together are therapeutically effective in treating the breast cancer.

In an embodiment of the invention an amount of 2-methoxy-*N*-{4-methoxy-6-[(1*H-*pyrazol-1-yl)methyl]-1,2-benzoxazol-3-yl}benzene-1-sulfonamide or a pharmaceutically acceptable salt thereof, is administered with an amount of 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol or a pharmaceutically acceptable salt thereof, wherein the amounts together are therapeutically effective in treating the breast cancer.

In an embodiment of the invention an amount of 2-methoxy-*N*-{4-methoxy-6-[(1*H-*pyrazol-1-yl)methyl]-1,2-benzoxazol-3-yl}benzene-1-sulfonamide or a pharmaceutically acceptable salt thereof, is administered with an amount of fulvestrant, wherein the amounts together are therapeutically effective in treating the breast cancer.

In an embodiment of the invention an amount of 2-methoxy-*N*-{4-methoxy-6-[(1*H-*pyrazol-1-yl)methyl]-1,2-benzoxazol-3-yl}benzene-1-sulfonamide or a pharmaceutically acceptable salt thereof, is administered with an amount of palbociclib or a pharmaceutically acceptable salt thereof, and an amount of fulvestrant, wherein the amounts together are therapeutically effective in treating the breast cancer.

In an embodiment of the present invention, the breast cancer to be treated is hormone receptor positive (HR+) breast cancer; the hormone receptor positive (HR+) breast cancer is selected from the group consisting of progesterone receptor positive (PR+) breast cancer and estrogen receptor positive (ER+) breast cancer; and the hormone receptor positive (HR+) breast cancer is progesterone receptor positive (PR+) breast cancer.

In an embodiment of the present invention, the breast cancer to be treated is estrogen receptor positive (ER+) breast cancer; the estrogen receptor positive (ER+) breast cancer is human epidermal growth factor receptor 2 negative (HER2-); the estrogen receptor positive (ER+) breast cancer is human epidermal growth factor receptor 2 positive (HER2+).

The invention relates to a lysine acetyltransferase 6 (KAT6) inhibitor for use in a method of overcoming clinical resistance to endocrine therapy comprising administering to a patient in need thereof an amount of a KAT6 inhibitor and an amount of a CDK4 inhibitor, wherein the amounts together are therapeutically effective in overcoming clinical resistance to endocrine therapy, wherein the endocrine therapy is used for treating breast cancer; wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; and wherein the CDK4 inhibitor is either 1,5-anhydro-3-({5-chloro-4-[4-fluoro-2-(2-hydroxypropan-2-yl)-1-(propan-2-yl)-1*H-*benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof.

In an embodiment the breast cancer is hormone receptor positive (HR+) breast cancer the hormone receptor positive (HR+) breast cancer is selected from the group consisting of progesterone receptor positive (PR+) breast cancer and estrogen receptor positive (ER+) breast cancer; the hormone receptor positive (HR+) breast cancer is progesterone receptor positive (PR+) breast cancer; the hormone receptor positive (HR+) breast cancer is estrogen receptor positive (ER+) breast cancer; the estrogen receptor positive (ER+) breast cancer is human epidermal growth factor receptor 2 negative (HER2-); and the estrogen receptor positive (ER+) breast cancer is human epidermal growth factor receptor 2 negative (HER2-).

In an embodiment the CDK4 inhibitor is a CDK4 selective inhibitor, which CDK4 selective inhibitor is 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof.

In an embodiment the CDK4 inhibitor is a CDK4/6 inhibitor, which CDK4/6 inhibitor is palbociclib, or a pharmaceutically acceptable salt thereof.

### Brief Description of the Drawings

Figure 1A shows the number of cells that proliferated in T747D ER+ breast cancer cells after removal of palbociclib treatment.
Figure 1B shows the number of cells that proliferated in MCF7 ER+ breast cancer cells after removal of palbociclib treatment.
Figure 2 shows an outline of a pooled RNAi screen used to identify genes required for reversible proliferation arrest during palbociclib treatment.
Figure 3A shows the workflow used to validate KAT6A as an epigenetic enzyme required for ER+ breast cancer cell proliferation and recovery from palbociclib arrest.
Figure 3B shows the results of the proliferation colony formation assays and the palbociclib recovery colony formation assays in T47D and ZR75-1 ER+ breast cancer cells.
Figure 4A shows the results of the proliferation colony formation assay in MCF7 ER+ breast cancer cells.
Figure 4B shows the results of the palbociclib recovery assay in MCF7 ER+ breast cancer cells.
Figure 5A shows the the results of the proliferation colony formation assay in CAMA1 ER+ breast cancer cells.
Figure 5B shows the number of cells per culture for KAT6A_5, KAT6A_6, shCB3 and KAT6A_10_1u in CAMA1 ER+ breast cancer cells after 14 days with KAT6A knockdown.
Figure 6 shows the morphology in CAMA1 cells after KAT6A knockdown.
Figure 7A shows that KAT6A knockdown has a moderate effect on proliferation and a substantial synthetic lethal effect when combined with 100 nM palbociclib in EFM192A ER+ breast cancer cells.
Figure 7B shows the efficient knockdown of KAT6A by shKAT6A_5 and shKAT6A_6 at the mRNA level in EFM192A ER+ breast cancer cells.
Figure 7C shows the efficient knockdown of KAT6A by both shKAT6A_5 and shKAT6A_6 at the protein level in EFM192A ER+ breast cancer cells.
Figure 8A shows that Compound A inhibits proliferation of T47D ER+ breast cancer cells.
Figure 8B shows that a combination of Compound A and palbociclib blocks recovery of T47D ER+ breast cancer cells from palbociclib growth arrest.
Figure 8C shows that Compound B inhibits proliferation of T47D ER+ breast cancer cells.
Figure 8D shows that a combination of Compound B and palbociclib blocks recovery of T47D ER+ breast cancer cells from palbociclib growth arrest.
Figure 9 shows that Compound A inhibits proliferation and blocks recovery of ZR-75-1 ER+ breast cancer cells from palbociclib growth arrest.
Figure 10 shows that Compound A as a single agent and in combination with palbociclib inhibits recovery of MCF7 ER+ breast cancer cells from palbociclib growth arrest.
Figure 11A shows the results of KAT6A knockdown in T47D, ZR75-1 and CAMA1 ER+ breast cancer cells.
Figure 11B shows the reduction of *ESR1* mRNA in T47D, ZR75-1 and CAMA1 ER+ breast cancer cells.
Figure 11C shows the reduction of ERα protein in T47D, ZR75-1 and CAMA1 ER+ breast cancer cells.
Figure 12A shows that KAT6A knockdown led to a significant reduction in ERα protein in T47D ER+ breast cancer cells and that re-expression of ESR1 from an ectopic promoter restored ERα protein level.
Figure 12B shows that KAT6A knockdown led to a significant reduction in *ESR1* transcript in T47D ER+ breast cancer cells and that re-expression of ESR1 from an ectopic promoter restored ESR1 transcript level.
Figure 12C shows that KAT6A knockdown led to a significant reduction in cell growth in T47D ER+ breast cancer cells and that re-expression of ESR1 from an ectopic promoter restored cell growth in the presence of KAT6A depletion.
Figure 12D shows that re-expression of *ESR1* partially restored recovery of T47D cells from cell cycle arrest by palbociclib in combination with KAT6A depletion.
Figure 13A shows that a combination of Compound C and palbociclib inhibits cell growth and blocks recovery of T47D ER+ breast cancer cells.
Figure 13B shows that a combination of Compound C and palbociclib inhibits cell growth and blocks recovery of ZR75-1 ER+ breast cancer cells.
Figure 13C shows that a combination of Compound D and palbociclib inhibits cell growth and blocks recovery of T47D ER+ breast cancer cells.
Figure 13D shows that a combination of Compound D and palbociclib inhibits cell growth in and blocks recovery of ZR75-1 ER+ breast cancer cells.
Figure 14A shows that treatment of Compound C results in a dose-dependent depletion of both ERα and Cyclin D1 in T47D ER+ breast cancer cells.
Figure 14B shows that treatment of Compound D results in a dose-dependent depletion of both ERα and Cyclin D1 in T47D ER+ breast cancer cells.
Figure 14C shows that Compound D results in a dose-dependent depletion of *ESR1* and *CCND1* mRNA, as measured by qPCR, in T47D ER+ breast cancer cells.
Figure 14D shows that Compound D results in a dose-dependent depletion of *ESR1* and *CCND1* mRNA, as measured by qPCR, in T47D ER+ breast cancer cells.
Figure 15A shows the sensitivity of Y537S, D538G, and Y537S/D538 mutations to fulvestrant, palbociclib, and Compound C as a single agent and in combination with palbociclib in T47D ER+ breast cancer cells.
Figure 15B shows the sensitivity of Y537S, D538G, and Y537S/D538 mutations to fulvestrant, palbociclib, and Compound D as a single agent and in combination with palbociclib in T47D ER+ breast cancer cells.
Figure 16A shows a reduction of *ESRI* mRNA by palbociclib and Compound A, as a single agent and in combination, in T47D ER+ breast cancer cells.
Figure 16B shows a reduction of *ESRI* mRNA by palbociclib as a single agent and in combination with Compound C, in T47D ER+ breast cancer cells.
Figure 16C shows a reduction of ERα level by palbociclib as a single agent and in combination with Compound C in T47D and MCF7 ER+ breast cancer cells.
Figure 16D shows a reduction of *ESR1* mRNA level, as measured by qPCR, by palbociclib as a single agent and in combination with Compound C in T47D and MCF7 ER+ breast cancer cells.
Figure 17A shows tumor growth inhibition and growth delay by Compound E, palbociclib and fulvestrant as single agents and in doublet and triplet combinations in the ST340 PDX model.
Figure 17B shows the tolerability of Compound E, palbociclib and fulvestrant as single agents and in doublet and triplet combinations in the ST340 PDX model.
Figure 18A shows tumor growth inhibition and growth delay by Compound E, palbociclib and fulvestrant as single agents and in doublet and triplet combinations in the ST941 PDX model.
Figure 18B shows the tolerability of Compound E, palbociclib and fulvestrant as single agents and in doublet and triplet combinations in the ST941 PDX model.

### Detailed Description of the Invention

As used herein, the term "about" when used to modify a numerically defined parameter (e.g., the dose of a KAT6 inhibitor or a CDK inhibitor) means that the parameter may vary by as much as 10% below or above the stated numerical value for that parameter. For example, a dose of about 5 mg means 5% ± 10%, i.e. it may vary between 4.5 mg and 5.5 mg.

As used herein, terms, including, but not limited to, "agent", "component", "composition, "compound", "drug", "targeted agent", "targeted therapeutic agent", and "therapeutic agent" may be used interchangeably to refer to the compounds of the present invention, specifically a KAT6 inhibitor and a CDK4 inhibitor.

The following abbreviations may be used herein: BID (twice a day, twice daily, two times daily); Dox (doxycycline); DMEM (Dulbecco's Modified Eagle's Medium); DMSO (dimethylsulphoxide); dNTP (deoxyribonucleotide triphosphate); FBS (fetal bovine serum); RPMI (Roswell Park Memorial Institute); PBS (phosphate buffered saline); PCR (polymerase chain reaction); PEG300 (polyethylene glycol 300); mpk (mg/kg or mg drug per kg body weight of animal); PO (oral); Q7D (every 7 days, once a week); QD (daily, every day); and SC (subcutaneous).

As used herein, a "KAT6 inhibitor" includes an inhibitor of KAT6A, an inhibitor of KAT6B, and an inhibitor of KAT6A and KAT6B. KAT6 inhibitors are disclosed in International Publication No. WO2019/043139A1; International Publication No WO2019/243491A1; International Publication No. WO2020/002587; and International Application Serial No. PCT/IB2020/055667.

The preparation of 2-methoxy-*N*-{4-methoxy-6-[(1*H*-pyrazol-1-yl)methyl]-1,2-benzoxazol-3-yl}benzene-1-sulfonamide (COMPOUND E) is described below.

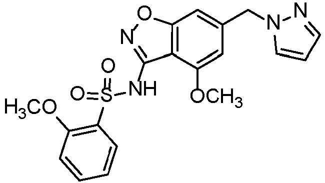

### Synthesis of 1-(methanesulfonyl)-1H-pyrazole (Int-13) according to Scheme 1.

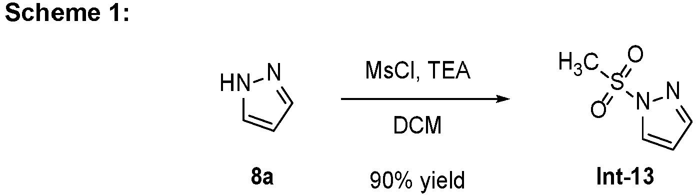

To a solution of 1*H*-pyrazole **(8a)** (33.0 g, 485 mmol) and TEA (73.6 mg, 727 mmol) in DCM was added MsCl (73.9 g, 645 mmol) slowly at 0 °C. The mixture was stirred at 0 °C for 10 min and then room temperature for 1 h. TLC analysis (1:1 ethyl acetate (EtOAc)/petroleum ether) showed consumption of the starting material. The reaction was diluted with saturated aqueous NH₄Cl (200 mL) and the mixture was separated. The aqueous layer was extracted with DCM (200 mL). The combined organic layers were washed with brine (300 mL) and saturated aqueous Na₂CO₃ (300 mL), dried over anhydrous Na₂SO₄, filtered, and concentrated to provide 1-(methanesulfonyl)-1*H*-pyrazole **(Int-13)** (64 g, 90% yield) as a pale-yellow oil. ¹H NMR (400 MHz, CDCl₃) δ 8.04 (d, J=2.6 Hz, 1H), 7.86 - 7.79 (m, 1H), 6.46 (dd, J=1.6, 2.7 Hz, 1H), 3.33 (s, 3H).

### Synthesis of 4-methoxy-6-[(1H-pyrazol-1-yl)methyl]-1,2-benzoxazol-3-amine to Scheme 2.

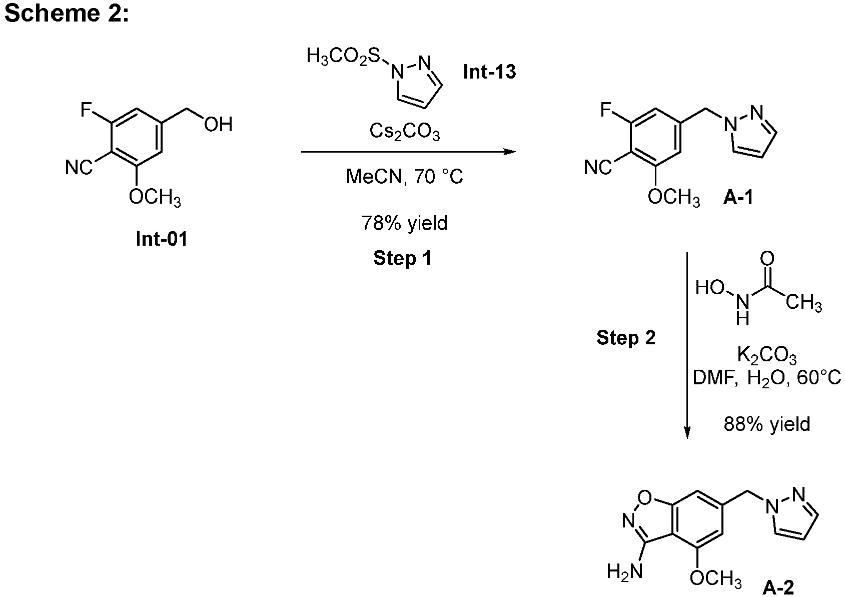

### Step 1: Synthesis of 2-fluoro-6-methoxy-4-[(1H-pyrazol-1-yl)methyl]benzonitrile (A-1).

To a solution of 2-fluoro-4-(hydroxymethyl)-6-methoxybenzonitrile **(Int-01)** (7.0 g, 38.6 mmol) and 1-(methanesulfonyl)-1H-pyrazole **(Int-13)** (6.2 g, 42.5 mmol) in MeCN (150 mL) was added Cs₂CO₃ (18.9 g, 58 mmol). The mixture was stirred at 70 °C for 2 h. LCMS analysis showed consumption of the starting material. The reaction was filtered and the filtrate was concentrated to dryness. The crude residue was purified by flash chromatography (40 g SiO₂, 1:1 EtOAc/petroleum ether) to provide 2-fluoro-6-methoxy-4-[(1*H*-pyrazol-1-yl)methyl]benzonitrile **(A-1)** (7.0 g, 78% yield) as a yellow solid. *m*/*z* (ESI+) 231.8 (M+H)⁺.

### Step 2: Synthesis of 4-methoxy-6-[(1H-pyrazol-1-yl)methyl]-1,2-benzoxazol-3-amine (A-2).

To a solution of 2-fluoro-6-methoxy-4-[(1*H*-pyrazol-1-yl)methyl]benzonitrile **(A-1)** (7.0 g, 30.3 mmol) and *N*-hydroxyacetamide (6.8 g, 90.8 mmol) in DMF (200 mL) and H₂O (30 mL) was added K₂CO₃ (25.1 g, 182 mmol). The mixture was stirred at 60 °C for 16 h. TLC analysis (EtOAc) showed consumption of the starting material. The reaction mixture was concentrated to remove the majority of the DMF and then diluted with H₂O (100 mL). The resultant precipitate was collected by filtration. The filter cake was washed with H₂O (3x20 mL) and dried in vacuum to provide 4-methoxy-6-[(1*H*-pyrazol-1-yl)methyl]-1,2-benzoxazol-3-amine **(A-2)** (6.0 g). The above filtrate was extracted with EtOAc (2x30 mL). The combined organic layers were dried over Na₂SO₄, filtered, and concentrated. The residue was purified by flash chromatography (SiO₂, EtOAc) to provide an additional batch of 4-methoxy-6-[(1*H*-pyrazol-1-yl)methyl]-1,2-benzoxazol-3-amine **(A-2)** (0.5 g). The two batches of product were combined and dried under vacuum to provide 4-methoxy-6-[(1*H*-pyrazol-1-yl)methyl]-1,2-benzoxazol-3-amine **(A-2)** (6.5 g, 88% yield) as a yellow solid. ¹H NMR (400 MHz, DMSO-*d*₆) δ 7.88 (d, *J*=2.0 Hz, 1H), 7.51 (d, *J*=1.3 Hz, 1H), 6.70 (s, 1H), 6.63 (s, 1H), 6.31 (t, *J*=2.0 Hz, 1H), 6.08 - 5.78 (m, 2H), 5.52 - 5.31 (m, 2H), 3.93 - 3.73 (m, 3H). *m*/*z* (ESI+) 244.8 (M+H)⁺.

### Preparation of 2-methoxy-N-{4-methoxy-6-[(1H-pyrazol-1-yl)methyl]-1,2-benzoxazol-3-yl}benzene-1-sulfonamide (COMPOUND E) according to Scheme 3 (Route A).

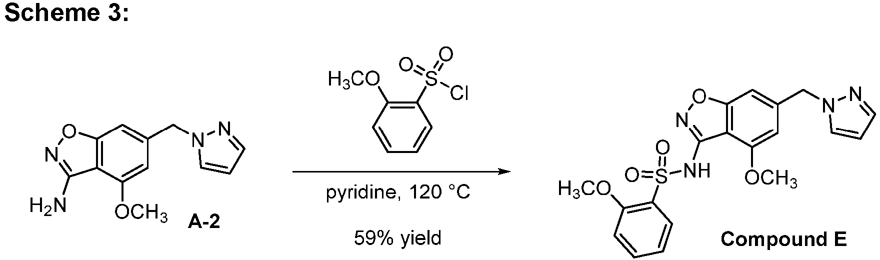

To a suspension of 4-methoxy-6-[(1*H*-pyrazol-1-yl)methyl]-1,2-benzoxazol-3-amine **(A-2)** (2.5 g, 10 mmol) in pyridine (8.0 mL) was added 2-methoxybenzene-1-sulfonyl chloride (3.17 g, 15.4 mmol). The reaction was stirred at 120 °C for 1.5 h. The mixture was cooled to room temperature and diluted with MeOH. The resultant suspension was filtered. and the filter cake was washed with MeOH (30 mL). The solids were dissolved in DCM (50 mL) and MeOH (30 mL) was added. The DCM was removed under vacuum and the precipitate was collected by filtration. The filter cake was dried by lyophilization to provide 2-methoxy-*N*-{4-methoxy-6-[(1*H*-pyrazol-1-yl)methyl]-1,2-benzoxazol-3-yl}benzene-1-sulfonamide **(COMPOUND E)** (2.5 g, 59% yield) as a white solid. ¹H NMR (400 MHz, DMSO-*d*₆) δ 10.18 (s, 1H), 7.87 (d, *J*= 2.0 Hz, 1H), 7.80 (dd, *J*=1.6, 7.9 Hz, 1H), 7.66 - 7.59 (m, 1H), 7.49 (d, *J*=1.5 Hz, 1H), 7.19 (d, *J*=8.3 Hz, 1H), 7.09 (t, *J*=7.7 Hz, 1H), 6.83 (s, 1H), 6.74 (s, 1H), 6.30 (t, *J*=2.0 Hz, 1H), 5.44 (s, 2H), 3.82 (s, 3H), 3.78 (s, 3H); *m*/*z* (ESI+) 415.0 (M+H)⁺.

### Alternative preparation of 2-methoxy-N-{4-methoxy-6-[(1H-pyrazol-1-yl)methyl]-1,2-benzoxazol-3-yl}benzene-1-sulfonamide (COMPOUND E) according to Scheme 4.

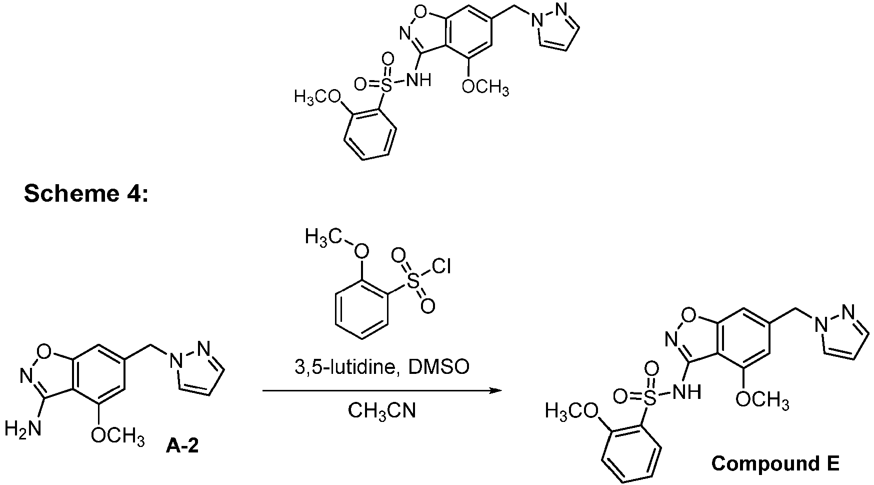

A 100 mL reactor equipped with an overhead stirrer was charged with 4-methoxy-6-(1*H*-pyrazol-1-ylmethyl)-1,2-benzoxazol-3-amine **(A-2)** (10.00 g, 40.94 mmol), 2-methoxybenzenesulfonyl chloride (10.15 g, 49.13 mmol), and acetonitrile (100 mL). The resulting suspension was stirred at 25 °C for 55 minutes. Via pipette, dimethylsulfoxide (0.36 mL, 4.09 mmol) was added in one portion. Via syringe, 3,5-lutidine (14.8 mL, 122.82 mmol) was added dropwise over 15 minutes. The resulting light-yellow suspension was stirred at 25 °C for 18 hours to reach >98% conversion as judged by LCMS. The reaction mixture was acidified with 1 M aq. HCl (100 mL), then concentrated to ~80 mL (rotary evaporator, 40 °C, 85 mbar). The slurry was treated with additional 1 M aq. HCl (40 mL) to rinse down the walls of the vessel, then stirred at 20 °C for 2.5 hours. The resulting precipitate was collected by suction filtration. The filter cake was washed with water (2 x 50 mL), then dried under vacuum at 35 °C for 48 hours, affording crude 2-methoxy-*N*-{4-methoxy-6-[(1*H*-pyrazol-1-yl)methyl]-1,2-benzoxazol-3-yl}benzene-1-sulfonamide **(Compound E)** (15.2 g, 90% yield, 98% purity by LCMS) as a solid. *m*/*z* 415.1 (M+H)⁺.

To purify the crude product, a suspension of crude 2-methoxy-*N*-{4-methoxy-6-[(1*H*-pyrazol-1-yl)methyl]-1,2-benzoxazol-3-yl}benzene-1-sulfonamide **(Compound E)** (14.00 g, 33.78 mmol) in dichloromethane (210 mL) was heated in a 40 °C bath until a clear solution was obtained (10 minutes). The mixture was filtered, and the filtrate returned to a clean reaction vessel, using additional dichloromethane (70 mL) to quantitate the transfer. Ethyl acetate (140 mL) was added to the solution over 2 minutes, then the mixture stirred for 2.5 hours. No crystallization was observed, so the solution was concentrated under reduced pressure (200 mbar) to remove dichloromethane (volume was reduced by about 70 mL). More ethyl acetate (140 mL) was added to the residue, and the mixture stirred at room temperature for 21 hours. The resulting suspension was concentrated under reduced pressure (40 °C, 200 mbar) to about 280 mL, then stirred at room temperature for 3 hours. The solids were collected by filtration, with additional ethyl acetate (70 mL) used to rinse the reaction vessel and filter cake. The filter cake was dried in a vacuum oven at 35 °C for 23 hours, affording 2-methoxy-*N*-{4-methoxy-6-[(1*H*-pyrazol-1-yl)methyl]-1,2-benzoxazol-3-yl}benzene-1-sulfonamide **(Compound E)** (12.0 g, 85% yield, 97.9% purity by UPLC, no single impurity larger than 0.5%) as a solid. *m*/*z* 415.1 (M+H)⁺.

To purify further, a suspension of 2-methoxy-*N*-{4-methoxy-6-[(1*H*-pyrazol-1-yl)methyl]-1,2-benzoxazol-3-yl}benzene-1-sulfonamide **(Compound E)** (2.0 g, 4.73 mmol) in acetone (80 mL) was heated to reflux (bath temperature 55 °C) with stirring for 2 hours. While the mixture was still heated, ethyl acetate (30 mL) was added slowly, so that the internal temperature remained above 45 °C. The resulting slurry was concentrated to about 30 mL under mild vacuum (bath temp 65 °C), then cooled slowly at a rate of 1 °C/min to 20 °C (~31 minutes). The resulting precipitate was collected by suction filtration. The filter cake dried under vacuum at 50 °C for 22 hours, yielding 2-methoxy-*N*-{4-methoxy-6-[(1*H*-pyrazol-1-yl)methyl]-1,2-benzoxazol-3-yl}benzene-1-sulfonamide **(Compound E)** (1.825 g, 93% yield, 99.5% purity by UPLC) as a crystalline solid. ¹H NMR (400 MHz, CHLOROFORM-d) δ 8.14 (dd, *J*=1.7, 7.8 Hz, 1H), 8.04 (s, 1H), 7.59 - 7.51 (m, 2H), 7.44 (d, *J*=2.2 Hz, 1H), 7.14 - 7.06 (m, 1H), 6.95 (d, *J*=8.3 Hz, 1H), 6.78 (d, *J*=0.6 Hz, 1H), 6.45 (s, 1H), 6.32 (t, *J*=2.1 Hz, 1H), 5.38 (s, 2H), 3.97 (s, 3H), 3.91 (s, 3H).

### Preparation of 2-methoxy-N-{4-methoxy-6-[(1H-pyrazol-1-yl)methyl]-1,2-benzoxazol-3-yl}benzene-1-sulfonamide anhydrous free base (Form 1) (Compound E) according to Scheme 5 (Route B).

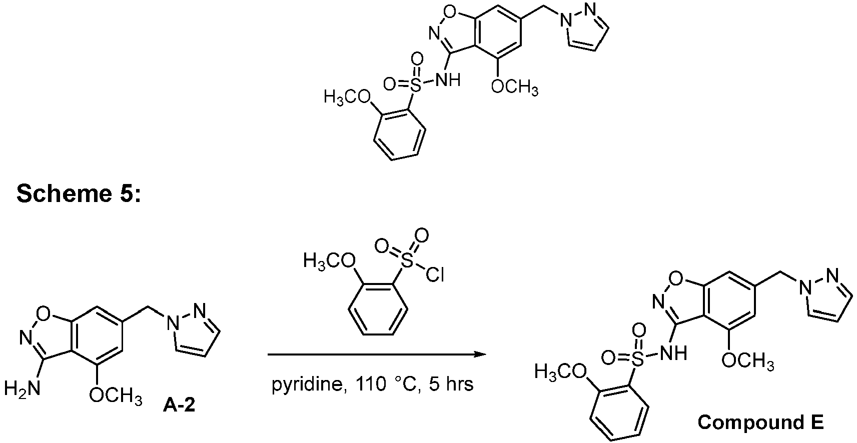

2-methoxybenzene-1-sulfonyl chloride (7.6 g, 37 mmol) was placed in a 2-neck round bottom flask equipped with an internal thermometer. 4-methoxy-6-[(1*H*-pyrazol-1-yl)methyl]-1,2-benzoxazol-3-amine **(A-2)** (8.18 g, 33.5 mmol) was added and the contents dissolved in pyridine (55 mL, 0.6 M) with gentle heating. Heating was initiated with an oil bath temperature of 110° C and internal temperature of 101 ° C. After 5 h of heating, the reaction was complete as determined by LCMS analysis. The reaction was cooled to room temperature and partitioned between DCM (200 mL), 6 N HCl (100 mL) and ice water (100 mL). The product was extracted into DCM (x3) and the combined DCM extract was washed with 1 N HCl (x3) to remove traces of pyridine. The DCM extract was dried over MgSO₄ and concentrated to a dark oil. The oil was purified via flash chromatography eluting with a gradient of 40 - 100% EtOAc in heptane to afford 4.6 g of the product, which was confirmed by NMR. The 4.6 g of the product was recrystallized by first dissolving in CH₃CN (60 mL) at reflux until most of the solids had dissolved. This hot solution was filtered using a pre-heated / hot glass funnel fitted with fluted filter paper. This step removes any inorganic or silica gel impurities. The filter paper was washed with small portions of CH₃CN adding up to a total wash volume of 10 mL. The filtrate was collected in a 250 mL beaker equipped with a stir bar. MTBE (45 mL) was added to the hot filtrate and stirring was initiated. After 30 seconds of stirring, a white precipitate began to form. Stirring was continued at 400 rpm while a gentle stream of N₂ gas was forced across the top of the solution to help speed up the evaporation process. The forced N₂ evaporation was continued for 3 h until the total volume was 50 mL. The white solid was filtered washing with MTBE (x2) and heptane (x2). The white powder was placed in a 3-inch diameter crystallizing dish, covered with piece of filter paper and heated in a 70°C vacuum oven for 48 h using a slow flow of N₂ in and out of the drying oven to aid the drying process. After drying, 3.9 g of crystalline product was obtained, which was confirmed by NMR. Melting point = 203-204° C. Anal. Calcd for C₁₉H₁₈N₄O₅S: C, 55.06; H, 4.38; N, 13.52. Found: C, 55.09; H, 4.41; N, 13.57.

The preparation of 2,6-dimethoxy-*N*-{4-methoxy-6-[(1*H*-pyrazol-1-yl)methyl]-1,2-benzoxazol-3-yl}benzene-1-sulfonamide (COMPOUND C) is described below.

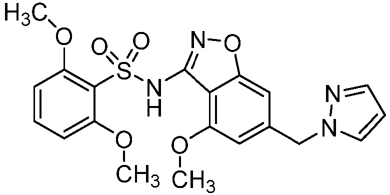

### Preparation of 2,6-dimethoxy-N-{4-methoxy-6-[(1H-pyrazol-1-yl)methyl]-1,2-benzoxazol-3-yl}benzene-1-sulfonamide according to Scheme 6.

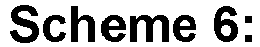

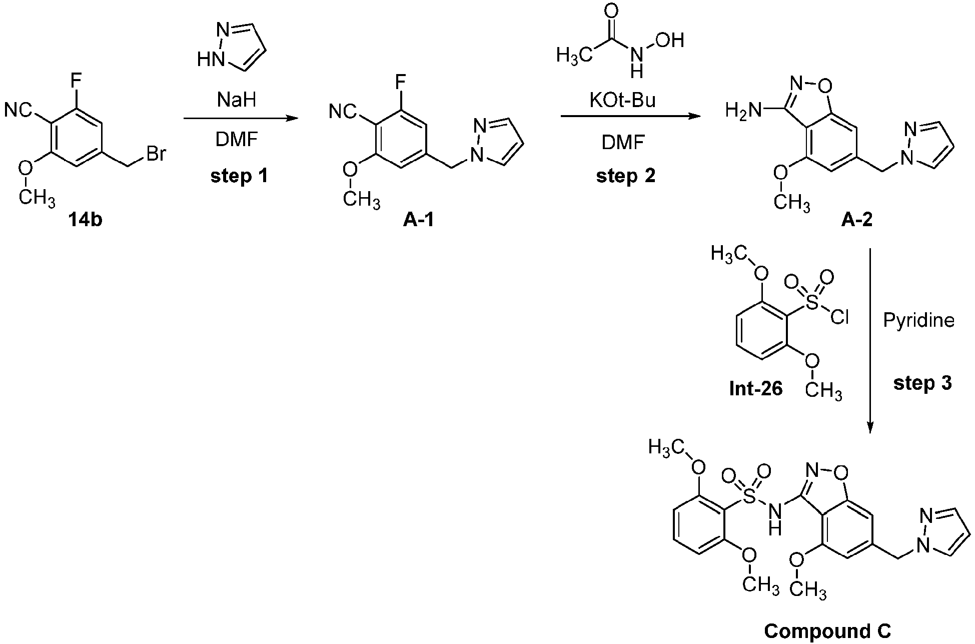

### Step 1: Alternate synthesis of 4-((1H-pyrazol-1-yl)methyl)-2-fluoro-6-methoxybenzonitrile (A-1) from 14b

A solution of 1*H*-pyrazole (2.0 g, 29.6 mmol) and sodium hydride (NaH) (60% w/w dispersion in mineral oil, 1.5 g, 37.1 mmol) in DMF (520 mL) was stirred at 0 °C for 1 h. A solution of 4-(bromomethyl)-2-fluoro-6-methoxybenzonitrile **(14b)** (6.0 g, 24.7 mmol) in DMF (80 mL) was then added and the mixture was stirred at room temperature (RT) overnight. The reaction was quenched with water and the mixture was extracted with EtOAc. The combined organic layers were washed with brine, dried over anhydrous sodium sulfate (Na₂SO₄), filtered and concentrated under reduced pressure. The residue was purified by column chromatography (Pet. Ether/EtOAc = 6/1) to give 4-((1*H*-pyrazol-1-yl)methyl)-2-fluoro-6-methoxybenzonitrile **(A-1)** (2.4 g, 42%) as a yellow solid. *m*/*z* 232.0 [M+H]⁺.

### Step 2: Alternate synthesis of 6-((1H-pyrazol-1-yl)methyl)-4-methoxybenzo[d]isoxazol-3-amine (A-2) using potassium tert-butoxide

To a solution of acetohydroxamic acid (3.7 g, 49.5 mmol) in anhydrous DMF (150 mL) at RT was added potassium *tert*-butoxide (5.6 g, 49.5 mmol) and the mixture was stirred at RT for 1 h. 4-((1*H*-Pyrazol-1-yl)methyl)-2-fluoro-6-methoxybenzonitrile **(A-1)** (3.8 g, 16.5 mmol) was then added and stirring was continued at 60 °C for 4 h. Water was added and the mixture was extracted with EtOAc. The combined organic layers were dried over anhydrous Na₂SO₄, filtered and concentrated under reduced pressure. The residue was purified by column chromatography (Pet. Ether/EtOAc = 5/1) to give 6-((1*H*-pyrazol-1-yl)methyl)-4-methoxybenzo[*d*]isoxazol-3-amine **(A-2)** (2.1 g, 53%) as a yellow solid. *m*/*z* 245.0 [M+H]⁺. ¹H NMR (400 MHz, DMSO-*d*₆) δ 7.87 (dd, *J*=1.6, 0.4 Hz, 1H), 7.50 (dd, *J*=1.6, 0.4 Hz, 1H), 6.69 (s, 1H), 6.62 (s, 1H), 6.30 (t, *J*=2.1 Hz, 1H), 5.93 (s, 2H), 5.41 (s, 2H), 3.86 (s, 3H).

### Step 3: Synthesis of N-(6-((1H-pyrazol-1-yl)methyl)-4-methoxybenzo[d]isoxazol-3-yl)-2,6-dimethoxybenzenesulfonamide (Example 98)

A mixture of 6-((1*H*-pyrazol-1-yl)methyl)-4-methoxybenzo[d]isoxazol-3-amine **(A-2)** (50 mg, 0.205 mmol) and 2,6-dimethoxybenzenesulfonyl chloride **(Int-26)** (73 mg, 0.308 mmol) in pyridine (1 mL) was heated at 120 °C for 2 h under microwave irradiation (Batch 1).

A mixture of 6-((1*H*-pyrazol-1-yl)methyl)-4-methoxybenzo[d]isoxazol-3-amine **(A-2**)(500 mg, 2.1 mmol) and 2,6-dimethoxybenzenesulfonyl chloride **(Int-26)** (746 mg, 3.2 mmol) in pyridine (5 mL) was heated at 120 °C for 2 h under microwave irradiation (Batch 2).

This reaction was repeated once again on exactly the same scale (Batch 3).

A mixture of 6-((1*H*-pyrazol-1-yl)methyl)-4-methoxybenzo[d]isoxazol-3-amine **(A-2**)(350 mg, 1.4 mmol) and 2,6-dimethoxybenzenesulfonyl chloride **(Int-26)** (509 mg, 2.2 mmol) in pyridine (4 mL) was heated at 120 °C for 2 h under microwave irradiation (Batch 4).

The four reaction mixtures were combined, diluted with water, adjusted to pH 5-6 with 2 M aqueous HCl and extracted with EtOAc (300 mL x 3). The combined organic extracts were dried over anhydrous Na₂SO₄, filtered and concentrated under reduced pressure. The residue was purified by column chromatography (Pet. Ether/EtOAc = 2/1) to give N-(6-((1*H*-pyrazol-1-yl)methyl)-4-methoxybenzo[d]isoxazol-3-yl)-2,6-dimethoxybenzenesulfonamide **(Compound C)** (1.07 g, 43%) as a white solid. *m*/*z* 445.0 [M+H]⁺. ¹H NMR (400 MHz, DMSO-*d*₆) δ 9.58 (s, 1H), 7.87 (d, *J*=2.0 Hz, 1H), 7.50 - 7.46 (m, 2H), 6.83 (s, 1H), 6.76 (m, 3H), 6.30 (s, 1H), 5.44 (s, 2H), 3.87 (s, 3H), 3.76 (s, 6H).

### Alternative preparation of 2,6-dimethoxy-N-{4-methoxy-6-[(1H-pyrazol-1-yl)methyl]-1,2-benzoxazol-3-yl}benzene-1-sulfonamide according to Scheme 7.

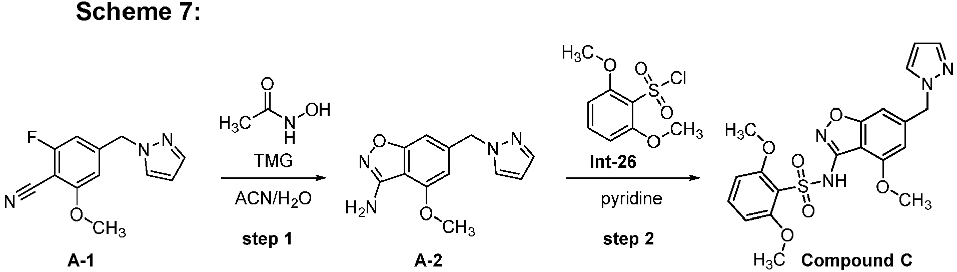

### Step 1: Alternate synthesis of 4-methoxy-6-[(1H-pyrazol-1-yl)methyl]-1,2-benzoxazol-3-amine (A-2) using 1,1,3,3-tetramethylguanidine.

A suspension of 2-fluoro-6-methoxy-4-(1*H*-pyrazol-1-ylmethyl)benzonitrile **(A-1)** (15.43 g, 66.7 mmol), *N*-hydroxyacetamide (15.0 g, 200 mmol), and 1,1,3,3-TMG (46.1 g, 400 mmol) in acetonitrile (270 mL) and deionized water (30 mL) was heated to 60 °C for 7 hours. The acetonitrile was removed under vacuum, and the residual thick oil was partitioned between ethyl acetate (300 mL) and deionized water (250 mL). The aqueous layer was extracted with ethyl acetate (2 x 150 mL). All the organic layers were combined and washed with saturated aqueous NaCl. Some solids began to form in the organic layer, so methanol (~10 mL) was added and the suspension heated until homogeneous. After cooling to room temperature, the organic layer was dried over sodium sulfate, filtered, and concentrated. The resulting pale-yellow solid was suspended in ethyl acetate (125 mL) and heated briefly to reflux. The suspension was allowed to cool to room temperature, the resulting solids were collected by filtration, and the filter cake was rinsed with heptane. The filtrate and heptane rinse were concentrated to dryness, the residual solid suspended in ethyl acetate (15 mL), the suspension briefly heated to reflux, and a second crop of precipitate was collected as before. The combined precipitate crops were dried under vacuum to give 4-methoxy-6-[(1*H*-pyrazol-1-yl)methyl]-1,2-benzoxazol-3-amine **(A-2)** (11.86 g, 48.6 mmol) as a pale-yellow powder. ¹H NMR (400 MHz, DMSO-*d*₆) δ 7.87 (d, *J*=1.8 Hz, 1H), 7.49 (d, *J*=1.2 Hz, 1H), 6.69 (s, 1H), 6.62 (s, 1H), 6.30 (t, *J*=2.1 Hz, 1H), 5.93 (s, 2H), 5.41 (s, 2H), 3.86 (s, 3H). LCMS: [M+H]⁺ 245.

### Step 2: Synthesis of 22,6-dimethoxy-N-{4-methoxy-6-[(1H-pyrazol-1-yl)methyl]-1,2-benzoxazol-3-yl}benzene-1-sulfonamide (Compound C)

A mixture of 4-methoxy-6-[(1*H*-pyrazol-1-yl)methyl]-1,2-benzoxazol-3-amine **(A-2)** (9.5 g, 39 mmol) and 2,6-dimethoxybenzenesulfonyl chloride **(Int-26)** (12.1 g, 51.1 mmol) in pyridine (20 mL) was heated to 97 °C internal for 1 hour. After cooling to 50 °C, the solution was poured into a flask containing crushed ice (200 g) and 6N HCl (100 mL). The reaction flask was rinsed with dichloromethane to quantitate the transfer. The resulting aqueous mixture was extracted with dichloromethane (4 x 100 mL). The combined organic extracts were washed with deionized water and satd. aq. NaCl, dried over magnesium sulfate, filtered, and concentrated to a yellow foam. Methyl acetate (50 mL) was added to the foam and the suspension stirred at room temperature for 1 hour. Solids were collected by suction filtration and rinsed with heptane. After drying under vacuum, crude 2,6-dimethoxy-*N*-[4-methoxy-6-(1*H*-pyrazol-1-ylmethyl)-1,2-benzoxazol-3-yl]benzenesulfonamide **(Compound C**)(16.1 g, 95%) was obtained as an orange-tan solid. Trituration of the crude solid with methyl acetate two more times did not remove the orange color, so the crude product was triturated in warm dichloromethane, allowed to cool to room temperature, and filtered to give a cream-colored white solid. The dichloromethane mother liquor was further purified by chromatography (330 g silica column, eluting with 60-100% ethyl acetate in heptane) to give a white solid. The solids from both the DCM trituration and the chromatography of the DCM filtrate were combined, stirred in refluxing methyl acetate, and cooled to room temperature over 2 hours. The resulting solid was collected by suction filtration and dried in a 100 °C vacuum oven overnight, affording purified 2,6-dimethoxy-*N*-[4-methoxy-6-(1*H*-pyrazol-1-ylmethyl)-1,2-benzoxazol-3-yl]benzenesulfonamide **(Compound C)** (15.3 g, 89%) as an off-white powder.

Three batches of **Compound C** (total 54.3 g), prepared as described above, were combined, suspended in methyl acetate (250 mL), and heated to reflux for 1 hour. After removing from the heating bath, stirring was continued for 4 hours as the mixture cooled to room temperature. The resulting precipitate was collected by filtration and rinsed with heptane. The solid was dried under vacuum at room temperature for 2 hours, then dried further in a 130 °C vacuum oven for 16 hours, affording 2,6-dimethoxy-*N*-[4-methoxy-6-(1*H*-pyrazol-1-ylmethyl)-1,2-benzoxazol-3-yl]benzenesulfonamide **(Compound C)** (53.55 g, 99%) as an off-white solid. ¹H NMR (400 MHz, DMSO-*d*₆) δ 9.60 (s, 1H), 7.88 (d, *J*=1.7 Hz, 1H), 7.45-7.52 (m, 2H), 6.83 (s, 1H), 6.77 (d, *J*=8.4 Hz, 3H), 6.30 (t, *J*=2.1 Hz, 1H), 5.44 (s, 2H), 3.87 (s, 3H), 3.76 (s, 6H). LCMS: [M+H]⁺ 445. Anal. Calcd for C₂₀H₂₀N₄O₆S: C, 54.05; H, 4.54; N, 12.61; S, 7.21. Found: C, 53.91; H, 4.58; N, 12.51; S, 7.09.

Cyclin-dependent kinases (CDKs) and related serine/threonine kinases are important cellular enzymes that perform essential functions in regulating cell division and proliferation. CDK inhibitors include Pan-CDK inhibitors that target a broad spectrum of CDKs or selective CDK inhibitors that target specific CDK(s).

As used herein, a "CDK4 inhibitor" includes a CDK4 selective inhibitor and a CDK4/6 inhibitor. CDK4 selective inhibitors are disclosed in in International Publication No. WO 2019/207463. Examples of CDK4/6 inhibitors include, but are not limited to, abemaciclib, ribociclib and palbociclib. Additional examples of CDK4/6 inhibitors include lerociclib (also known as G1T38) and trilaciclib (also known as GTI128).

In an embodiment, the CDK4 selective inhibitors of the present invention is 1,5-anhydro-3-({5-chloro-4-[4-fluoro-2-(2-hydroxypropan-2-yl)-1-(propan-2-yl)-1*H-*benzimidazol-6-yl]pyrimidin-2-yl}amino)-2,3-dideoxy-D-threo-pentitol, or a pharmaceutically acceptable salt thereof.

In an embodiment, the CDK4/6 inhibitor of the present invention is palbociclib. Unless otherwise indicated herein, palbociclib (also referred to herein as "palbo" or "Palbo") refers to 6-acetyl-8-cyclopentyl-5-methyl-2-(5-piperazin-1-yl-pyridin-2-ylamino)-8*H*-pyrido[2,3-*d*]pyrimidin-7-one, or a pharmaceutically acceptable salt thereof.

The term "antiestrogen" as used herein refers to a class of drugs that prevent estrogens like estradiol from mediating the biological effects in the body. Antiestrogens act by blocking the estrogen receptor (ER) and/or inhibiting or suppressing estrogen production. In some embodiments, an antiestrogen is an aromatase inhibitor, a selective estrogen receptor degrader (SERD) or a selective estrogen receptor modulator (SERM). Examples of an aromatase inhibitor include, but are not limited to, anastrozole. Examples of a SERD include, but are not limited to, fulvestrant. Additional SERDs include elacestrant (RAD-1901, Radius Health), SAR439859 (Sanofi), RG6171 (Roche), AZD9833 (AstraZeneca), AZD9496 (AstraZeneca), rintodestrant (G1 Therapeutics), ZN-c5 (Zentalis), LSZ102 (Novartis), D-0502 (Inventisbio), LY3484356 (Lilly), and SHR9549 (Jiansu Hengrui Medicine). Examples of a SERM include, but are not limited to, tamoxifen, clomifene and raloxifene. Additional SERMS include toremifene, lasofoxifene, bazedoxifene and afimoxifene.

Some embodiments relate to the pharmaceutically acceptable salts of the compounds described herein. Pharmaceutically acceptable salts of the compounds described herein include the acid addition and base addition salts thereof.

Some embodiments also relate to the pharmaceutically acceptable acid addition salts of the compounds described herein. Suitable acid addition salts are formed from acids which form non-toxic salts. Non-limiting examples of suitable acid addition salts, i.e., salts containing pharmacologically acceptable anions, include, but are not limited to, the acetate, acid citrate, adipate, aspartate, benzoate, besylate, bicarbonate/carbonate, bisulphate/sulphate, bitartrate, borate, camsylate, citrate, cyclamate, edisylate, esylate, ethanesulfonate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methanesulfonate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, p-toluenesulfonate, tosylate, trifluoroacetate and xinofoate salts.

Additional embodiments relate to base addition salts of the compounds described herein. Suitable base addition salts are formed from bases which form non-toxic salts. Non-limiting examples of suitable base salts include the aluminum, arginine, benzathine, calcium, choline, diethylamine, diolamine, glycine, lysine, magnesium, meglumine, olamine, potassium, sodium, tromethamine and zinc salts.

The compounds described herein that are basic in nature are capable of forming a wide variety of salts with various inorganic and organic acids. The acids that may be used to prepare pharmaceutically acceptable acid addition salts of such basic compounds described herein are those that form non-toxic acid addition salts, e.g., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds described herein that include a basic moiety, such as an amino group, may form pharmaceutically acceptable salts with various amino acids, in addition to the acids mentioned above.

The chemical bases that may be used as reagents to prepare pharmaceutically acceptable base salts of those compounds of the compounds described herein that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine-(meglumine), and the lower alkanolammonium and other base salts of pharmaceutically acceptable organic amines.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002). Methods for making pharmaceutically acceptable salts of compounds described herein are known to one of skill in the art.

Compounds described herein containing one or more asymmetric carbon atoms can exist as two or more stereoisomers. Where a compound described herein contains an alkenyl or alkenylene group, geometric cis/trans (or Z/E) isomers are possible. Where structural isomers are interconvertible via a low energy barrier, tautomeric isomerism ('tautomerism') can occur. This can take the form of proton tautomerism in compounds described herein containing, for example, an imino, keto, or oxime group, or so-called valence tautomerism in compounds which contain an aromatic moiety. A single compound may exhibit more than one type of isomerism.

The compounds of the embodiments described herein include all stereoisomers (e.g., *cis* and *trans* isomers) and all optical isomers of compounds described herein (e.g., *R* and *S* enantiomers), as well as racemic, diastereomeric and other mixtures of such isomers. While all stereoisomers are encompassed within the scope of our claims, one skilled in the art will recognize that particular stereoisomers may be preferred.

In some embodiments, the compounds described herein can exist in several tautomeric forms, including the enol and imine form, and the keto and enamine form and geometric isomers and mixtures thereof. All such tautomeric forms are included within the scope of the present embodiments. Tautomers exist as mixtures of a tautomeric set in solution. In solid form, usually one tautomer predominates. Even though one tautomer may be described, the present embodiments include all tautomers of the present compounds.

Included within the scope of the present embodiments are all stereoisomers, geometric isomers and tautomeric forms of the compounds described herein, including compounds exhibiting more than one type of isomerism, and mixtures of one or more thereof. Also included are acid addition or base salts wherein the counterion is optically active, for example, d-lactate or I-lysine, or racemic, for example, dl-tartrate or dl-arginine.

The present embodiments also include atropisomers of the compounds described herein. Atropisomers refer to compounds that can be separated into rotationally restricted isomers.

Cis/trans isomers may be separated by conventional techniques well known to those skilled in the art, for example, chromatography and fractional crystallization.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where a compound described herein contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above.

A "patient" to be treated according to this invention includes any warm-blooded animal, such as, but not limited to human, monkey or other lower-order primate, horse, dog, rabbit, guinea pig, or mouse. For example, the patient is human. Those skilled in the medical art are readily able to identify individual patients who are afflicted with cancer, for example, breast cancer and in particular, estrogen receptor positive breast cancer and who are in need of treatment.

The term "combination", as used herein, unless otherwise indicated, means a fixed-dose combination or a combination of agents that is administered intermittently, concurrently or sequentially, according to the same or different route of administration and according to the same or different dosage schedules. As used herein, an "effective" or a "therapeutically effective" amount refers to an amount of an agent, compound, or composition that is of sufficient quantity to result in a decrease in severity of disease symptoms, an increase in frequency and duration of disease symptom-free periods, or a prevention of impairment or disability due to the disease affliction - either as a single dose or according to a multiple dose regimen, alone or in combination with other agents. One of ordinary skill in the art would be able to determine such amounts based on such factors as the patient's size, the severity of the patient's symptoms, and the particular combination, composition or route of administration selected. The patient or subject may be a human or non-human mammal in need of treatment. In one embodiment, the patient is human.

The term "locally advanced", as used herein, as it relates to cancer, may or may not be treated with curative intent. The term "metastatic" as used herein, as it relates to cancer, cannot be treated with curative intent. Those skilled in the art will be able to recognize and diagnose locally advanced and metastatic cancer in a patient.

For convenience, certain well-known abbreviations, may be used herein, including: castration resistant prostate cancer (CRPC), estrogen receptor positive (ER+), human epidermal growth factor receptor 2 negative (HER2-), hormone receptor (HR), human epidermal growth factor receptor 2 positive (HER2+), non-small cell lung cancer (NSCLC) and progesterone receptor (PR).

In one embodiment, the breast cancer is HR+ breast cancer.

In one embodiment, the HR+ breast cancer is PR+ and/or ER+ breast cancer.

In one embodiment, the breast cancer is PR+ breast cancer.

In one embodiment, the breast cancer is ER+ breast cancer.

In one embodiment, the breast cancer is ER+ HER2- breast cancer.

In one embodiment, the breast cancer is ER+ HER2+ breast cancer.

In one embodiment, the breast cancer is locally advanced or metastatic ER+ breast cancer.

In one embodiment, the breast cancer is locally advanced or metastatic ER+ HER2- breast cancer.

In one embodiment, the breast cancer is locally advanced or metastatic ER+ HER2+ breast cancer.

The term "additive" is used to mean that the result of the combination of two compounds, components or targeted agents is no greater than the sum of each compound, component or targeted agent individually. The term "additive" means that there is no improvement in the disease condition or disorder being treated over the use of each compound, component or targeted agent individually.

The terms "synergy" or "synergistic" are used to mean that the result of the combination of two compounds, components or targeted agents is greater than the sum of each agent together. The terms "synergy" or "synergistic" means that there is an improvement in the disease condition or disorder being treated, over the use of each compound, component or targeted agent individually. This improvement in the disease condition or disorder being treated is a "synergistic effect". A "synergistic amount" is an amount of the combination of the two compounds, components or targeted agents that results in a synergistic effect, as "synergistic" is defined herein.

Determining a synergistic interaction between one or two components, the optimum range for the effect and absolute dose ranges of each component for the effect may be definitively measured by administration of the components over different weight per weight ratio ranges and doses to patients in need of treatment. However, the observation of synergy in *in vitro* models or *in vivo* models can be predictive of the effect in humans and other species and *in vitro* models or *in vivo* models exist, as described herein, to measure a synergistic effect and the results of such studies can also be used to predict effective dose and plasma concentration ratio ranges and the absolute doses and plasma concentrations required in humans and other species by the application of pharmacokinetic/pharmacodynamic methods.

In accordance with the present invention, an amount of a first compound or component is combined with an amount of a second compound or component, and optionally with an amount of a third compound or component and the amounts together are effective or therapeutically effective in the treatment of breast cancer. The amounts, which together are effective or therapeutically effective, will relieve to some extent one or more of the symptoms of the disorder being treated. In reference to the treatment of cancer, an effective or therapeutically effective amount refers to that amount which has the effect of (1) reducing the size of the tumor, (2) inhibiting (that is, slowing to some extent, preferably stopping) tumor metastasis emergence, (3) inhibiting to some extent (that is, slowing to some extent, preferably stopping) tumor growth or tumor invasiveness, and/or (4) relieving to some extent (or, preferably, eliminating) one or more signs or symptoms associated with the cancer. Therapeutic or pharmacological effectiveness of the doses and administration regimens may also be characterized as the ability to induce, enhance, maintain or prolong disease control and/or overall survival in patients with these specific tumors, which may be measured as prolongation of the time before disease progression".

In an embodiment, the invention relates to a KAT6 inhibitor for use in a method for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of a KAT6 inhibitor, and an amount of a CDK4 inhibitor or an antiestrogen, wherein the amounts together are therapeutically effective in treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, locally advanced or metastatic ER+ HER2+ breast cancer, and wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; wherein the antiestrogen is fulvestrant; and wherein the CDK4 inhibitor is either 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof. In another embodiment, the invention relates to a KAT6 inhibitor for use in a method for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of a KAT6 inhibitor, and an amount of a CDK4 inhibitor or an antiestrogen, wherein the amounts together achieve synergistic effects in the treatment of breast cancer, HR+ breast cancer, PR+ breast cancer, ER+ breast cancer, ER+ HER2-breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, and wherein the KAT6 inhibitor is 2-methoxy-*N*-{4-methoxy-6-[(1*H-*pyrazol-1-yl)methyl]-1,2-benzoxazol-3-yl}benzene-1-sulfonamide, or a pharmaceutically acceptable salt thereof; wherein the antiestrogen is fulvestrant; and wherein the CDK4 inhibitor is either 1,5-anhydro-3-({5-chloro-4-[4-fluoro-2-(2-hydroxypropan-2-yl)-1-(propan-2-yl)-1*H-*benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof. In an embodiment, the amount of the CDK4 inhibitor in the present invention further comprises administering an amount of fulvestrant.

In an embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof for use in a method for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H-*pyrazol-1-yl)methyl]-1,2-benzoxazol-3-yl}benzene-1-sulfonamide, or a pharmaceutically acceptable salt thereof, and an amount of palbociclib, or a pharmaceutically acceptable salt thereof, wherein the amounts together are therapeutically effective in treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer. In another embodiment, the invention relates to 2-methoxy-*N*-{4-methoxy-6-[(1*H-*pyrazol-1-yl)methyl]-1,2-benzoxazol-3-yl}benzene-1-sulfonamide, or a pharmaceutically acceptable salt thereof for use in a method for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2-breast cancer, locally advanced or metastatic ER+ HER2+ breast cancer, comprising administering to a patient in need thereof an amount of 2-methoxy-*N*-{4-methoxy-6-[(1*H-*pyrazol-1-yl)methyl]-1,2-benzoxazol-3-yl}benzene-1-sulfonamide, or a pharmaceutically acceptable salt thereof, and an amount of palbociclib, or a pharmaceutically acceptable salt thereof, wherein the amounts together achieve synergistic effects in the treatment of breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer.
In an embodiment, the amount of palbociclib, or a pharmaceutically acceptable salt thereof, in the present invention further comprises administering an amount of fulvestrant.

In an embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof for use in a method for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, and an amount of 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof, wherein the amounts together are therapeutically effective in treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer. In another embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof for use in a method for treating breast cancer, HR+ breast cancer, PR+ breast cancer, ER+ breast cancer, ER+ HER2-breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, and an amount of 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof, wherein the amounts together achieve synergistic effects in the treatment of breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer. In an embodiment, the amount of 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof, in the present invention further comprises administering an amount of fulvestrant.

In an embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof for use in a method for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, and an amount of fulvestrant, wherein the amounts together are therapeutically effective in treating cancer, particularly breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer.

The term "endocrine therapy", which is also known as hormone therapy, as used herein, relates to treatment that adds, blocks or removes hormones. In the treatment of breast cancer, there are two types of endocrine therapy: drugs that stop estrogen and progesterone from helping breast cancer cells grow and drugs that keep to ovaries from making the hormones. Endocrine therapy drugs used in the treatment of breast cancer include, but are not limited to, anastrozole, exemestane, fulvestrant, goserelin, letrozole, leuprorelin, megestrol, tamoxifen, and toremifene.

In an embodiment, the invention relates to a KAT6 inhibitor for use in a method of overcoming clinical resistance to endocrine therapy, wherein the endocrine therapy is used for treating breast cancer, HR+ breast cancer, PR+ breast cancer, ER+ breast cancer, ER+ HER2-breast cancer, ER+ HER2+ breast cancer, locally advanced or metastatic ER+ breast cancer, locally advanced or metastatic ER+ HER2- breast cancer, locally advanced or metastatic ER+ HER2+ breast cancer comprising administering to a patient in need thereof an amount of a KAT6A inhibitor and an amount of a CDK4 inhibitor, wherein the amounts together are effective in overcoming clinical resistance to endocrine therapy, and wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; and wherein the CDK4 inhibitor is either 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof. In another embodiment, the invention relates to a KAT6 inhibitor for use in a method of overcoming clinical resistance to endocrine therapy, wherein the endocrine therapy is used for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in
need thereof an amount of a KAT6A inhibitor and an amount of a CDK4 inhibitor, wherein the amounts together achieve synergistic effects in of overcoming clinical resistance to endocrine therapy, and wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; and wherein the CDK4 inhibitor is either 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof.

In an embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, for use in a method of overcoming clinical resistance to endocrine therapy, wherein the endocrine therapy is used for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, and an amount of palbociclib, or a pharmaceutically acceptable salt thereof, wherein the amounts together are effective in overcoming clinical resistance to endocrine therapy. In another embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, for use in a method of overcoming clinical resistance to endocrine therapy, wherein the endocrine therapy is used for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, and an amount of palbociclib, or a pharmaceutically acceptable salt thereof, wherein the amounts together achieve synergistic effects in of overcoming clinical resistance to endocrine therapy. In an embodiment, the amount of palbociclib, or a pharmaceutically acceptable salt thereof, in the the present invention further comprises administering an amount of fulvestrant.

In an embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, for use in a method of overcoming clinical resistance to endocrine therapy, wherein the endocrine therapy is used for treating breast cancer, HR+ breast cancer, PR+ breast cancer, ER+ breast cancer, ER+ HER2- breast cancer, ER+ HER2+ breast cancer, locally advanced or metastatic
ER+ breast cancer, locally advanced or metastatic ER+ HER2- breast cancer, locally advanced or metastatic ER+ HER2+ breast cancer, comprising administering to a patient in need thereof an amount of 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, and an amount of 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof, wherein the amounts together are effective in overcoming clinical resistance to endocrine therapy. In another embodiment, the invention relates to 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, for use in a method of overcoming clinical resistance to endocrine therapy, wherein the endocrine therapy is used for treating breast cancer, HR+ breast cancer, PR+ breast cancer, ER+ breast cancer, ER+ HER2- breast cancer, ER+ HER2+ breast cancer, locally advanced or metastatic ER+ breast cancer, locally advanced or metastatic ER+ HER2-breast cancer, locally advanced or metastatic ER+ HER2+ breast cancer, comprising administering to a patient in need thereof an amount of 2-methoxy*-N*-{4-methoxy-6-[(1*H*-pyrazol-1-yl)methyl]-1,2-benzoxazol-3-yl}benzene-1-sulfonamide, or a pharmaceutically acceptable salt thereof, and an amount of 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof, wherein the amounts together achieve synergistic effects in overcoming clinical resistance to endocrine therapy.

Those skilled in the art will be able to determine, according to known methods, the appropriate amount, dose or dosage of each compound, as used in the methods and combinations of the present invention, to administer to a patient, taking into account factors such as age, weight, general health, the compound or compounds administered, the route of administration, the nature and advancement of the breast cancer, ER+ breast cancer, ER+ HER2- breast cancer, ER+ HER2+ breast cancer, locally advanced or metastatic ER+ breast cancer, locally advanced or metastatic ER+ HER2- breast cancer, locally advanced or metastatic ER+ HER2+ breast cancer, requiring treatment, and the presence of other medications.

In an embodiment, palbociclib, or a pharmaceutically acceptable salt thereof, is administered at a daily dosage of about 125 mg once daily, about 100 mg once daily, about 75 mg once daily, or about 50 mg daily. In an embodiment, which is the recommended starting dose, palbociclib, or a pharmaceutically acceptable salt thereof, is administered at a daily dosage of about 125 mg once a day. For example, palbociclib, or a pharmaceutically acceptable salt thereof, is administered at a dose of about 100 mg once daily, about 75 mg once daily, or about 50 mg once daily. In an embodiment, palbociclib, or a pharmaceutically acceptable salt thereof, is administered at a dose of about 100 mg once daily. In an embodiment, palbociclib, or a pharmaceutically acceptable salt thereof, is administered at a dose of about 75 mg once daily. In an embodiment, palbociclib, or a pharmaceutically acceptable salt thereof, is administered at a dose of about 50 mg once daily. Dosage amounts provided herein refer to the dose of the free base form of palbociclib, or are calculated as the free base equivalent of an administered palbociclib salt form. For example, a dosage or amount of palbociclib, such as 100 mg, 75 mg or 50 mg, refers to the free base equivalent.

In an embodiment, 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof, is administered at a daily dosage of from about 1 mg to about 1000 mg per day. In some embodiments, the CDK4 inhibitor is administered at a daily dosage from about 10 mg to about 500 mg per day. In some embodiments, the CDK4 inhibitor is administered at a dosage of from about 25 mg to about 300 mg per day. In some embodiments the CDK4 inhibitor is administered at dosages of about: 1, 2, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 260, 270, 275, 280, 290, 300, 325, 350, 375, 400, 425, 450, 475 or 500 mg on a QD, BID, TID or QID schedule.

In an embodiment, 2,6-dimethoxy-*N*-{4-methoxy-6-[(1*H*-pyrazol-1-yl)methyl]-1,2-benzoxazol-3-yl}benzene-1-sulfonamide, or a pharmaceutically acceptable salt thereof, or 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof are administered in a dose in the range of may be in the range from about 1 mg to about 1 gram; from about 1 mg to about 250 mg; from about 1 mg to about 100 mg; from about 1 mg to about 50 mg; from about 1 mg to about 25 mg; and from about 1 mg to about 10 mg.

The invention may be accomplished through various administration or dosing regimens. The compounds of the combination of the present invention can be administered intermittently, concurrently or sequentially. In an embodiment, the compounds of the combination of the present invention can be administered in a concurrent dosing regimen.

Repetition of the administration or dosing regimens may be conducted as necessary to achieve the desired reduction or diminution of cancer cells. A "continuous dosing schedule", as used herein, is an administration or dosing regimen without dose interruptions, e.g., without days off treatment. Repetition of 28 day treatment cycles without dose interruptions between the treatment cycles is an example of a continuous dosing schedule. In an embodiment, the compounds of the combination of the present invention can be administered in a continuous dosing schedule. In an embodiment, the compounds of the combination of the present invention can be administered concurrently in a continuous dosing schedule.

In an embodiment, 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, is administered once daily to comprise a complete cycle of 28 days. Repetition of the 28 day cycles is continued during treatment with the combination of the present invention.

The standard recommended dosing regimen, which includes the standard dosing schedule, for palbociclib, or a pharmaceutically acceptable salt thereof, is administration once daily for 21 consecutive days followed by 7 days off treatment to comprise a complete cycle of 28 days. Repetition of the 28 day cycles is continued during treatment with the combination of the present invention.

The standard clinical dosing regimen, for palbociclib, or a pharmaceutically acceptable salt thereof, is administration of 125 mg once daily for 21 consecutive days followed by 7 days off treatment to comprise a complete cycle of 28 days. Repetition of the 28 day cycles is continued during treatment with the combination of the present invention.

In further embodiments of the invention, 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof, is administered in combination with palbociclib and letrozole, where the palbociclib is administered at 125 mg orally, once daily for 21 days followed by 7 days off, and where the letrozole is administered at 2.5 mg orally, daily.

Administration of the compounds of the combination of the present invention can be effected by any method that enables delivery of the compounds to the site of action. These methods include oral routes, intraduodenal routes, parenteral injection (including intravenous, subcutaneous, intramuscular, intravascular or infusion), topical, and rectal administration. Each compound of the combination may be administered according to the same or different route of administration.

The compounds for use in the present invention may be formulated prior to administration. The formulation will preferably be adapted to the particular mode of administration. These compounds may be formulated with pharmaceutically acceptable carriers as known in the art and administered in a wide variety of dosage forms as known in the art. In making the pharmaceutical compositions of the present invention, the active ingredient will usually be mixed with a pharmaceutically acceptable carrier, or diluted by a carrier or enclosed within a carrier. Such carriers include, but are not limited to, solid diluents or fillers, excipients, sterile aqueous media and various non-toxic organic solvents. Dosage unit forms or pharmaceutical compositions include tablets, capsules, such as gelatin capsules, pills, powders, granules, aqueous and nonaqueous oral solutions and suspensions, lozenges, troches, hard candies, sprays, creams, salves, suppositories, jellies, gels, pastes, lotions, ointments, injectable solutions, elixirs, syrups, and parenteral solutions packaged in containers adapted for subdivision into individual doses.

Parenteral formulations include pharmaceutically acceptable aqueous or nonaqueous solutions, dispersion, suspensions, emulsions, and sterile powders for the preparation thereof. Examples of carriers include water, ethanol, polyols (propylene glycol, polyethylene glycol), vegetable oils, and injectable organic esters such as ethyl oleate. Fluidity can be maintained by the use of a coating such as lecithin, a surfactant, or maintaining appropriate particle size. Exemplary parenteral administration forms include solutions or suspensions of the compounds of the invention in sterile aqueous solutions, for example, aqueous propylene glycol or dextrose solutions. Such dosage forms can be suitably buffered, if desired.

Additionally, lubricating agents such as magnesium stearate, sodium lauryl sulfate and talc are often useful for tableting purposes. Solid compositions of a similar type may also be employed in soft and hard filled gelatin capsules. Preferred materials, therefor, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled in this art. For examples, see Remington's Pharmaceutical Sciences, Mack Publishing Company, Easter, Pa., 15th Edition (1975).

A kit comprising the therapeutic agents of the combination of the present invention and written instructions for administration of the therapeutic agents is disclosed. The written instructions elaborate and qualify the modes of administration of the therapeutic agents, for example, for simultaneous or sequential administration of the therapeutic agents of the present invention. The written instructions elaborate and qualify the modes of administration of the therapeutic agents, for example, by specifying the days of administration for each of the therapeutic agents during a 28 day cycle.

### Examples and Reference Examples

### Example 1: Palbociclib Reversibly Arrested ER+ Breast Cancer Cells

### Overview:

Complete Senescence is a terminal state whereby cells undergo irreversible cell cycle arrest and fail to divide. This presents a barrier to tumor growth. In tissue culture, senescent cells arrest in the cell cycle and acquire particular hallmark characteristics which include, large flattened shape and high senescence associated beta-galactosidase activity (SA-β-gal). ER+ breast cancer cell lines treated with palbociclib arrest in the G1 phase of the cell cycle and acquire these senescent characteristics.

Palbociclib treatment induced senescent characteristics in ER+ breast cancer cells, but the treatment with palbociclib in ER+ breast cancer cells did not induce complete senescence.

### Materials and Methods:

T47D and MCF7 were confirmed as ER+ breast cancer cell lines using short tandem repeat ("STR") testing (ATCC STR profiling service). The growth media used for the T47D cells was DMEM (Gibco catalog #11995-065) + 10% FBS (Gibco catalog #10082-147) and for the MCF7 cells was RPMI (Gibco catalog #11875-093) + 10% FBS (Gibco catalog #10082-147).

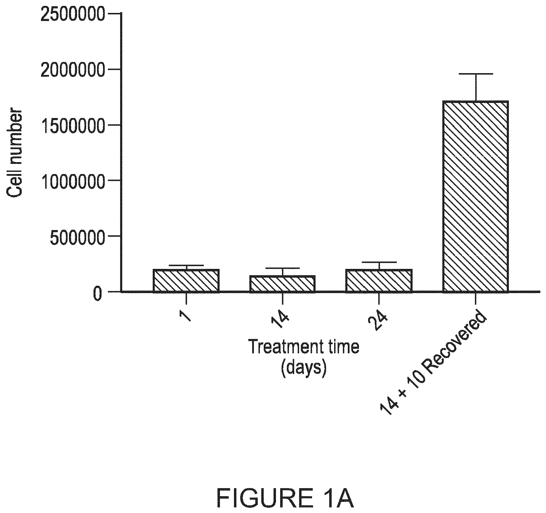

T47D cells were seeded into 6-well tissue culture plates (Falcon catalog # 353046) at 10% confluence. After allowing 18-24 hours for the cells to attach to the plates, 500 nM palbociclib was added to each culture. Growth media and palbociclib was refreshed twice per week (every 3-4 days). This dose of palbociclib resulted in complete cell cycle arrest of the T47D cells. For T47D cultures, the cells were suspended by trypsinization from triplicate cultures on days 1, 14, and 24 of palbociclib treatment and the cell density of each suspension was measured using a Vi-Cell XR cell counter. In a separate experiment, three additional T47D cultures were treated for 14 days with 500 nM palbociclib, then the palbociclib was washed off the cells and the cells were allowed to recover for 10 days. After 10 days of recovery, the cells were suspended by trypsinization and the cell density of each suspension was measured by cell counter. The T47D cultures that were treated with 500 nM palbociclib remained stably arrested for up to 24 days of continuous treatment, the T47D cultures that were allowed 10 days to recover after removal of palbociclib showed significant cell proliferation **(****Figure 1A****).**

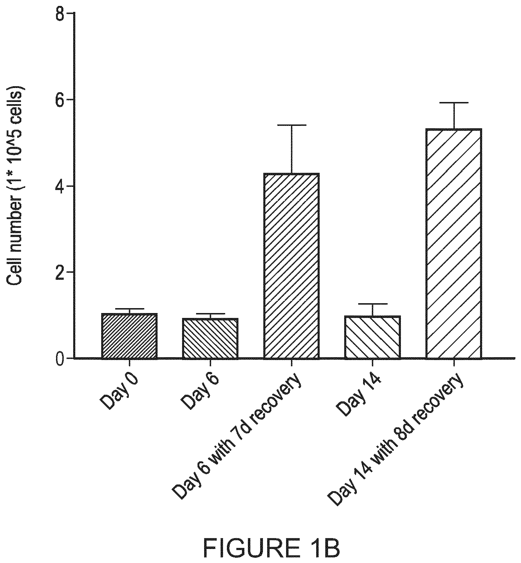

In a similar experiment MCF7 cells were seeded into 6-well tissue culture plates at 10% confluence, allowed 18-24 hours to attach, then treated with 500 nM palbociclib for either 6 or 14 days. This dose of palbociclib resulted in complete cell cycle arrest of MCF7. After 6 and 14 days of palbociclib treatment, the cells in each well were suspended by trypsinization and cell densities were measured by cell counter. For the additional MCF7 cultures that were treated, in parallel, for either 6 or 14 days with 500 nM palbociclib, the palbociclib was washed off the cells and they were allowed 7-8 days to recover. After this recovery period, the cells were suspended by trypsinization and the cell densities of each suspension were measured. Similar to theT47D cells, palbociclib treatment stably arrested MCF7 cells for up to 14 days of treatment **(****Figure 1B****).** However, after removing palbociclib from the MCF7 cultures treated for either 6 or 14 days, the cells readily re-entered the cell cycle and proliferated.

### Results:

The T47D growth arrest resulting from 500 nM palbociclib treatment was reversible upon palbociclib withdrawal and therefore did not induce complete senescence. Similarly, the MCF7 growth arrest resulting from 500 nM palbociclib treatment was reversible upon palbociclib withdrawal and therefore did not induce complete senescence.

Although 500 nM palbociclib induced senescent characteristics in ER+ breast cancer cells, the palbociclib treatment did not induce complete senescence because growth arrest was reversible upon removal of palbociclib.

### Example 2: Identification of KAT6A as an Epigenetic Enzyme Required for Reversible Proliferation Arrest of ER+ Breast Cancer Cells by Palbociclib

### Overview

Based on the results of Example 1, an assay for a pooled RNAi screen was developed to identify genes that were required to enable recovery of cell division of ER+ breast cancer cells from palbociclib growth arrest. The assay measured whether knockdown of a specific protein blocked the ability for the cell to recover from palbociclib induced growth arrest.

The pooled RNAi screen identified KAT6A as an epigenetic enzyme required for recovery from palbociclib induced proliferation arrest and survival of ER+ breast cancer cells after treatment with palbociclib.

### Materials and Methods

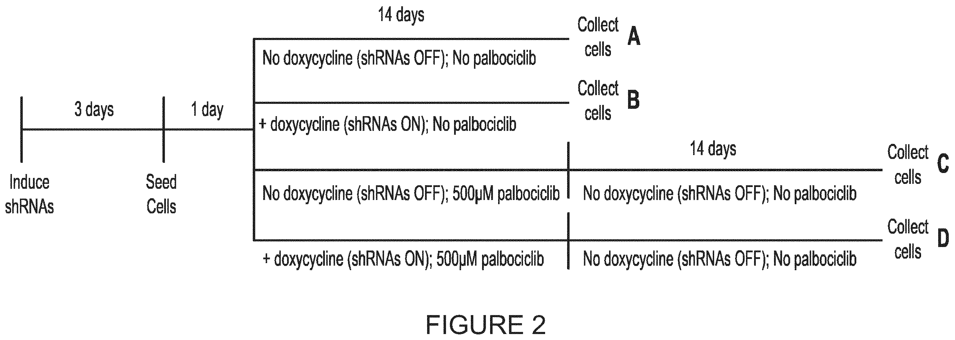

The miR30 shRNA design (Jose M Silva et al. Second-generation shRNA libraries covering the mouse and human genomes. 2005. Nature Genetics 37(11): 1281-1288) was the shRNA platform utilized for the screen and downstream genetic studies. An outline of the pooled RNAi screen performed to identify genes required for reversible proliferation arrest, e.g., survival, of ER+ breast cancer cells during palbociclib treatment is shown in **Figure 2****.**

T47D cells were infected with lentivirus encoding a pool of 4106 different doxycycline-inducible ("Tet-ON") shRNAs targeting 418 different epigenetic enzymes., which were selected from screening an shRNA library targeting epigenetic enzymes. The infection, selection, and expansion of T47D cells infected by virus encoding the different shRNAs was performed in doxycycline-free growth media and therefore the different shRNAs were not expressed in the cells and no epigenetic enzymes were knocked down.

Once sufficient cells were expanded to enable the screen, the cells were seeded to T150 tissue culture flasks at 10-20 % confluence in growth media containing 2 µg/mL puromycin (maintained selection for cells with the shRNAs) and 0.2 µg/mL doxycycline (Sigma catalog # D9891). Addition of doxycycline to the growth media resulted in the induction of expression of the Tet-ON regulated shRNAs. Upon induction of shRNA expression, the shRNAs were processed by the cells to target the knockdown of the 418 different epigenetic enzymes. A minimum of 1000 cells per shRNA (4106000 cells) in each T47D culture were maintained throughout the screen to ensure sufficient representation of each shRNA in the T47D cultures. Expression of the shRNAs were induced for 3 days in order to knockdown the different epigenetic enzymes, then the cells were seeded to T150 flasks for the screen assay.

In parallel, T47D cultures were maintained in doxycycline-free media where expression of the shRNAs were not induced. These cultures were the control cultures for comparison against the cultures in which the shRNAs were induced. After 3 days of expansion of the T47D-shRNA cells in growth media +/- 0.2 µg/mL doxycycline, the cells were suspended and seeded for the screen. A minimum of 1000 cells per shRNA were seeded into triplicate cultures in either of 4 different growth conditions. These growth conditions included (1) growth media no palbociclib no doxycycline **(Proliferation screen - shRNAs OFF),** (2) growth media no palbociclib + 0.2 µg/mL doxycycline **(Proliferation screen - shRNAs ON),** (3) growth media + 500 nM palbociclib no doxycycline **(Palbociclib Recovery screen - shRNAs OFF),** and (4) growth media + 500 nM palbociclib + 0.2 µg/mL doxycycline **(Palbociclib Recovery screen - shRNAs ON).** Growth media for each of these four growth conditions was replaced every 3-4 days (twice per week). When the proliferation screen cultures neared confluence, they were split to maintain sub-confluent cultures and no fewer than 1000 cells per shRNA were seeded to the new cultures in growth media +/- 0.2 ug/mL doxycycline. Since the palbociclib treated cultures were growth arrested, it was not necessary to split these cultures since they remained sub-confluent for the duration of palbociclib treatment. After 14 days of treatment the cells from each of the triplicate proliferation screen cultures (+ doxycycline, shRNAs ON and no doxycycline, shRNAs OFF) were collected and stored for downstream analysis. Both palbociclib and doxycycline were washed off the palbociclib recovery screen cultures and these cultures were allowed to recover 14 days in the absence of palbociclib and epigenetic gene knockdown. It was necessary to restore expression of the epigenetic genes during this recovery phase of the screen to minimize depletion of cells with shRNAs targeting genes essential for cell proliferation. Thus, cells with shRNAs that depleted in the cultures during the recovery phase included shRNAs that knocked down genes that in combination with palbociclib treatment blocked recovery from proliferation arrest. After removal of palbociclib, these cultures began to expand and when they neared confluence the cells were suspended and split to new T150 tissue culture flasks maintaining a minimum of 1000 cells per shRNA. After 14 days of recovery, cells from each of the triplicate palbociclib recovery screen cultures (recovered from + palbociclib shRNAs ON and + palbociclib shRNAs OFF conditions) were collected and stored for downstream analysis.

Cells collected from the triplicate samples for each of the 4 different growth conditions of the proliferation and palbociclib recovery screens (12 samples) were lysed and genomic DNA was purified using the Qiagen DNeasy Genomic DNA kit (catalog # 69504) and the manufacturer's protocol. A nested PCR method was applied to amplify the shRNAs from the genomic DNA samples and to add illumina indexes for downstream MiSeq analysis (Illumina, Inc.).

After MiSeq was completed, the FASTQ file generated was processed to yield gene-level scores as follows. The shRNA reads measured from each growth condition and replicate were summed, shRNAs with fewer than 10 reads in any shRNA OFF sample were excluded (n = 45 / 4,106), and gene-level guide abundance changes (enrichment and depletion) were assessed by MAGeCK-MLE (Li W, Köster J, Xu H, Chen CH, Xiao T, Liu JS, Brown M, Liu XS. Quality control, modeling, and visualization of CRISPR screens with MAGeCK-VISPR. Genome Biol. 2015 Dec 16;16:281.) using 8 threads and 10 permutation rounds, comparing the shRNA ON (+ doxycycline) samples to shRNA OFF (no doxycycline) samples for each of the proliferation and palbociclib recovery screens. Gene-level beta values (effect sizes) were plotted for the recovery and proliferation arms of the shRNA screen (data not shown). A negative gene-level beta value indicated that cells with knockdown of the named gene (shRNA ON) were depleted from the culture under the specific growth condition compared to cells in which the gene was not knocked down (shRNA OFF). The gene-level beta score for KAT6A in the Proliferation Screen was -0.86, which indicated that KAT6A knockdown inhibited proliferation of the cells in the screen and these cells were depleted in the Proliferation Screen cultures.

### Results:

The gene-level beta score for KAT6A in the Palbociclib Recovery Screen was - 1.11, which indicated that KAT6A knockdown inhibited recovery of the cells from palbociclib proliferation arrest and these cells were depleted in the Palbociclib Recovery Screen cultures. Importantly, KAT6A in the Palbociclib Recovery Screen scored with the most negative gene-level beta value of all the genes screened, which indicated that KAT6A knockdown caused the strongest inhibition of T47D cell recovery from palbociclib proliferation arrest than any of the other genes screened.

In the proliferation and palbociclib recovery RNAi screens, a number of epigenetic enzymes required for: 1) proliferation of the breast cancer cell line; 2) recovery from palbociclib arrest; and 3) both proliferation of the breast cancer cell line and recovery from palbociclib arrest, were identified. KAT6A was identified as an epigenetic enzyme required for both breast cancer cell proliferation and recovery of breast cancer cells from palbociclib arrest. KAT6A knockdown was the strongest hit in the palbociclib recovery screen against the ER+ breast cancer cell line T47D. This demonstrated that not only was KAT6A important for T47D cells to proliferate, it was also required for T47D cells to survive and recover cell division after palbociclib was removed. These results suggested that KAT6A depletion during palbociclib treatment resulted in irreversible growth arrest of the breast cancer cells.

### Example 3: Validation of KAT6A as an Epigenetic Enzyme Required for Reversible Proliferation Arrest of ER+ Breast Cancer Cells by Palbociclib

### Overview

Following the results of Example 2, KAT6A was validated as an epigenetic enzyme required for ER+ breast cancer cell proliferation and recovery from palbociclib arrest.

### Materials and Methods

The ER+ breast cancer cell lines T47D, ZR-75-1, MCF7, and CAMA1 were selected for testing based on differences in KAT6A expression. The KAT6A gene is amplified and overexpressed in ZR-75-1 and CAMA1 cells. KAT6A is overexpressed in T47D cells. The KAT6A gene is neither amplified not overexpressed in MCF7 cells. The growth media for T47D and MCF7 was as described in Example 1. The growth media used for ZR-75-1 and CAMA1 was the same as that used for MCF7. Tetracycline-free FBS (Takara catalog # 631106) was used in the growth media to integrate Tet-ON shRNAs into these cell lines. Western blot analysis, as described below, confirmed ≥ 75% KAT6A protein knockdown by these shRNAs in T47D, ZR-75-1, and MCF7 cells Q-PCR confirmed >80% KAT6A mRNA knockdown by these shRNAs in CAMA1.

Whole cell extracts for western blot analysis were performed on the ER+ breast cancer cell lines, T47D, ZR-75-1, MCF7, and CAMA1.

Western blot analysis of KAT6A was performed on the whole cell lysates. KAT6A expression in Tet-ON KAT6A shRNA CAMA1 cells was measured using Q-PCR analysis.

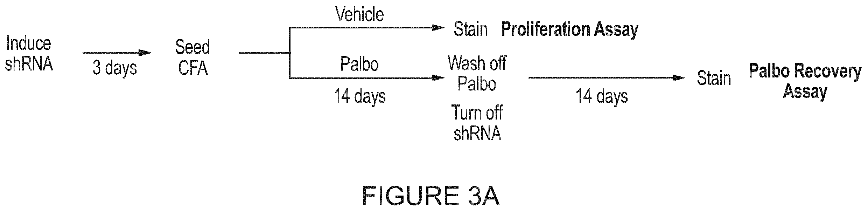

The consequences of KAT6A knockdown in T47D, ZR-75-1, and MCF7 cells against cell proliferation and recovery from palbociclib arrest was tested using the workflow described in **Figure 3A****.** Expression of the shRNAs was induced in the cell lines by adding 0.2 µg/mL doxycycline to the growth media, which initiated knockdown of KAT6A by the different shRNAs in these cell lines. After 3 days of shRNA induction, the cells were seeded into 6-well plates for ~10% confluence. All cultures were seeded in growth media + 0.2 µg/mL doxycycline to maintain shRNA expression and KAT6A knockdown. The cells were seeded into 6-well plates for this colony formation assay ("CFA"). After allowing 18-24 hours for the cells to attach to the wells 500 nM palbociclib was added to half of the cell cultures.

**Proliferation assay:** After 10-14 days, the untreated cultures expressing the non-targeting negative control shRNAs (shRenilla) were confluent and the KAT6A RNAi and shRenilla RNAi cultures were stained to visualize cell density. To stain the cultures, growth media was aspirated and discarded from each cell culture, cells attached in the wells were washed once with 2 mL Phosphate buffered saline (Hyclone, catalog # SH30256.01), then each well of cells was stained with 1 mL of 0.4 % solution of Sulforhodamine B sodium salt (SRB stain, Sigma, catalog # S1402) dissolved in 1 % acetic acid. The cultures were incubated in the stain for 10 minutes at room temperature. The stain was then aspirated from each culture and discarded. Excess stain was washed from each well with 3 washes each of 2 mL 1% acetic acid.

**Palbociclib recovery assay:** For the cell cultures incubated in growth media + 0.2 µg/mL doxycycline + 500 nM palbociclib, after 14 days of palbociclib treatment the growth media containing palbociclib was washed from each cell culture and growth media containing no doxycycline and no palbociclib was added back to allow KAT6A re-expression and recovery from palbociclib cell cycle arrest. After 10-14 days of recovery from palbociclib arrest, the cultures were stained with SRB stain as described above.

### Results

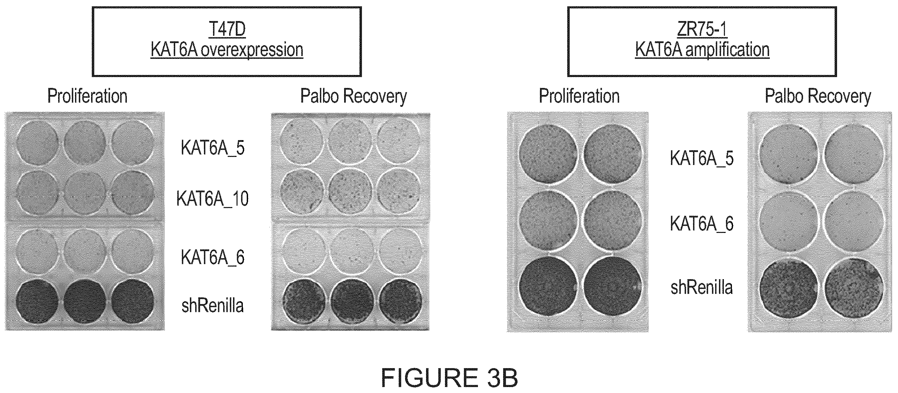

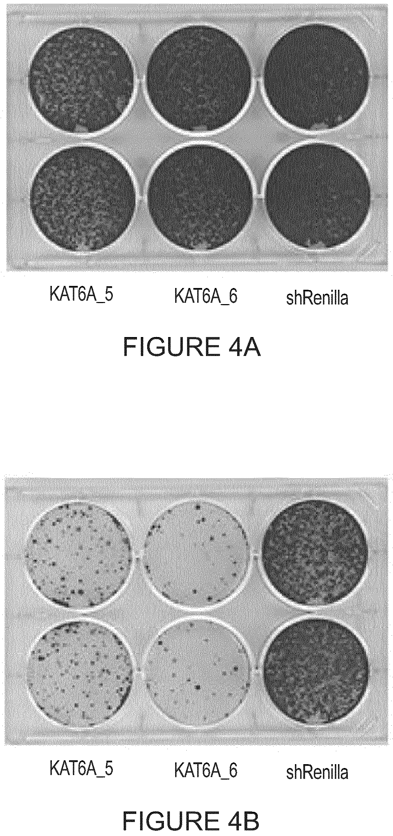

The results of the proliferation colony formation assays demonstrated that KAT6A was required for proliferation of T47D and ZR-75-1 cells, but not MCF7 cells **(****Figures 3B** **and** **4A****).** The weak SRB staining of the T47D and ZR-75-1 cultures induced to express the different KAT6A shRNAs versus the non-targeting shRenilla shRNA indicated poor cell proliferation over the proliferation assay time course. In contrast, a greater than 90% KAT6A knockdown by the most potent KAT6A_6 shRNA in MCF7 cells did not significantly inhibit the proliferation of this cell line, which was reflected by the equivalent staining of the KAT6A_6 MCF7 cultures compared to the shRenilla negative control cultures. The dependence of T47D cells on KAT6A for proliferation was consistent with the RNAi screen described above in Example 2.

The results of the palbociclib recovery colony formation assays demonstrated that KAT6A expression during palbociclib treatment was required for T47D, ZR-75-1, and MCF7 cells to recover cell division after palbociclib withdrawal from the cells **(****Figures 3B** **and** **4B****).** For each of these cell lines, SRB staining of the recovered cultures was much weaker for the cultures that recovered from the combination of KAT6A knockdown + palbociclib compared to the combination of the non-targeting control shRNA + palbociclib. These results validated the dropout of KAT6A shRNAs observed in the palbociclib recovery screen in T47D and demonstrated a broad dependence of ER+ breast cancer cells on KAT6A to recover from palbociclib growth arrest.

In contrast to the results above, the dependence of CAMA1 cells on KAT6A to recover from palbociclib growth arrest could not be measured. The profiling of the response of the CAMA1 cell line to palbociclib demonstrated that unlike most ER+ breast cancer cells, single agent palbociclib treatment resulted in irreversible proliferation arrest of CAMA1 cells.

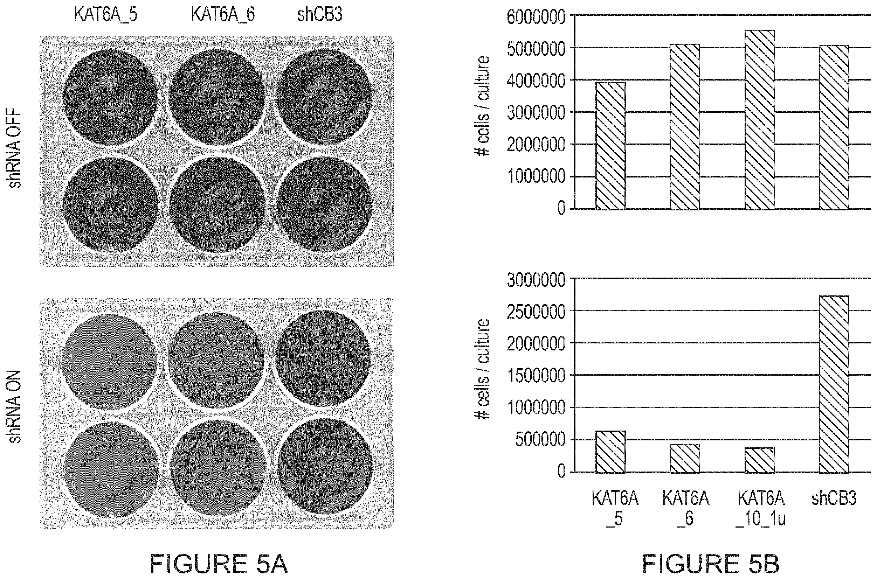

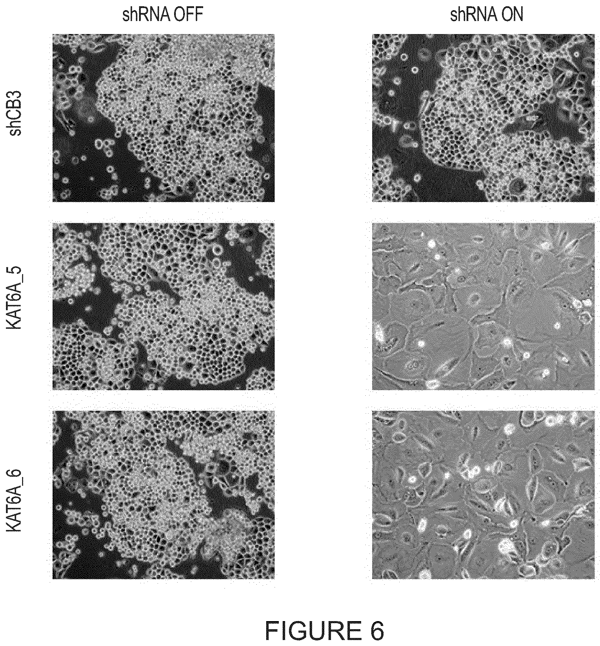

Results from SRB staining of CAMA1 cultures induced to express either of two different potent KAT6A shRNAs, KAT6A_5 and KAT6A_6, compared to the non-targeting CB3 shRNA did not indicate a significant difference in culture growth (Figure **5A****).** However, close inspection of the cells using a light microscope showed that KAT6A knockdown in CAMA1 cells resulted in a dramatic change in cell morphology that included an enlarged, flattened cell morphology indicative of cellular senescence **(****Figure 6****).** These large cells covered the tissue culture plate surface and took up the SRB stain suggesting equivalent staining, even though there were many fewer cells in these cultures compared to the negative control cultures where KAT6A was not knocked down. Taking this into consideration, cell number for CAMA1 cultures with KAT6A knockdown was measured as a separate assay to assess the effect of KAT6A knockdown on cell proliferation. Indeed, when cell number was measured in CAMA1 cultures that were expanded for 14 days with KAT6A knockdown by either the KAT6A_5, KAT6A_6, or KAT6A_10_1u, shRNAs there were consistently and significantly fewer CAMA1 cells compared to cells expressing the non-targeting negative control shCB3 shRNA **(****Figure 5B****).** These results were consistent with the results for T47D and ZR-75-1 and demonstrated that CAMA1 cells were dependent on KAT6A for proliferation. Moreover, visual observation of CAMA1 cells with KAT6A knockdown suggested that KAT6A depletion in this cell line was inducing senescence.

### Example 4: RNAi Knockdown of KAT6A Demonstrated Palbociclib Combination Activity Against ER+ Breast Cancer Cell Lines

### Overview

This Example illustrates that the combination of KAT6A inhibition with palbociclib is broadly effective against ER+ breast cancer cell lines.

### Materials and Methods:

A panel of luminal ER+ breast cancer cell models with KAT6A expression level ranging from low to high **(Table 1)** was evaluated in the proliferation assay and the palbociclib recovery assay as described in Example 1.

**Table 1**

| **Cell Line** | **KAT6A Status** | **ER Status** | **PR Status** | **HER2 Status** | **Subtype** | **KAT6A RNAi Sensitivity** | **KAT6A RNAi + Palbo Sensitivity** |
|---|---|---|---|---|---|---|---|
| EFM19 | Low | + | + | - | Luminal | Yes | Yes |
| MCF7 | Low | + | + | - | Luminal | Partial | Yes |
| EFM192 A | Medium | + | + | + | Luminal | Yes | Yes |
| MDAMB -175VII | Medium | + | - | - | Luminal | Yes | Yes |
| T47D | High | + | + | - | Luminal | Yes | Yes |
| ZR75-1 | High | + | +/- | - | Luminal | Yes | Yes |
| CAMA1 | High | + | +/- | - | Luminal | Yes | Yes |

### Step 1: Generation of stable cell lines that express individual non-targeting control shRNA or shRNAs against KAT6A

EFM19 and EFM192A cell were obtained from Leibniz Institute DSMZ-German Collection of Microorganisms and Cell Cultures. MDAMB175 VII cells were obtained from American Type Culture Collection (ATCC).

Generation and characterization of stable cell lines derived from T47D, ZR75-1, CAMA1 and MCF7 were described in Example 3.

Stable cell lines derived from EFM19, EFM192A and MDAMB175 VII were generated by infecting individual cell lines with lentivirus carrying a puromycin-resistance gene and Tet-OFF non-targeting control shRNA (shRenilla or shCB3) or KAT6A shRNAs (shRNA5 and shRNA6), which sequences have been described in Example 3. With the Tet-OFF shRNA system, addition of doxycycline in the culture medium turns off shRNA expression while removal of doxycycline in the culture medium induces shRNA expression. Cells were infected with lentivirus under low MOI, followed by puromycin selection for infected cells with single copy integration of Tet-OFF shRNA. The entire process was conducted in the presence of 0.2 µg/mL doxycycline to turn off shRNA expression during cell line generation process.

On day 1, cells of individual cell line were plated into 6-well tissue culture plates in DMEM or RPMI tissue culture medium supplemented with 10% tetracycline-free fetal bovine serum (Takara, catalog# 631367) and penicillin/streptomycin (Gibco #15140122). On day 2 in the afternoon, culture medium was removed and 700 µL serum-free DMEM (Gibco catalog #11995-065) + 0.2 µg/mL doxycycline was added back into each well, followed by addition of 2-20 µL of lentivirus carrying individual shRNA. After brief mixing, cells were returned to incubate at 37 °C, 5% CO₂ overnight. On day 3 in the morning, 2 mL fresh tissue culture medium +10% FBS (tetracycline-free) + Pen/Strep + 0.2 µg/mL doxycycline per well were added and returned to plates to incubate at 37 °C, 5% CO₂ for 48 hours. On day 4, medium was removed from each well and fresh medium + 10% FBS (tetracycline-free) + P/S + 0.2 µg/mL doxycycline + 2 µg/mL puromycin (InvivoGen, catalog# ant-pr-1) was added. In the following days, culture medium was refreshed every 2-3 days to keep puromycin selection and allow cell expansion.

### Step 2: Colony formation assay

The colony formation assay was performed in two arms: **proliferation arm** and **palbociclib recovery arm** as described in Example 3. Cell lines carrying shRNA were plated at low density (2 - 5 x10⁴ cells/well) in the absence and presence of doxycycline into 6-well culture dishes and allowed to attach overnight. The following day, plates were refreshed with vehicle or palbociclib (100 - 500 nM) containing medium with (+doxycycline) or without (-doxycycline) induction of shRNA. Vehicle-treated cells were refreshed every three or four days and monitored until the non-targeting control cells were confluent. For the palbociclib recovery arm, palbociclib-treated cells were treated for 14 days with media refreshment every three to four days. At the end of treatment, cells were washed three times with complete media supplemented with 0.2 µg/mL doxycycline to simultaneously turn off shRNA expression and allow cells to recover from palbociclib-induced cell cycle arrest. When non-targeting control cells were confluent, cells in each well were fixed and stained with sulforhodamine B (SRB) for visualization as described in Example 3. To quantify the stain, stained cells were dissolved in 2 mL of 10 mM Tris-HCl, pH 7.5 and shaken for 10 min on a shaker. Samples were then diluted with 10 mM Tris-HCl, pH 7.5, transferred to a 96-well microtiter plate in a volume of 100 µL and read on SpetraMax at 565 nm.

### Results:

KAT6A depletion impacted proliferation of multiple ER+ breast cancer cell models as summarized in **Table 1.** In addition, cooperative inhibition of KAT6A knockdown with palbociclib was consistent across all cell lines tested, as demonstrated by the palbociclib recovery arm of the colony formation assay.

Proliferation of MCF7 cells was minimally affected by KAT6A knockdown **(****Figure 4A****).** However, when combined with palbociclib, KAT6A depletion significantly blocked cell recovery from palbociclib-induced cell cycle arrest **(****Figure 4B****).**

These results demonstrate that KAT6A inhibition acts synergistically with palbociclib to block arrested cancer cells from re-entering cell cycle.

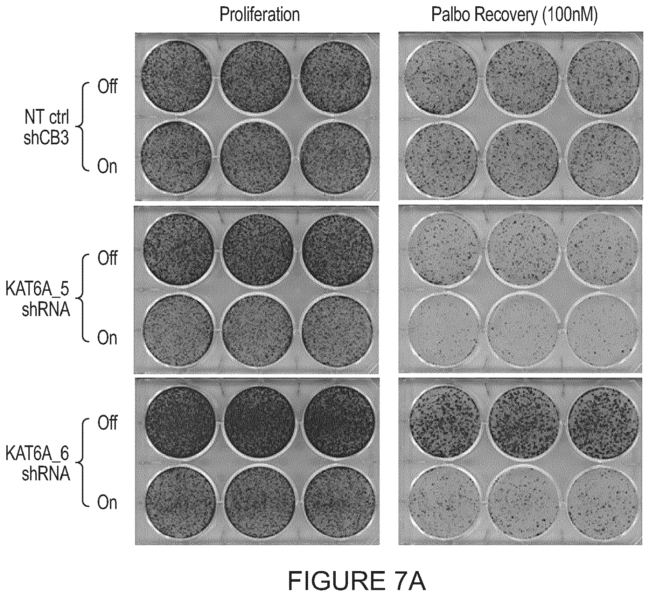

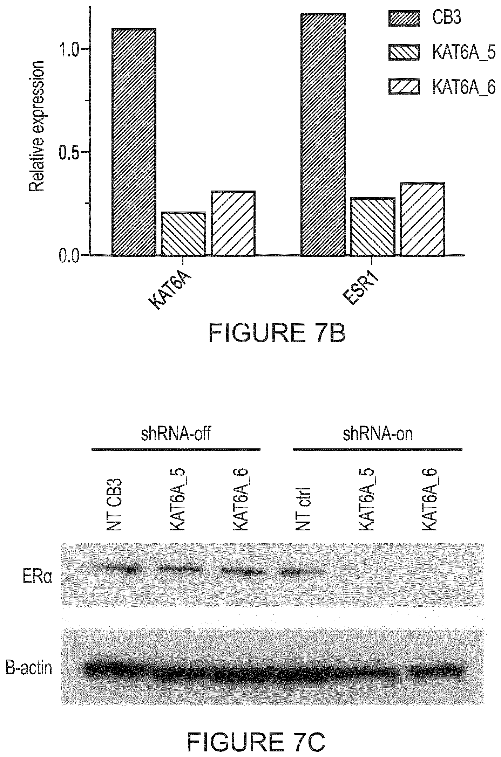

EFM192A is an ER+ HER2+ breast cell line with medium level of KAT6A expression. KAT6A knockdown showed a moderate effect on proliferation and a substantial synthetic lethal effect on blocking cell recovery from palbociliclib-induced arrest when combined with 100 nM palbociclib **(****Figure 7A****).** Efficient knockdown of KAT6A by both shKAT6A_5 and shKAT6A_6 was verified at mRNA **(****Figure 7B****)** and protein level **(****Figure 7C****).**

The inhibitory effects towards proliferation and palbociclib recovery was irrelevant to the KAT6A expression level in these cell models, which demonstrated broad palbociclib/KAT6A inhibitor combination activity against ER+ luminal breast cancer in both KAT6A-high and -low models.

### Example 5: Small Molecule KAT6 Inhibitors Inhibited ER+ Breast Cancer Cell Proliferation and Recovery from Palbociclib Arrest

Small molecule KAT6 inhibitors were evaluated to determine the dependence for proliferation and recovery from palbociclib arrest of ER+ breast cancer cells on the catalytic function of KAT6A.

### Materials and Methods:

Small molecule KAT6 inhibitors, COMPOUND A and COMPOUND B, were evaluated against ER+ breast cancer cells lines, T47D, ZR-75-1, and MCF7. The growth media used for the cell lines was the same as described in Examples 1 and 3 above.

T47D, ZR-75-1, or MCF7 cells were seeded into 6-well tissue culture plates at 10% seeding confluence (100,000-150,000 cells per well). After allowing 18-24 hours for the cells to attach to the wells, growth media was aspirated and discarded and new growth media containing 0-20 µM of either COMPOUND A or COMPOUND B was added in 2-fold increments of descending concentration from 20 µM to the cells (proliferation assay). In parallel, seeded cultures with the same titration of the KAT6 inhibitors was added to the cells. 500 nM palbociclib was also added for each concentration of the KAT6 inhibitors (palbociclib recovery assay). The cultures were incubated 12-14 days and treated with these inhibitors / inhibitor combinations. Growth media containing the inhibitors was replaced on the cell cultures every 3-4 days (twice per week).

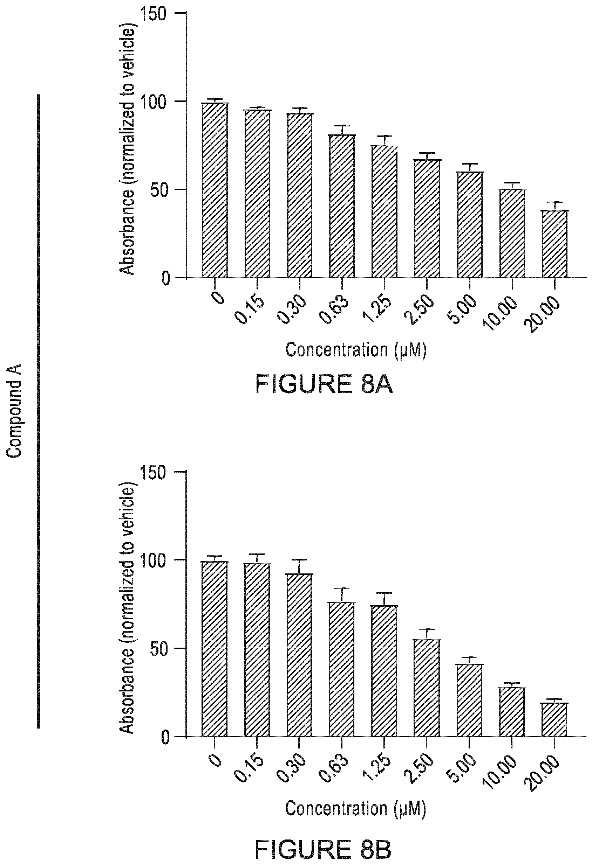

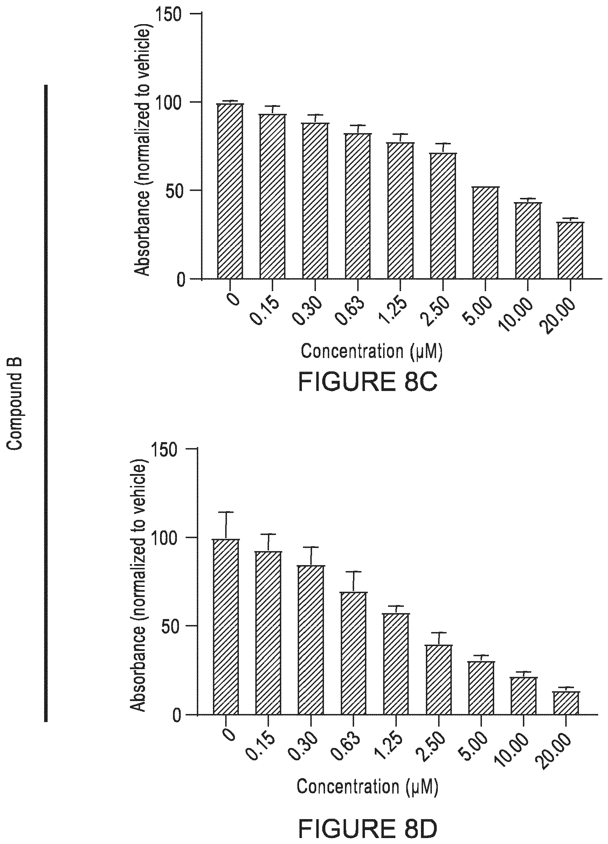

For the KAT6 inhibitor treated T47D cultures (proliferation assay), after 12 days the vehicle (DMSO) treated cultures were >90% confluent and the cell cultures were SRB stained as described in Example 3. The stained 6-well plates were scanned using an Epson Perfection V600 Photo Scanner and Epson Scan version 3.9.2.0 software. To quantitate SRB staining in each 6-well culture, the cultures were destained with 2 mL 10 mM Tris pH 7.5 per well and the absorbance at 565 nm (O.D. 565) of the released stain was measured using a SpectraMax Plus plate reader and SoftMaxPro 5.4.3 software. Inhibition of T47D cell proliferation by both COMPOUND A and COMPOUND B were equivalent with 50% proliferation inhibition observed with 10 µM of the KAT6 inhibitors **(****Figures 8A** **and** **8C****).**

For the T47D cultures arrested with 500 nM palbociclib and treated with increasing concentration of the KAT6 inhibitors (palbociclib recovery assay), after 14 days of combination treatment the growth media containing the inhibitors was aspirated from the cultures and discarded. Each culture was washed 3 times with 2 mL growth media containing no inhibitors. Then, the cultures were allowed to recover in growth media containing no inhibitors. After allowing 14 days for the cultures to recover, those that recovered from single agent 500 nM palbociclib arrest were >90% confluent and all the palbociclib recovery cultures were SRB stained, scanned, and destained / measured as described above. The combination of palbociclib with either COMPOUND A or COMPOUND B inhibited the recovery of T47D cells with 50% inhibition using 5 µM and 2.5 µM KAT6 inhibitor respectively **(****Figures 8B** **and** **8D****).** The activities of COMPOUND A and COMPOUND B KAT6 inhibitors against T47D cell proliferation and recovery from palbociclib arrest were equivalent.

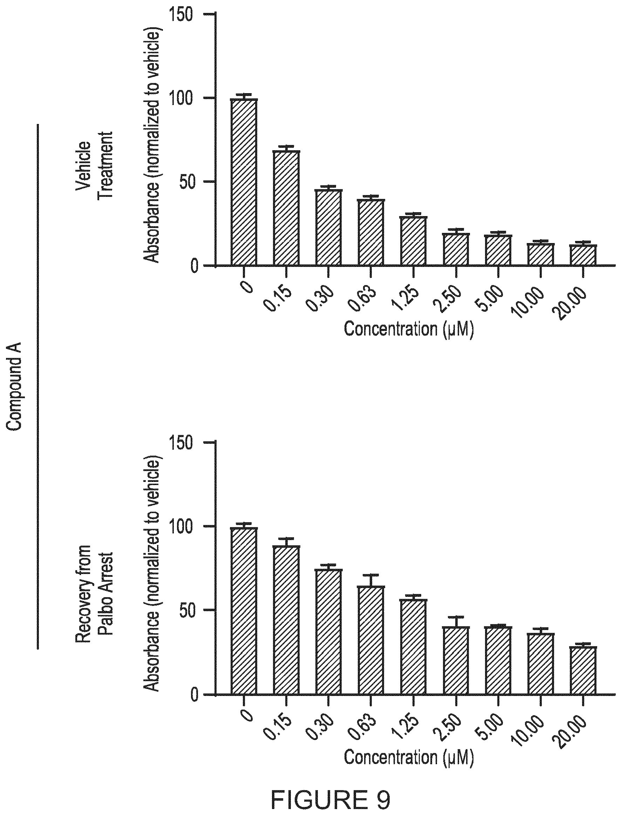

For the ZR-75-1 and MCF7 experiments, only COMPOUND A was tested. For the single agent treated ZR-75-1 cultures the vehicle treated cultures were >90% confluent after 12 days of treatment. These cultures were SRB stained, scanned, and destained / measured as described above. 50% inhibition of proliferation was observed with 0.3 µM COMPOUND A **(****Figure 9****).** For the ZR-75-1 cultures arrested with 500 nM palbociclib in combination with increasing concentration of COMPOUND A, same as for T47D cells, after 14 days of combination inhibitor treatment both inhibitors were aspirated from the cell cultures then each cell culture was washed 3 times each with 2 mL growth media containing no inhibitors and the cell cultures were allowed time to recover from inhibitor treatment in growth media containing no inhibitors. After 22 days of recovery, the ZR-75-1 cultures that had been treated with single agent 500 nM palbociclib were >90% confluent and these palbociclib recovery assay cultures were SRB stained, scanned, and destained / measured as described above. The combination of COMPOUND A with palbociclib treatment inhibited recovery of ZR-75-1 cells with 50% inhibition observed with 2.5 µM COMPOUND A **(****Figure 9****).**

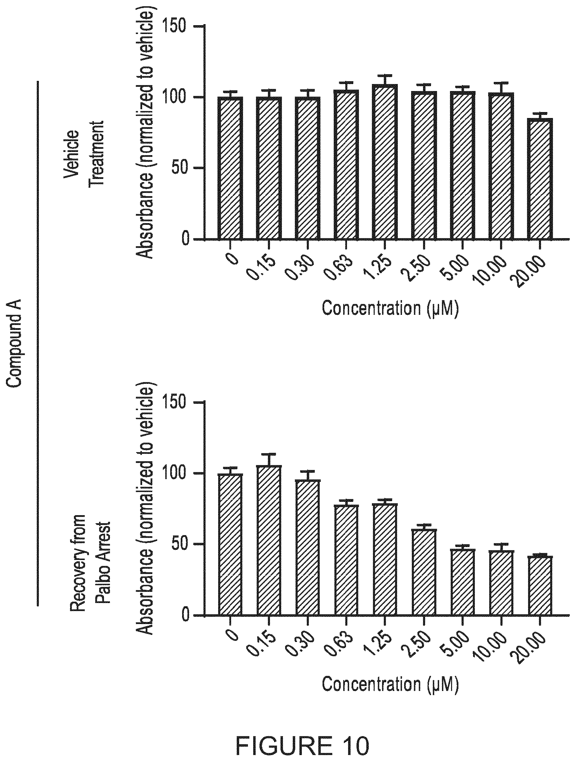

The MCF7 cells were tested with COMPOUND A. For the single agent treated MCF7 cultures, the vehicle treated cultures were >90% confluent after 10 days of treatment. These cultures were SRB stained, scanned, and destained / measured as described above. In contrast to T47D and ZR-75-1 cell lines, 50% inhibition of MCF7 proliferation was not achieved in this assay with up to 20 µM COMPOUND A **(****Figure** 10). For the MCF7 cultures arrested with 500 nM palbociclib in combination with increasing concentration of COMPOUND A, same as for T47D cells, after 14 days of combination inhibitor treatment both inhibitors were aspirated from the cell cultures then each cell culture was washed 3 times each with 2 mL growth media no inhibitors and the cell cultures were allowed time to recover from inhibitor treatment in growth media no inhibitors. After 10 days of recovery, the MCF7 cultures that had been treated with single agent 500 nM palbociclib were >90% confluent and these palbociclib recovery assay cultures were SRB stained, scanned, and destained / measured as described above. COMPOUND A cooperated with palbociclib treatment to inhibit recovery of MCF7 cells with 50% inhibition observed with 5 µM COMPOUND A **(****Figure 10****).**

### Results

Treatment of the T47D, ZR-75-1, and MCF7 ER+ breast cancer cell lines with the small molecule KAT6 inhibitor COMPOUND A displayed results consistent with the results from KAT6A knockdown in these cell lines, specifically that combining KAT6A inhibition with palbociclib strongly delayed re-entry into the cell cycle and recovery of cell division after palbociclib removal and restoration of KAT6A expression. COMPOUND A inhibited proliferation of T47D and ZR-75-1 cells, but not MCF7 cells. COMPOUND A combination with 500 nM palbociclib inhibited / delayed recovery of cell proliferation of all three breast cancer cell lines after ending treatment with the inhibitors.

KAT6A catalytic function is important for its activity driving proliferation of ER+ breast cancer cell lines harboring KAT6A gene amplification / overexpression and also for its activity enabling recovery of cells from proliferation arrest induced by palbociclib in ER+ breast cancer cell lines, independent of KAT6A gene amplification / expression level.

The KAT6 inhibitors, COMPOUND A and COMPOUND B, mimicked the effects of KAT6A knockdown against the breast cancer cell lines T47D, ZR-75-1, and MCF7 both in terms of direct activity against cell proliferation as well as combination activity with palbociclib against their recovery from palbociclib induced proliferation arrest.

These results demonstrate that KAT6 inhibitor synergizes with palbociclib to inhibit proliferation of ER+ breast cancer cells and overcome clinically relevant resistance to endocrine therapy.

### Example 6: Treatment with KAT6 inhibitor, COMPOUND A, Downregulated Estrogen Receptor Alpha Expression and the Estrogen Receptor Regulon

### Overview:

Changes in transcription resulting from treatment with small molecule KAT6 inhibitor, COMPOUND A, in T47D cells, as a single agent and in combination with palbociclib was evaluated to determine the response of breast cancer cells and was compared to KAT6A knockdown.

### Materials and Methods

Parental T47D cells, in which no shRNA was introduced, or T47D cells, in which Tet-ON regulated KAT6A shRNAs (KAT6A_5 or KAT6A_6) or a non-targeting shRNA (shRenilla) were stably integrated, as described in Example 3, were evaluated.

T47D cell lines having either the Tet-ON KAT6A_5, KAT6A_6, or shRenilla shRNAs were treated with 0.2 µg/mL doxycycline to induce expression of the respective shRNA in the T47D cells and initiate knockdown of KAT6A (KAT6A_5 and KAT6A_6 cells only). The cells were treated with doxycycline for 4 days, which provided suffient time for KAT6A protein depletion in the cells. On day 3 of doxycycline treatment, the KAT6A_5, KAT6A_6, and shRenilla T47D cells were suspended and seeded into 6-well tissue cultures plates at 10% confluence. Parental T47D cells (no shRNA) were also seeded into 6-well tissue culture plates at 10% confluence. On day 4 after shRNA induction, half the T47D KAT6A_5, KAT6A_6, and shRenilla cultures were treated with 0.5 µM palbociclib and half the cultures were kept untreated. All cultures were kept in growth media containing 0.2 µg/mL doxycycline to maintain KAT6A knockdown in the cells throughout the experiment. The parental T47D cells were split into four groups as follows: (1) vehicle treated (DMSO), (2) vehicle + 0.5 µM palbociclib, (3) 10 µM COMPOUND A treated, and (4) 10 µM COMPOUND A + 0.5 µM palbociclib. The parental T47D and T47D-shRNA cell lines were subsequently treated for 6 days and the growth media, containing the inhibitors represented the different growth conditions described above, was replaced once on day 3 of treatment. On the sixth day of treatment, the cells were collected for GeneChip analysis **(Table 2).**

**Table 2**

| **Sample** | **# Biological Replicates** | **Conc. Compound A (µM)** | **Conc. Palbociclib (µM)** | **Conc. Doxycycline (µg/µL)** |
|---|---|---|---|---|
| Vehicle | 2 | | | |
| Vehicle + Palbociclib | 2 | | 0.5 | |
| Compound A | 3 | 10 | | |
| Compound A + Palbociclib | 3 | 10 | 0.5 | |
| shRenilla | 2 | | | 0.2 |
| shRenilla + Palbociclib | 2 | | 0.5 | 0.2 |
| shKAT6A_5 | 2 | | | 0.2 |
| shKAT6A_5 + Palbociclib | 2 | | 0.5 | 0.2 |
| shKAT6A_6 | 2 | | | 0.2 |
| shKAT6A_6 + Palbociclib | 2 | | 0.5 | 0.2 |

RNA was purified from each sample described above using the Qiagen RNeasy kit (Qiagen catalog #74136) following the manufacturer's protocol. cDNA synthesis was performed using the Ovation Pico WTA System (NuGEN) and Ribo-SPIA technique. The Ovation Pico WTA products were fragmented and biotin labeled using the Encore Biotin Module (NuGEN). For each sample, 5 µg of biotin-labeled cDNA was hybridized to Human genome U133 + 2.0 oligonucleotide arrays (Affymetrix) using buffers and conditions recommended by the manufacturer. The GeneChips were then washed and stained with Streptavidin R-Phycoerythrin (Molecular Probes) using the GeneChip Fluidics Station 450 and scanned with an Affymetrix GeneChip Scanner 3000. Gene expression data were processed by Microarray Suite 5.0 (MAS5) algorithm.

Differential gene expression analysis demonstrated highly significant overlap in gene expression changes for (1) T47D cells with KAT6A knockdown by either of the 2 independent KAT6A shRNAs (KAT6A_5 vs. KAT6A_6; p-value = 5.4 x 10⁻¹⁹⁷), (2) T47D cells with KAT6A knockdown by the KAT6A_5 shRNA versus COMPOUND A treated (p-value = 3.7 x 10⁻¹⁷⁶), and (3) T47D cells with KAT6A knockdown by the KAT6A_6 shRNA vs. COMPOUND A treated (p-value =1.98 x 10⁻¹²²).

### Results:

Changes in gene expression for T47D cells treated with the small molecule KAT6 inhibitors were similar to the changes in gene expression for T47D cells with KAT6A knockdown. Gene set enrichment analysis of the differentially expressed genes for T47D cells with KAT6A knockdown or treatment with COMPOUND A demonstrated significant enrichment for estradiol response genes as well as a stem cell gene signature. Furthermore, both KAT6A knockdown and COMPOUND A treatment resulted in significant downregulation of ESR1 (encoding estrogen receptor alpha (ERα)) expression and the downstream ERα regulated genes, such as CCND1. These results showed consistent effects of KAT6A knockdown / catalytic inhibition on gene expression in the ER+ breast cancer cell line, T47D, and demonstrated the important function for KAT6A in regulation of estrogen receptor gene expression as well as stem cell gene expression.

### Example 7: Treatment with KAT6 Inhibitor, COMPOUND C, Downregulated Estrogen Receptor Alpha Expression and the Estrogen Receptor Regulon

### Overview:

A gene expression profiling study performed with KAT6 inhibitor, COMPOUND C, in T47D cells followed by RNA-Seq demonstrated that the KAT6 inhibitor maintained its function in down-regulating *ESR1* transcription in ER+ breast cancer cells.

### Materials and Methods:

T47D cells were seeded at 1 million cells in one 10-cm petri dish and allowed to attach overnight in RPMI-1640 media containing 10% FBS (Takara, #631106) and 1x Pen/Strep. The following day, cells were treated with vehicle control (DMSO) or COMPOUND C at 20 nM concentration. Media and compounds were refreshed three days later for a total of 6-day treatment. At the end of the 6-day treatment, cells were harvested by trypsinization and lysed in Buffer RLT (Qiagen, Cat No./ID 79216) + β-mercaptoethanol. RNA lysates were submitted for RNA sequencing ("RNA-Seq") (WuXi NextCODE^{®}).
RNA-Seq data analysis: Adapter sequences were trimmed off from the Illumina raw reads, provided by WuXi NextCODE^{®}, using Trimmomatic (v0.36) for paired-end reads, and low-quality reads were filtered out. Reads remaining after the pre-processing step were mapped to the reference genome hg38 using STAR (v2.6.0c). Gene level expression was quantitated as expected counts and transcripts per million (TPM) using "rsem-calculate-expression" of RSEM (v1.3.0) with default parameters. Differential expression analysis of robustly expressed genes (max expression across samples >=10) was performed using DESeq2 (v1.18.1) package. Gene set enrichment analysis (GSEA, v3.0) was conducted using GseaPreranked option. Genes were ranked based on signedlog10pval. Gene set collections (c2.all.v6.1) from Molecular Signatures Database (MSigDB) were used in this analysis.

### Results:

Gene set enrichment analysis demonstrated that estradiol-induced genes were down-regulated in COMPOUND C-treated cells (NES (Normalized Enrichment Score) is -9,92, FDR (False Discovery Rate) < 0.0001), while estradiol-repressed genes were up-regulated in COMPOUND C-treated cells (NES is 6.27, FDR < 0.0001). These results, generated with COMPOUND C, were consistent with the gene set enrichment analysis of transcriptional profiling data with COMPOUND A as described in Example 6, further supporting the important role of KAT6A in regulating estrogen receptor-dependent response genes and role of KAT6A catalytic function in regulation of this process in ER+ breast cancer cells.

### Example 8: KAT6A Knockdown Lead to Depletion of Estrogen Receptor Alpha in ER+ Breast Cancer Cell Lines

In Examples 6 and 7, *ESR1* gene and estrogen receptor regulon expression were downregulated by KAT6A inhibition in T47D cells, as demonstrated by the gene expression analysis in T47D cells. In this Example, KAT6A depletion resulted in downregulation of ESR1 in other ER+ breast cancer cell lines.

### Materials and Methods:

T47D, ZR75-1 and CAMA1 cell lines, which all express KAT6A gene at a high level, prepared as described in Example 3, were selected for testing. In particular, T47D and ZR75-1 infected with Tet-OFF non-targeting shRenilla, shKAT6A_5 or shKAT6A_6 (expression induced by the absence of doxycycline) and CAMA1 infected with the Tet-ON version of the same set of shRNAs (expression induced by the presence of doxycycline).

Cells were seeded at a density of 200k/well in a T-25 culture flask in RPMI + 10% FBS (tetracycline-free) + Pen/Strep with and without doxycycline. Culture media were refreshed every 3 days. After culturing for 9 days, cells were harvested via trypsinization for protein and RNA. Total RNA was isolated using a RNeasy Plus mini kit (Qiagen, cat# 74134). cDNA was synthesized by reverse transcription with iScript cDNA Synthesis Kit (BIO-RAD, cat# 1708890). Quantitative PCR was run using Quantifast Probe PCR + ROX vial kit (Qiagen, Cat# 204354) on a CFX Realtime system C1000 cycler with following probes form ThermoFisher Scientific: KAT6A (Hs00198899_m 1), ESR1 (Hs01046816_m1) and GAPDH (Hs99999905_m1). The cycling conditions are: 95 °Cx3 min, (95 °Cx3 sec, 60 °Cx30 sec) x 40 cycles.

Protein lysates were collected in RIPA lysis buffer supplemented with HALT (Thermo Fisher Scientific). Forty micrograms of protein extracts were heated in sample buffer at 70°C, separated by Tris-Bis protein gels (NuPAGE Noves 10% Gels, ThermoFisher), and transferred onto a nitrocellulose membrane (iBlot2, Life Technologies). Membranes were blocked and incubated overnight at 4°C with following antibodies: anti-ERα (#8644, Cell Signaling), anti-β-actin (#4970, Cell Signaling). Membranes were subsequently blotted with horseradish peroxidase-conjugated anti-rabbit secondary antibody (7074P2, Cell Signaling) and bands were visualized by an enhanced chemiluminescence system (Thermo Scientific) per manufacturer's instructions.

### Results:

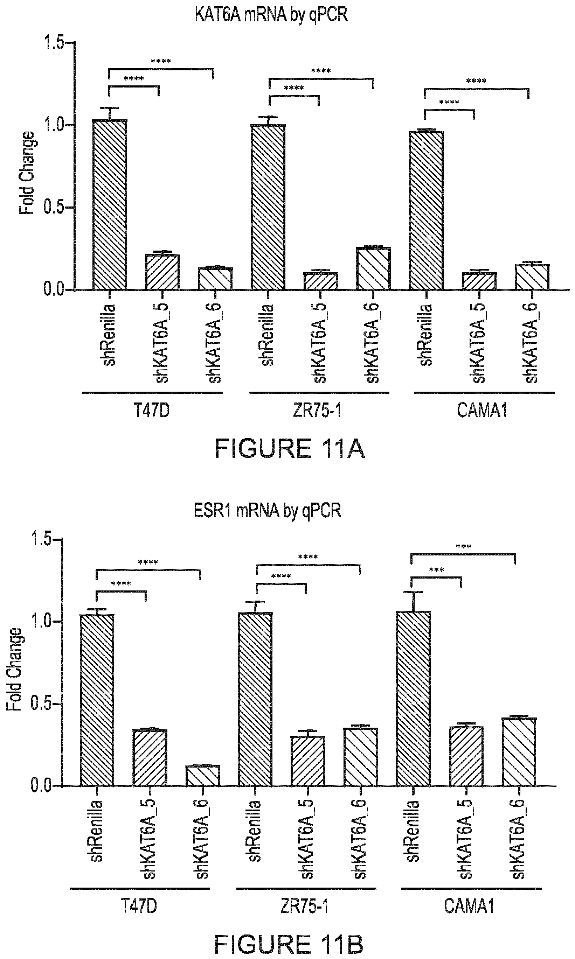

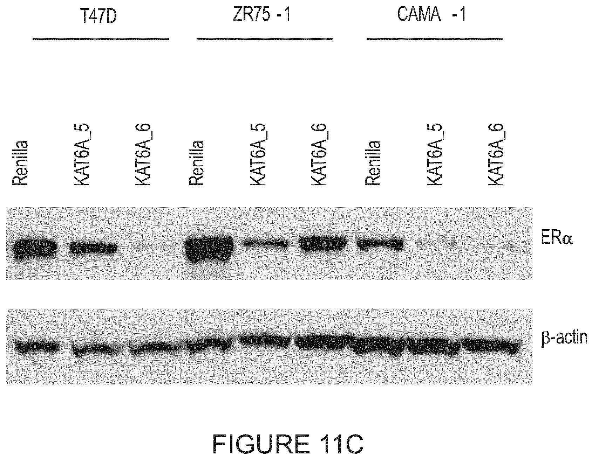

In T47D, ZR75-1 and CAMA1 cells, reduction of *ESR1* mRNA **(****Figure 11B****)** and ERα protein **(****Figure 11C****)** correlated with KAT6A knockdown **(****Figure 11A****),** demonstrating that KAT6A regulated *ESR1* transcription in the high KAT6A-expressing ER+ breast cancer cell lines.

### Example 9: Re-expression of ESR1 Rescued T47D Cells from Growth Inhibition by KAT6A Knockdown and Partially Restored Cell Recovery from Palbociclib-Induced Cell Cycle Arrest

This Example illustrates that re-expression of *ESR1* rescued the proliferation phenotype caused by KAT6A knockdown.

### Step 1: Generation of ESR1 rescue cell lines

*ESR1* open reading frame was cloned downstream of the CMV promoter in a pLenti7.3/V5-TOPO (Invitrogen) based vector that carries a blasticidin selection marker. The construct and plenti7 vector were then packaged into lentiviral particles. T47D cells carrying Tet-OFF shRenilla or shKAT6A_6 were transduced with either lentivirus of the pLenti7 vector or ESR1-expressing construct under low MOI and stable cell lines were generated by selection with 20 µg/mL blasticidin (InvivoGen, catalog# ant-bl-05) followed by cell expansion.

### Step 2: Confirmation of re-expression of ESR1 from CMV promoter in the presence of KAT6A depletion

To confirm that *ESR1* expression was restored by *ESR1* expression from the CMV promoter, KAT6A knockdown in following cell lines generated in Step 1 was induced by the absence of doxycycline:
1. T47D/Tet-OFF shRenilla/plenti7
2. T47D/ Tet-OFF shRenilla/pLenti7-CMV-ESR1
3. T47D/Tet-OFF shKAT6A_6/plenti7
4. T47D/Tet-OFF shKAT6A_6/pLenti7-CMV-ESR1

Cells were seeded at a density of 25000/well in a 12-well culture plate in RPMI + 10% FBS (tetracycline-free) + Pen/Strep + 20 µg/mL blasticidin with and without doxycycline. Culture media were refreshed every 3 days. After culturing for 7 days, cells were harvested by trypsinization for protein and RNA samples. Protein lysates were prepared and total RNA was isolated from each sample. SDS-PAGE, Western blotting and qPCR were performed as described in Example 8.

### Step 3: Rescue in the proliferation and palbociclib recover assay

The colony formation assay was performed to assess direct proliferation and recovery from palbociclib induced cell cycle arrest, as described in Example 4.

### Results:

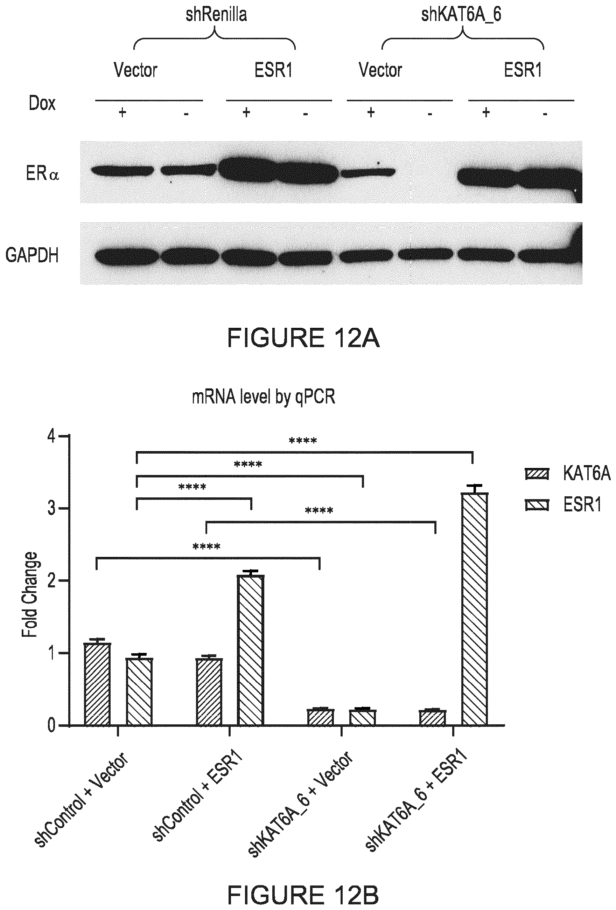

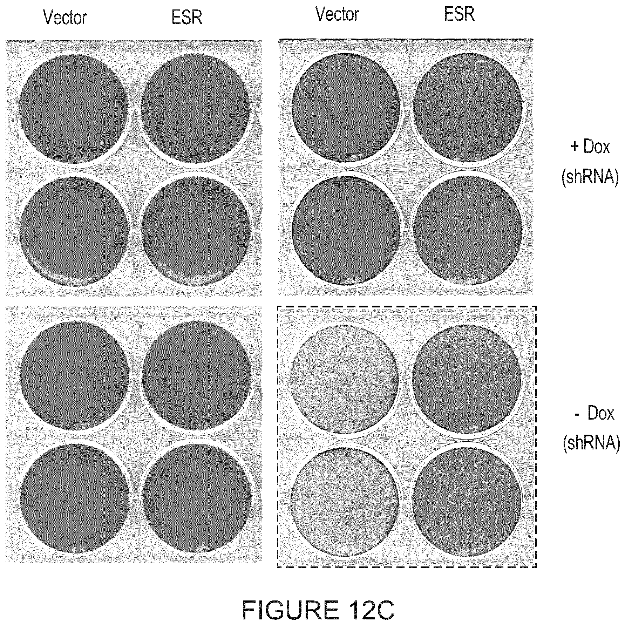

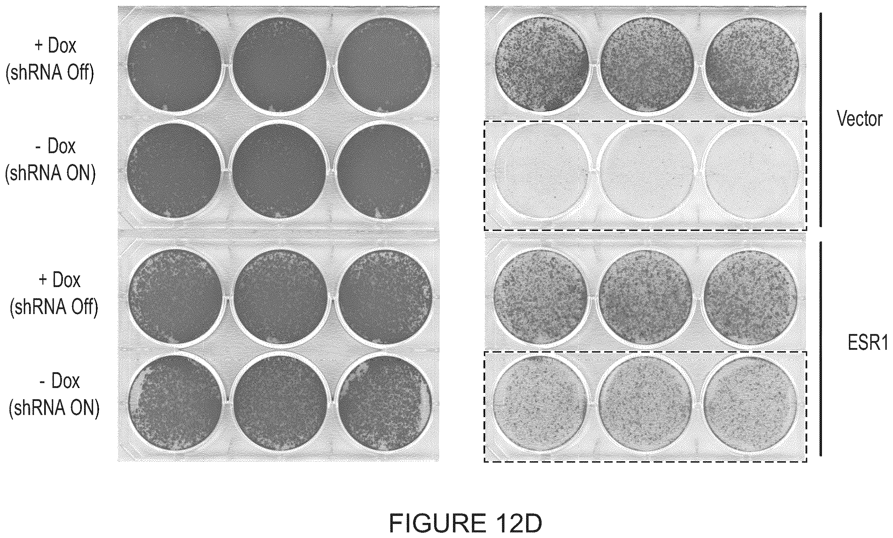

KAT6A knockdown led to a significant reduction in *ESR1* transcript **(****Figure 12B****)** and ERα protein **(****Figure 12A****)** as well as cell growth **(****Figure 12C****).** However, the ectopic expression of *ESR1* was sufficient to partially restore cell growth in the presence of KAT6A depletion. Moreover, re-expression of *ESR1* also partially restored recovery of T47D cells from cell cycle arrest by 300 nM palbociclib in combination with KAT6A depletion **(****Figure 12D****).** These data demonstrated that ERα plays an essential role in KAT6A-mediated growth inhibition of T47D cells alone and in combination with palbociclib.

### Example 10: KAT6 Small Molecule Inhibitors in Combination with Palbociclib Impacted Proliferation and Recovery of ER+ Breast Cancer Cells Lines

A dose-titration treatment utilizing small molecule KAT6 inhibitors demonstrated that the catalytic function of KAT6A was required for proliferation and recovery from palbociclib arrest of ER+ breast cancer cells, mimicking the results obtained with RNAi knockdown of KAT6A.

### Materials and Methods:

A dose-titration treatment was performed with COMPOUND C and COMPOUND D in T47D and ZR-75-1 cells (ATCC), according to the proliferation and palbociclib recovery colony formation assays as described in Example 4.

Cells were plated at low density into 6-well culture dishes and allowed to attach overnight at 37 °C, 5% CO₂. The next day, in parallel to the proliferation set, palbociclib was added at a final concentration of 300 nM to another set of plates, which served as the set for palbociclib recovery. To both proliferation and palbociclib recovery plates, DMSO or KAT6 inhibitor was added into each well at increasing doses. Medium and KAT6 inhibitor were refreshed twice a week. Cells were cultured for about two weeks of drug treatment until the DMSO-treated wells reached confluency. At the end of treatment, cells were washed once with phosphate-buffered saline and were fixed and stained with SRB for visualization followed by quantification. For the palbociclib recovery assay co-treated with KAT6 inhibitor, cells were treated with both palbociclib and KAT6 inhibitor for two weeks. At the end of the treatment, cells were washed three times with complete media to remove palbociclib and the KAT6 inhibitor and allowed to recover until DMSO-treated cells reached at least 80% confluency. Wells were fixed and stained with SRB for visualization followed by quantification. To quantify the stain, stained cells were dissolved in 2 mL of 10 mM Tris-HCl, pH 7.5 and shaken for 10 min on a shaker. Samples were then diluted with 10 mM Tris-HCl, pH 7.5, transferred to a 96-well microtiter plate in a volume of 100 µL and read on SpetraMax at 565 nm.

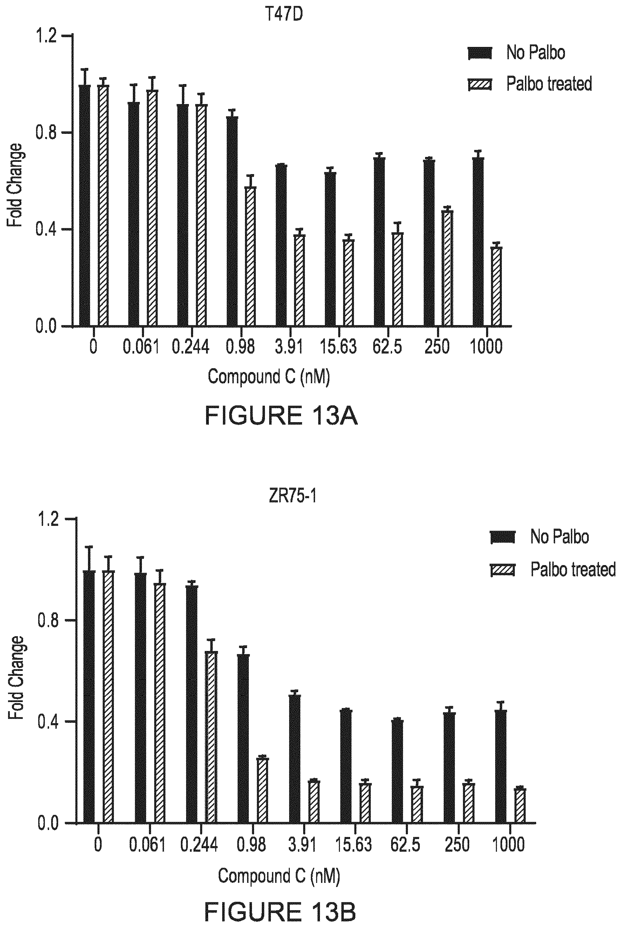

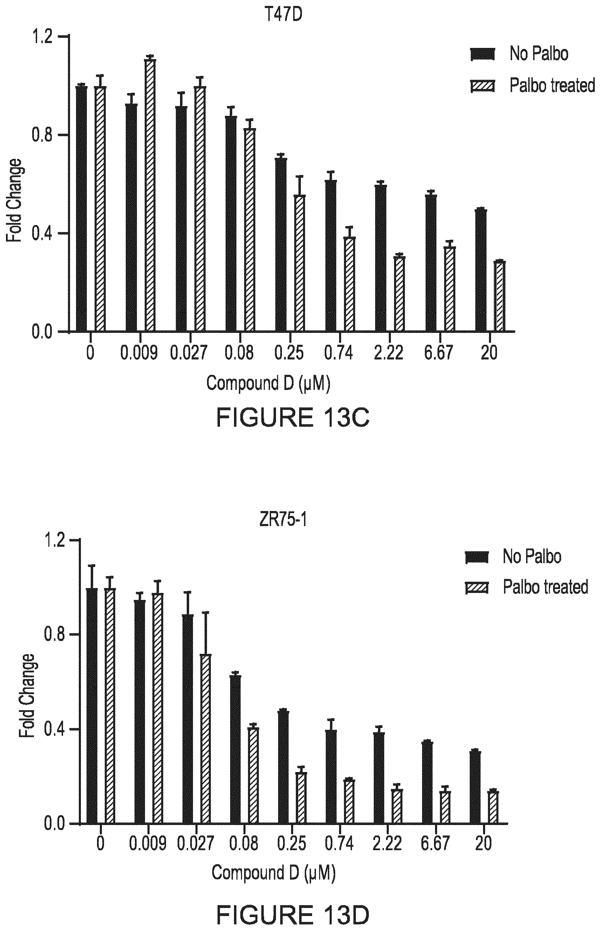

COMPOUND C was cytostatic to T47D cell growth when treated as a single agent, achieving maximal reduction of growth at about 40% **(****Figure13A****).** However, co-treatment of COMPOUND C with 300 nM palbociclib substantially enhanced its ability in blocking cell recovery from palbociclib-induced cell cycle arrest. In contrast, COMPOUND C demonstrated a strong dose-dependent cytotoxic effect on growth of ZR75-1 cells and achieved 50% growth inhibition at ~3.9 nM. When ZR75-1 cells were treated with increasing concentrations of COMPOUND C in the presence of 300 nM palbociclib, the anti-proliferative effect was significantly enhanced **(****Figure 13B****).** Similar cytostatic effects on T47D cells and cytotoxic effects on ZR75-1 were observed with COMPOUND D in the absence or presence of palbociclib, but with reduced potency compared to COMPOUND C **(****Figures 13C and 13D****).**

### Results:

Treatment with KAT6 small molecule inhibitors in combination with palbociclib demonstrated significantly improved potency in growth inhibition of these ER+ breast cancer cells compared to treatment with KAT6 small molecule inhibitors alone, indicating a strong dependence on catalytic function of KAT6A in cell proliferation. More importantly, these KAT6 inhibitors exhibited synergistic cooperation with palbociclib in blocking cell recovery from cell cycle arrest.

### Example 11: KAT6 Inhibitor Treatment Lead to Depletion of Estrogen Receptor Alpha in ER+ Breast Cancer Cells

A dose-titration treatment utilizing small molecule KAT6 inhibitors demonstrated that the KAT6 inhibitors down-regulated ERα expression in ER+ breast cancer cells, which mimicked the results obtained with RNAi knockdown of KAT6A.

### Materials and Methods:

A dose-titration treatment was performed with COMPOUND C and COMPOUND D in T47D cells (ATCC).

Cells were plated at 10,000 cells per well into 6-well culture dishes and allowed to attach overnight at 37 °C, 5% CO₂. The next day, either COMPOUND C or COMPOUND D was added to wells at increasing concentrations, in addition to DMSO as vehicle control. Cells were cultured for 14 days with medium and KAT6 inhibitor refreshed twice a week (every 3-4 days). At the end of drug treatment, one million cells were collected via trypsinization for protein lysates. Cells were lysed in 100 µL 1x LDS buffer diluted from 4x LDS buffer (ThermoFisher catalog # NP0007) with reducing agent (ThermoFisher catalog # NP0009). The lysates were subsequently sonicated and loaded onto a NuPAGE 4-12% Bis-Tris gel (ThermoFisher, catalog#NP0321BOX) followed by electrophoresis. Gels were then transferred onto a nitrocellulose membrane (iBlot2, ThermoFisher). Membranes were blocked and incubated overnight at 4 °C with following antibodies: anti-ERα (D8H8) Rabbit mAb (#8644, Cell Signaling) and anti-β-actin (8H10D10) mouse mAb (Cell Signaling, #3700S). Next day, after extensive washing in 1x TBST (with 0.1% Tween20) buffer (prepared by diluting 10 fold of the 10x Tris Buffered Saline (TBS) (BIO-RAD #1706435) and 100 fold of 10% Tween 20 (BIO-RAD #1610781), membranes were subsequently blotted with horseradish peroxidase-conjugated anti-rabbit secondary antibody (Cell Signaling #7074P2) for ERα and HRP-linked anti-mouse IgG secondary antibody (Cell Signaling #7076) for β-actin. Bands were visualized by an enhanced chemiluminescence (ECL) system (Thermo Scientific) per manufacturer's instructions. Subsequently, the portion of the membrane probed with anti-β-actin antibody was washed 3 times with 1xTBST+0.1% Tween20 to completely wash off ECL. This membrane was then probed with anti-Cyclin D1 rabbit mAb (Cell Signaling, #2922S) at room temperature for 2 hours on a shaker, followed by washing and probing with HRP-linked anti-rabbit secondary antibody (Cell Signaling #7074P2). ECL was performed to visualize bands on the film.

### Results:

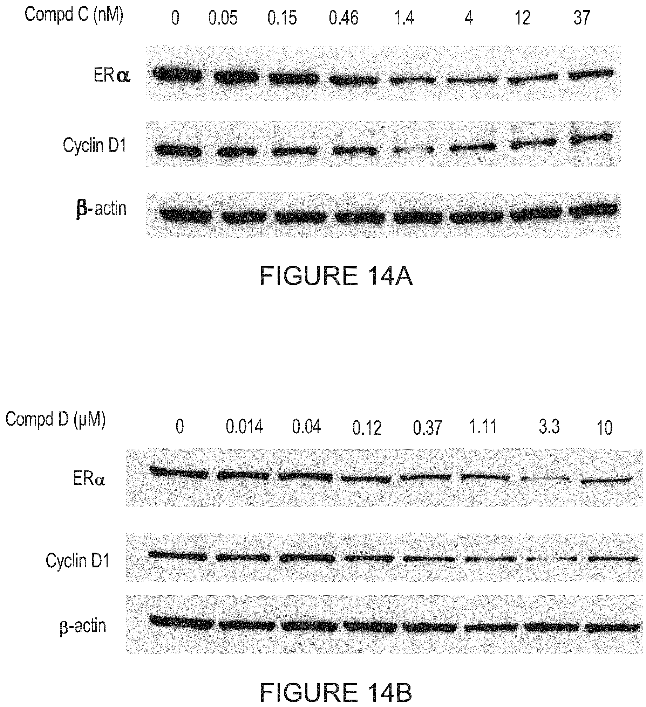

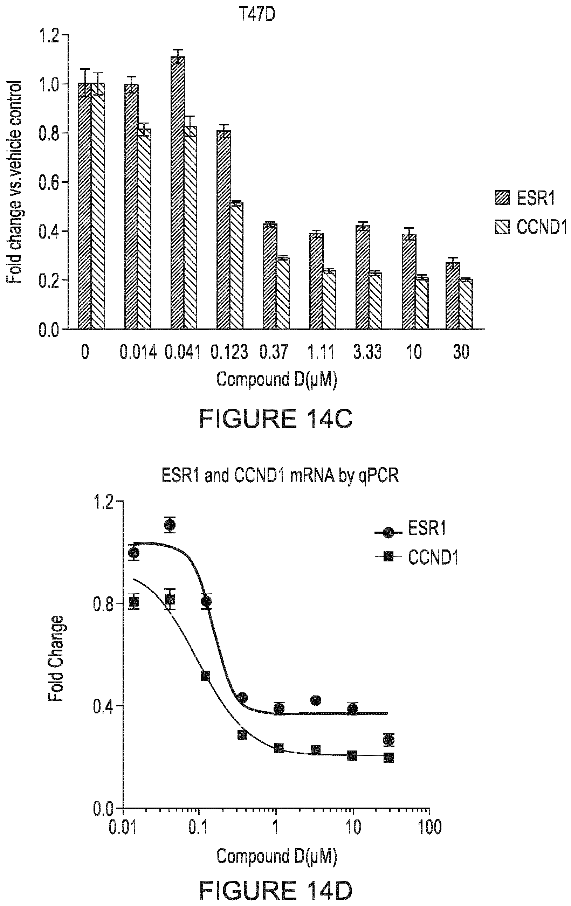

Treatment with COMPOUND C **(****Figure 14A****)** or COMPOUND D **(****Figure 14B****)** resulted in a dose-dependent depletion of both ERα and Cyclin D1, with COMPOUND C being more potent than COMPOUND D. Consistent with the reduction of both proteins, *ESR1* and *CCND1* (which encodes Cyclin D1), mRNA measured by qPCR also demonstrated a dose-dependent decrease by treatment with COMPOUND D **(****Figures 14C and 14D****).** This data showed that catalytic function of KAT6A was required for regulation of ERα expression, mimicking the results obtained with KAT6A knockdown.

### Example 12: KAT6 Inhibitor Synergized with Palbociclib against ER+ Breast Cancer Cells

The *in vitro* anti-proliferative effects of KAT6 small molecule inhibitors in combination with palbociclib were evaluated.

### Materials and Methods:

The following cell lines were obtained from ATCC (Manassas, VA) and grown from frozen stocks made within six months of original purchase: T47D, ZR75-1, MCF7, and CAMA1. Cells were maintained according to supplier guidelines.

Cells were seeded at 1000 cells per well for T47D, CAMA1 or MCF7 and 2000 cells per well for ZR75-1 into 96-well plates and allowed to attach overnight in RPMI-1640 media containing 10% FBS and 1x Pen/Strep. The following day, plates were treated with COMPOUND C, COMPOUND E or fulvestrant + palbociclib in a matrix across a range of concentrations. Individual KAT6 inhibitor was diluted four-fold from 1000 nM for T47D, CAMA1 and MCF7 and from 250 nM for ZR75-1, for a total 8 doses in the dilution series, in addition to two wells with DMSO-treated control, horizontally across the plate. Fulvestrant was diluted three-fold from 100 nM for T47D and CAMA1, 300 nM for ZR75-1 and from 1.235 nM for MCF7, for a total of 8 points in addition to DMSO control, horizontally across the plate. Palbociclib was diluted three-fold from 500 nM for T47D, CAMA1 and ZR75-1 and from 167 nM for MCF7, for a total of 5 points in addition to DMSO control, vertically across the plate. Media and compounds were refreshed every three days for a total of 10 days.

At the end of treatment, CyQUANT Direct (ThermoFisher, catalog# C35011) was used to stain the nucleus according to manufacturers' protocol and direct cell counts were made by the Celigo S imaging cytometer (Nexcelom BioScience). Percent of growth inhibition was calculated by normalizing the cell counts from a compound-treated well to the cell counts in DMSO/DMSO-control wells. Chalice Bioinformatics Software (Horizon Discovery) was used to calculate a Loewe Additivity score (Loewe, 1926) for an analysis of synergy. Synergy score was derived from the Excess Inhibition 2D matrix calculated according to the Loewe Additivity model in Chalice software (Lehar, 2009). A value of greater than 1 indicated the synergy of a combination while a value less than 1 indicated an antagonist effect.

### Results:

Combination treatment of COMPOUND C or COMPOUND E with palbociclib demonstrated synergistic inhibition of cell growth in T47D, ZR-75-1, CAMA1 and MCF7 cells **(Table 3).** Analysis of the combination data using the Loewe Additivity (ADD) model showed that the level of growth inhibition achieved with the combinations was synergistic. Synergy between COMPOUND C or COMPOUND E with palbociclib is equivalent to that observed with the palbociclib plus fulvestrant combination in T47D, CAMA1 or MCF7 cells, and also in ZR75-1 cells, in which fulvestrant was not synergistic with palbociclib **(Table 3).**

**Table 3**

| Cell Line | Fulvestrant + Palbociclib (10 day) | Compound C + Palbociclib (10 day) | Compound E + Palbociclib (10 day) | Compound E + Fulvestrant (13 day) | Compound E + Fulvestrant (10 day) |
|---|---|---|---|---|---|
| T47D | 4.19 | 4.39 | 3.89 | 6.47 | 2.69 |
| T47D | 2.99 | N/A | 4.38 | N/A | N/A |
| ZR75-1 | 0.77 | 3.01 | 2.90 | 1.45 | 1.64 |
| CAMA1 | 3.36 | 3.72 | 3.93 | 9.16 | 1.46 |
| MCF7 | 2.92 | 3.72 | 2.95 | 3.20 | 4.36 |

### Example 13: KAT6 Inhibitor Synergized with Fulvestrant against ER+ Breast Cancer Cells

The *in vitro* anti-proliferative effects of KAT6 small molecule inhibitors in combination with fulvestrant were evaluated.

### Materials and Methods:

The experimental design was provided in Example 12, except that fulvestrant was used to replace palbociclib. COMPOUND E was diluted four-fold from 1000 nM for a total 8 doses in the dilution series, in addition to two wells with DMSO-treated control, horizontally across the plate. Fulvestrant was diluted four-fold from 100 nM for T47D, CAMA1 and ZR75-1 and from 1.563 nM for MCF7, for a total of 5 points in addition to DMSO control, vertically across the plate. Media and compounds were refreshed every three days for 13 days.

At the end of treatment, CyQUANT Direct (ThermoFisher, catalog# C35011) was used to stain the nucleus according to manufacturers' protocol and direct cell counts were made by the Celigo S imaging cytometer (Nexcelom BioScience). Percent of growth inhibition was calculated by normalizing the cell counts from a compound-treated well to the cell counts in DMSO/DMSO-control wells. Chalice Bioinformatics Software (Horizon Discovery) was used to calculate a Loewe Additivity score (Loewe, 1926) for an analysis of synergy. Synergy score was derived from the Excess Inhibition 2D matrix calculated according to the Loewe Additivity model in Chalice software (Lehar, 2009). A value of greater than 1 indicated the synergy of a combination while a value less than 1 indicated an antagonist effect.

### Results:

Combination treatment of COMPOUND E with fulvestrant demonstrated synergistic effects in inhibiting proliferation of all four cell lines, T47D, ZR-75-1, CAMA1 and MCF7 **(Table 3).**

### Example 14: KAT6 Inhibitor Synergized with Selective CDK4 Inhibitor against ER+ Breast Cancer Cells

The *in vitro* anti-proliferative effects of KAT6 small molecule inhibitors in combination with the selective CDK4 inhibitor, COMPOUND F, were evaluated.

### Materials and Methods:

According to the procedure of Example 12, cells were seeded at 1000 cells per well for T47D, CAMA1 or MCF7 and 2000 cells per well for ZR75-1 into 96-well plates and allowed to attach overnight in RPMI-1640 media containing 10% FBS and 1x Pen/Strep. The following day, plates were treated with fulvestrant, COMPOUND C or COMPOUND E with COMPOUND F in a matrix across a range of concentrations. Individual KAT6 inhibitor was diluted four-fold from 1000 nM for T47D, CAMA1 and MCF7 and from 250 nM for ZR75-1, for a total 8 doses in the dilution series, in addition to two wells with DMSO-treated control, horizontally across the plate. Fulvestrant was diluted three-fold from 300 nM for ZR75-1, 100 nM for T47D and CAMA1, and from 1.235 nM for MCF7, for a total of 8 points in addition to DMSO control, horizontally across the plate. Compound F was diluted three-fold from 500 nM for T47D, CAMA1 and ZR75-1 and from 167 nM for MCF7, for a total of 5 points in addition to DMSO control, vertically across the plate.

Media and compounds were refreshed every three days for 10 days. At the end of treatment, CyQUANT Direct (ThermoFisher, catalog# C35011) was used to stain the nucleus according to manufacturers' protocol and direct cell counts were made by the Celigo S imaging cytometer (Nexcelom BioScience). Percent of growth inhibition was calculated by normalizing the cell counts from a compound-treated well to the cell counts in DMSO/DMSO-control wells. Chalice Bioinformatics Software (Horizon Discovery) was used to calculate a Loewe Additivity score (Loewe, 1926) for an analysis of synergy. Synergy score **(Table 4)** was derived from the Excess Inhibition 2D matrix calculated according to the Loewe Additivity model in Chalice software (Lehar, 2009). A value of greater than 1 indicated the synergy of a combination while a value less than 1 indicated an antagonist effect.

### Results:

Combination treatment of COMPOUND C or COMPOUND E with selective CDK4 inhibitor, COMPOUND F, synergistically inhibited cell growth of T47D, ZR-75-1, CAMA1 and MCF7 cells **(Table 4).**

**Table 4**

| Cell line | Fulvestrant + Compound F | Compound C + Compound F | Compound E + Compound F |
|---|---|---|---|
| T47D | 5.77 | 3.00 | 2.82 |
| ZR75-1 | 0.14 | 2.83 | 2.80 |
| CAMA1 | 2.50 | 2.64 | 2.69 |
| MCF7 | 3.20 | 2.73 | 2.80 |

### Example 15: KAT6 Inhibitor and Palbociclib Combination Overcame Resistance Mechanisms Resulting from ESR1 mutations in ER+ Breast Cancer Cells

Direct inhibition of *ESR1* transcription and ER-dependent genes was investigated to determine whether KAT6 inhibitors as a single agent or in combination with palbociclib overcame mutations in estrogen receptor-alpha (ERα), a primary resistance mechanism to endocrine therapies.

### Materials and Methods:

Gene editing by CRISPR / CAS9 was utilized to introduce clinically relevant *ESR1* mutations Y537S and D538G individually and together, into the endogenous *ESR1* gene under its native promoter in T47D cells (ATCC).

### Step 3: Characterization of T47D ESR1 mutant clones in drug treatment in vitro

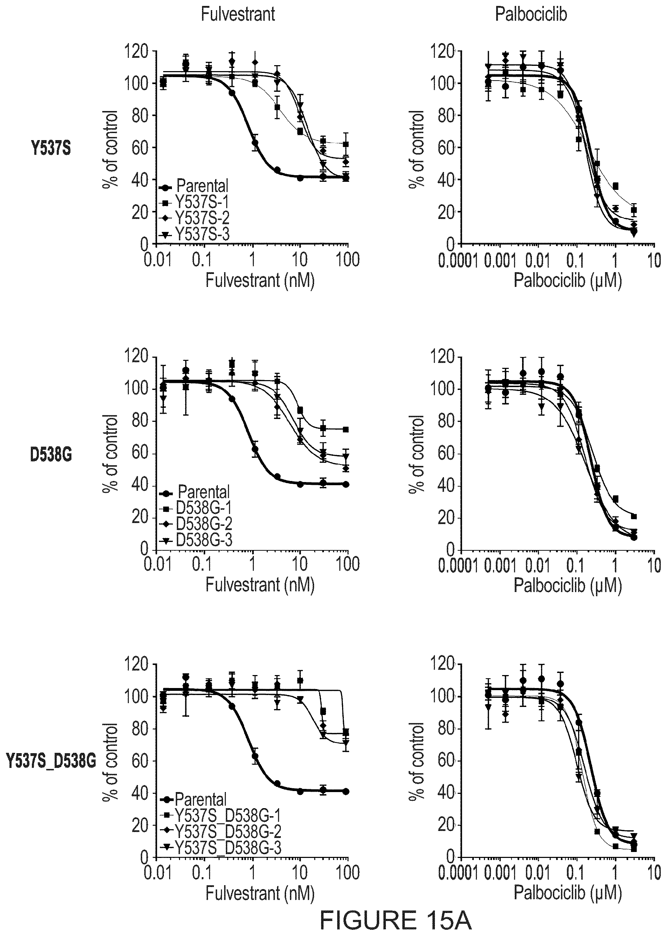

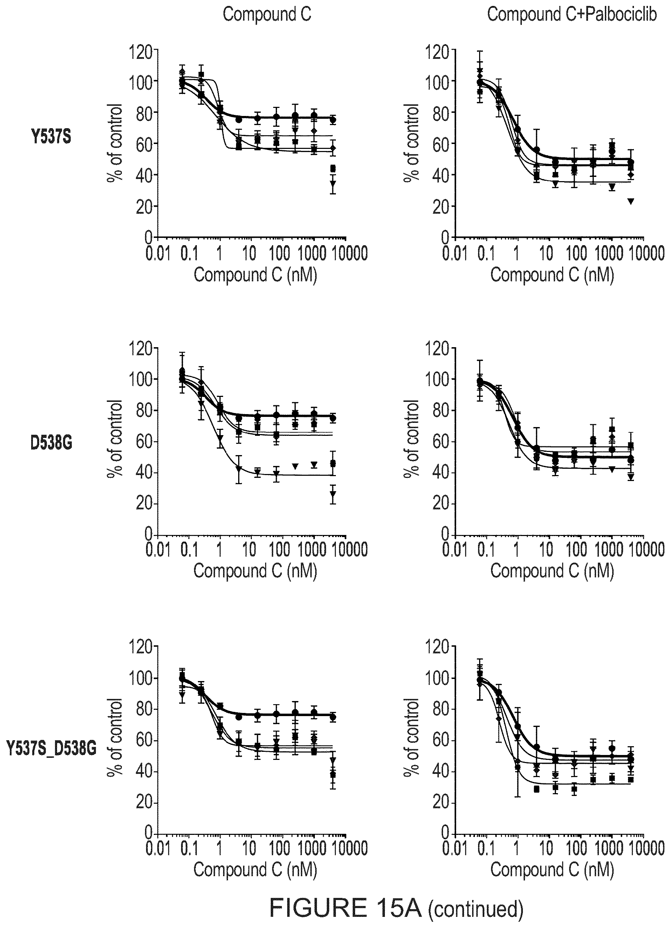

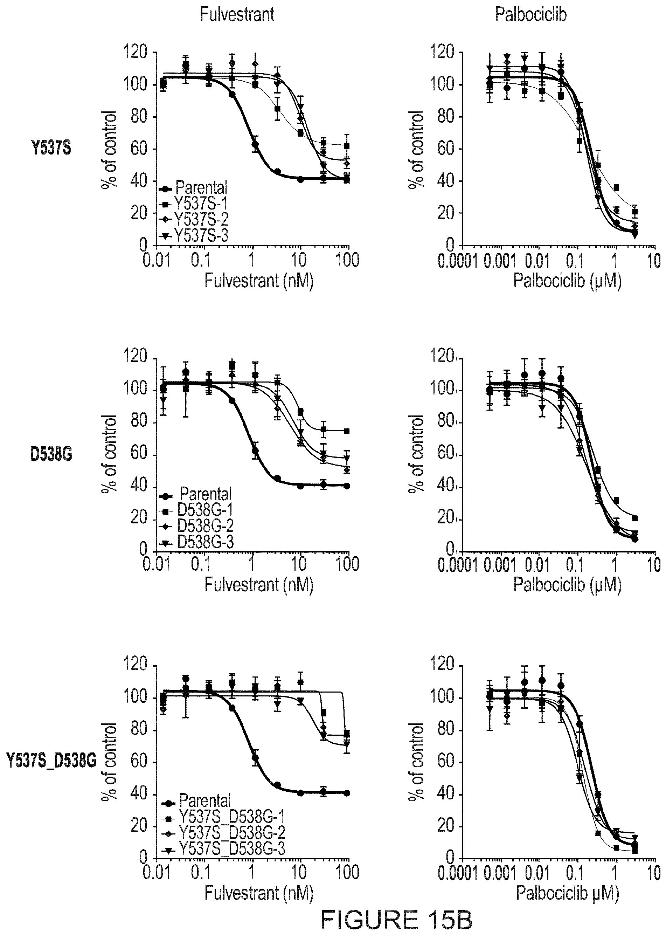

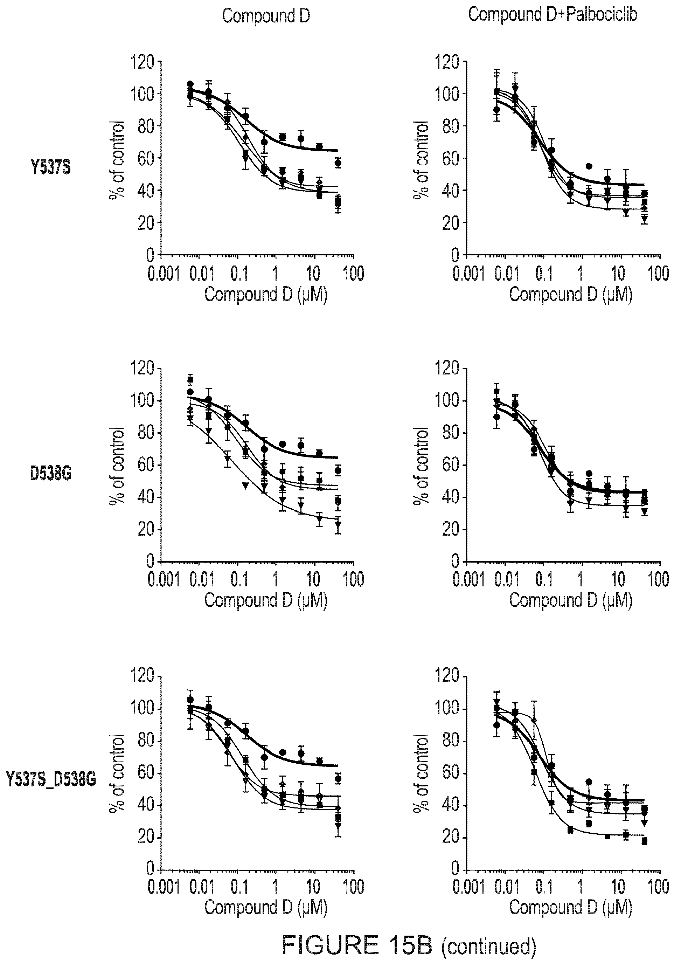

Each mutant clone was evaluated by its response to fulvestrant and KAT6 inhibitors **(Table 5).** Clones with Y537S, D538G, or Y537S/D538G demonstrated resistance to fulvestrant with 3 -17, 3 -11 and 22 -100-fold increase respectively in cell proliferation IC50 relative to the parental cell line **(****Figure 15A** **and** **15B****).** In contrast, clones with Y537S, D538G, or Y537S/D538G mutations demonstrated equivalent sensitivity to palbociclib **(****Figure 15A** **and** **15B****),** and enhanced sensitivity to KAT6 inhibitor COMPOUND C **(****Figure 15A****)** or COMPOUND D **(****Figure 15B****),** when compared to the parental cell line. In addition, these mutant clones remained sensitive to palbociclib in combination with COMPOUND C **(****Figure 15A****)** and palbociclib in combination with COMPOUND D **(****Figure 15B****).**

**Table 5**

| **Name** | **Clone ID** |
|---|---|
| Parental | T47D |
| Y537S-1 | SC#1-4 |
| Y537S-2 | SC#1-11 |
| Y537S-3 | SC#4-11 |
| D538G-1 | SC#2-2 |
| D538G-2 | SC#2-5 |
| D538G-3 | SC#2-13 |
| Y537S_D538G-1 | SC#3-6 |
| Y537S_D538G-2 | SC#3-9 |
| Y537S_D538G-3 | SC#3-12 |

### Results:

T47D cells harboring the *ESR1* mutations investigated above, displayed reduced sensitivity to fulvestrant, yet remained sensitive to KAT6 inhibitors, similar to T47D parental cells, as a single agent and in combination with palbociclib *in vitro.* These results demonstrate that KAT6 inhibitors as a single agent and in combination with palbociclib overcame the mechanism of resistance that emerged by ESR1-mutations.

### Example 16: KAT6 Inhibitor Synergized with Palbociclib against ER+ Breast Cancer Cells Harboring Clinically Relevant ESR1 Mutations

A palbociclib combination study was performed to determine whether knock-in of clinically relevant *ESR1* mutations altered synergy between KAT6 inhibitor and palbociclib in ER+ breast cancer cells.

### Materials and Methods:

A palbociclib combination study with *ESR1* mutant clones (Y537S, D538G and dual Y537S_D538G) was performed to assess synergy between palbociclib and KAT6 inhibitors, as described in Example 12.

Cells were seeded at 1000 cells per well into 96-well plates and allowed to attach overnight in RPMI-1640 media containing 10% FBS and 1x Pen/Strep. The following day, plates were treated with fulvestrant, COMPOUND C or COMPOUND E with palbociclib in a matrix across a range of concentrations. Each KAT6 inhibitor was diluted four-fold from 1000 nM for a total 8 doses in the dilution series, in addition to two wells with DMSO-treated control, horizontally across the plate. Fulvestrant was diluted three-fold from 100 nM for a total of 8 points in addition to DMSO control, horizontally across the plate. Palbociclib was diluted three-fold from 500 nM for a total of 5 points in addition to DMSO control, vertically across the plate. Media and compounds were refreshed every three days for a total of 10 days.

At the end of treatment, CyQUANT Direct (ThermoFisher, catalog# C35011) was used to stain the nucleus according to manufacturers' protocol and direct cell counts were made by the Celigo S imaging cytometer (Nexcelom BioScience). Percent of growth inhibition was calculated by normalizing the cell counts from a compound-treated well to the cell counts in DMSO/DMSO-control wells. Chalice Bioinformatics Software (Horizon Discovery) was used to calculate a Loewe Additivity score (Loewe, 1926) for an analysis of synergy. Synergy score was derived from the Excess Inhibition 2D matrix calculated according to the Loewe Additivity model in Chalice software (Lehar, 2009). A value of greater than 1 indicated the synergy of a combination while a value less than 1 indicated an antagonist effect.

### Results:

There was a decrease in synergy between fulvestrant and palbociclib when compared to the parental wild type, due to decreased sensitivity to fulvestrant of the mutant clones. However, the *ESR1* mutations did not affect the synergy between palbociclib and KAT6 inhibitor as indicated by the synergy scores **(Table** 6).

These data demonstrated that a KAT6 inhibitor in combination with palbociclib offered a mechanism to overcome clinically relevant resistance to endocrine therapy for the treatment of breast cancer.

**Table 6**

| **Cell line** | **Fulvestrant + Palbociclib** | **Compound C + Palbocilcib** | **Compound D + Palbociclib** | **Compound E + Palbociclib** |
|---|---|---|---|---|
| Parental | 2.99 | 3.38 | 2.23 | 4.38 |
| Y537S | 1.88 | 4.00 | 3.35 | 3.54 |
| D538G | 1.56 | 5.66 | 4.10 | 7.13 |
| Y537S_D538G (1) | 1.38 | 5.35 | 2.94 | 5.53 |
| Y537S_D538G (2) | 1.34 | 4.06 | 2.44 | 4.59 |

### Example 17: ESR1 Expression Downregulated by Palbociclib in ER+ Breast Cancer Cells

*ESR1* gene expression, a molecular mechanism underlying the synergy between KAT6 inhibitor and palbociclib, was further investigated.

### Materials and Methods:

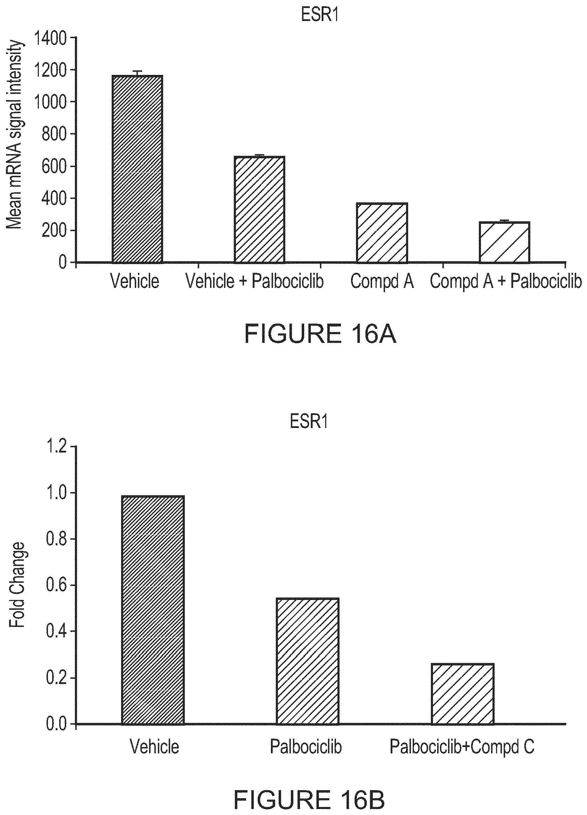

As described in Examples 6 and 7, a gene expression profiling study was performed in T47D cells by microarray **(****Figure 16A****)** and RNA-Seq **(****Figure 16B****).**

T47D (ATCC) and MCF7 cells were separately treated with palbociclib as a single agent and in combination with COMPOUND C. Cells were seeded into 6-well culture plates and allowed to attach overnight at 37 °C, 5% CO₂. The following day, palbociclib was added to culture medium at 500 nM with DMSO or 20 nM COMPOUND C. Medium was refreshed every 3 days. At 3, 6 and 8 days of treatment, cells were harvested for protein and RNA lysates.

Protein samples were subjected to SDS-PAGE electrophoresis and Western Blotting to probe for Phospho-Rb (Ser780) (Cell Signaling catalog # 9307S), Rb (Cell Signaling catalog # 9309S), ERα (Cell Signaling catalog# 2922S), and β-actin (Cell Signaling #3700).

### Results:

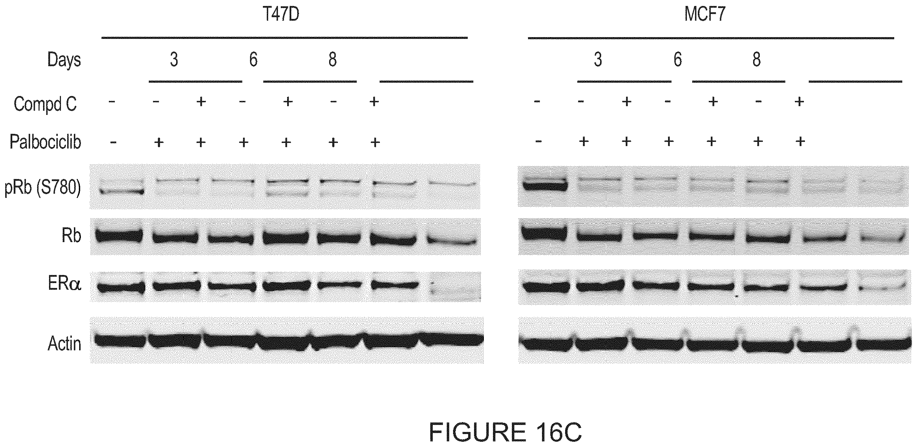

Palbociclib effectively suppressed phosphorylation of Rb protein in both cell lines at all three time-points **(****Figure 16C****).** In addition, consistent with the gene profiling studies by microarray or RNA-Seq, palbociclib treatment led to a gradual decrease in ERα protein level from day 3 to 8 and the reduction reached about 50% after 8 days of treatment in both cell lines.

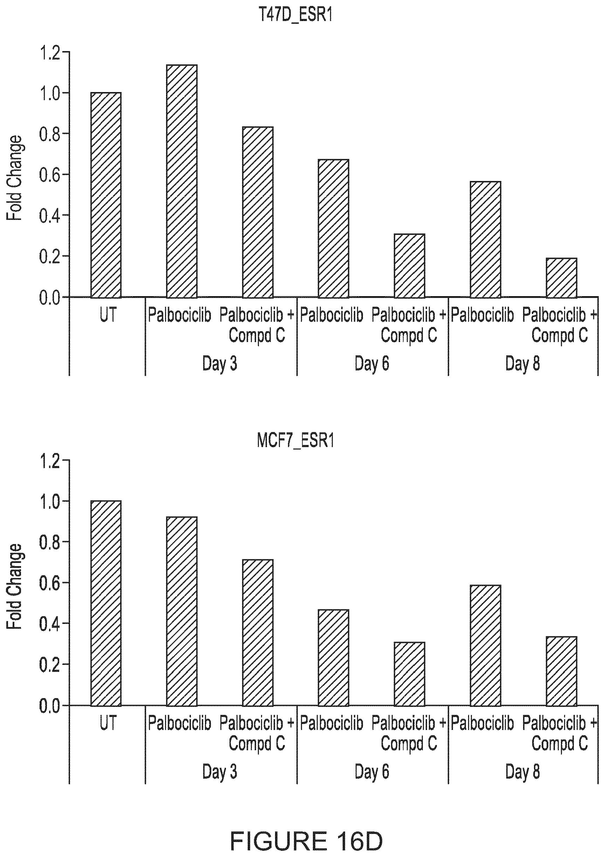

Palbociclib and COMPOUND C combination treatment resulted in a significant reduction of ERα level **(****Figure 16C****).** The decrease in ERα protein was consistent with the decrease in *ESR1* mRNA level as measured by qPCR **(****Figure 16D****).**

The results demonstrated a reduction in *ESR1* mRNA by palbociclib as a single agent and by KAT6 inhibitor as a single agent, with the KAT6 inhibitor demonstrating a greater reduction. Palbociclib in combination with a KAT6 inhibitor demonstrated a profound reduction of the *ESR1* transcript **(****Figure 16A****)** correlating with their synergistic anti-tumor combination activity.

### Example 18: KAT6 Inhibitor Demonstrated In Vivo Combinatorial Benefit with Palbociclib and Fulvestrant in ER+ Breast Cancer Patient-Derived Xenograft (PDX) Models

In vivo studies were performed in two clinically-relevant, patient-derived xenograft (PDX) models of ER+ breast cancer to determine whether a KAT6 inhibitor provided additional anti-tumor efficacy benefit when used in combination with palbociclib and fulvestrant.

### Materials and Methods:

Combination studies with palbociclib, fulvestrant and the KAT6 inhibitor, Compound E (KAT6i), were performed in two ER+ breast cancer PDX models: ST340 and ST941. ST340 was wildtype for *ESR1.* ST941 harbored the activating mutation Y537S in the ligand-binding domain (LBD) of *ESR1,* associated with resistance to endocrine therapy in the clinic. Both studies were conducted by XenoSTART (San Antonio, Texas), where 6-12 week-old female athymic nude mice (JAX, stock #007850) were subcutaneously implanted with ~70mg tumor fragments. Animals were supplemented with exogenous estradiol ad libitum via drinking water throughout the duration of the studies. Tumor measurements and animal weights were captured twice per week, and animals were stratified and enrolled into the study when the mean tumor volume (TV, formula: width² x length x 0.5) reached 150-300mm³.

Each study contained eight treatment groups of n=10 animals per group. The groups were as follows: 1) Vehicle (5% DMSO / 40% PEG300 / 55% 1x PBS), 2) Compound E, 3) palbociclib, 4) fulvestrant, 5) palbociclib + fulvestrant, 6) Compound E + fulvestrant, 7) Compound E + palbociclib, and 8) Compound E + palbociclib + fulvestrant. Group details for dose levels, routes, and regimens are listed in **Table 7.** Compound and formulation details are listed in **Table 8.** Animals remained on study and were continued on treatments until the mean tumor volume of each group reached ~1500mm³. At that point, the final dose was administered, animals were sacrificed, and terminal samples were collected. Blood microsamples were collected from each group for pharmacokinetics (PK) analysis at 0, 3, and 7 hrs (n=3/timepoint) on day 14 of dosing and again on the terminal day of dosing.

Tumor growth inhibition (TGI) was calculated in relation to the vehicle group on day 21 and day 17 for ST340 and ST941, respectively, where TGI Delta = 1 - (Treatedₜ - Treated₀)/(Referenceₜ - Reference₀). Analysis of covariance (ANCOVA) was used to determine statistical significance when comparing percent TGI between groups. Changes in bodyweights were calculated in relation to starting bodyweights at study initiation (day 0 of treatment).

**Table 7. Study Design and Dosing Details**

| **Group** | **N** | **Treatment** | **Dose (mg/kg)** | **Route** | **Schedule** |
|---|---|---|---|---|---|
| 1 | 10 | Vehicle | -- | PO | QD |
| 2 | 10 | Compound E | 3 | PO | QD |
| 3 | 10 | Palbociclib | 10 | PO | BID |
| 4 | 10 | Fulvestrant | 10 | SC | (Q7Dx4 + LD*) x3 |
| 5 | 10 | Palbociclib | 10 | PO | BID |
| | | Fulvestrant | 10 | SC | (Q7Dx4 + LD*) x3 |
| 6 | 10 | Compound E | 3 | PO | QD |
| | | Fulvestrant | 10 | SC | (Q7Dx4 + LD*) x3 |
| 7 | 10 | Compound E | 3 | PO | QD |
| | | Palbociclib | 10 | PO | BID |
| 8 | 10 | Compound E | 3 | PO | QD |
| | | Palbociclib | 10 | PO | BID |
| | | Fulvestrant | 10 | SC | (Q7Dx4 + LD*) x3 |

| | | | | | |
|---|---|---|---|---|---|
| * LD= loading dose to be administered on 3^{rd} day of each new dosing cycle (dosing cycle = 1 month). | | | | | |

**Table 8. Test Article and Formulation**

| **Test Article** | **Vehicle** |
|---|---|
| Compound E | 5% DMSO / 40% PEG300 / 55% 1x PBS |
| Fulvestrant | Peanut oil |
| Palbociclib | 0.5% MC A4M in water |

### Results:

TGI analysis showed that fulvestrant only resulted in ~20% growth inhibition compared to vehicle and was not significant in either ST340 or ST941 **(Table 9, 12),** indicating the models were largely insensitive to ER antagonism. Because ST340 originated from a patient who had prior treatment with a HER2 inhibitor, and ST941 contained an *ESR1* activating mutation, the lack of response to fulvestrant aligned with the prior molecular characteristics of these models. Both models were also only moderately responsive to CDK4/6 inhibition by palbociclib, which resulted in ~40% TGI compared to vehicle. The effects of single-agent treatment with Compound E were also moderate, with slightly better efficacy seen in ST340 (55%, **Table 9)** than in ST941 (35%, **Table 12).**

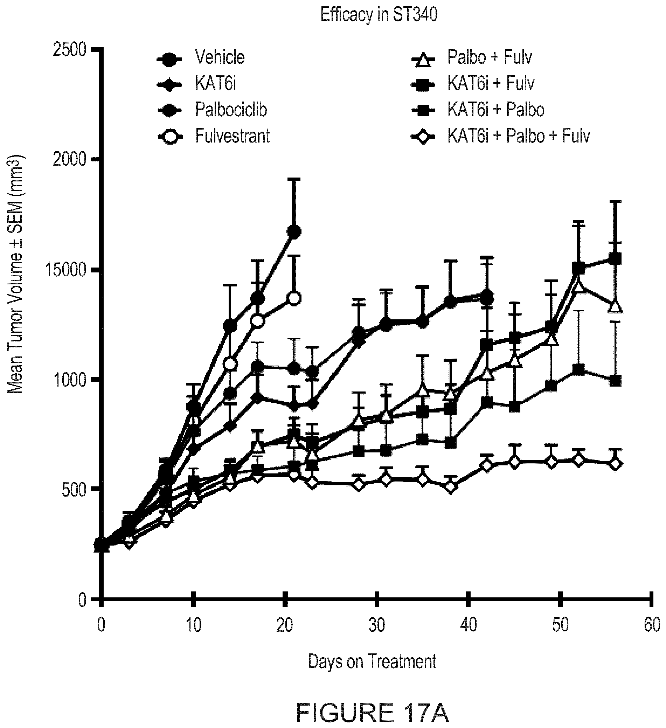

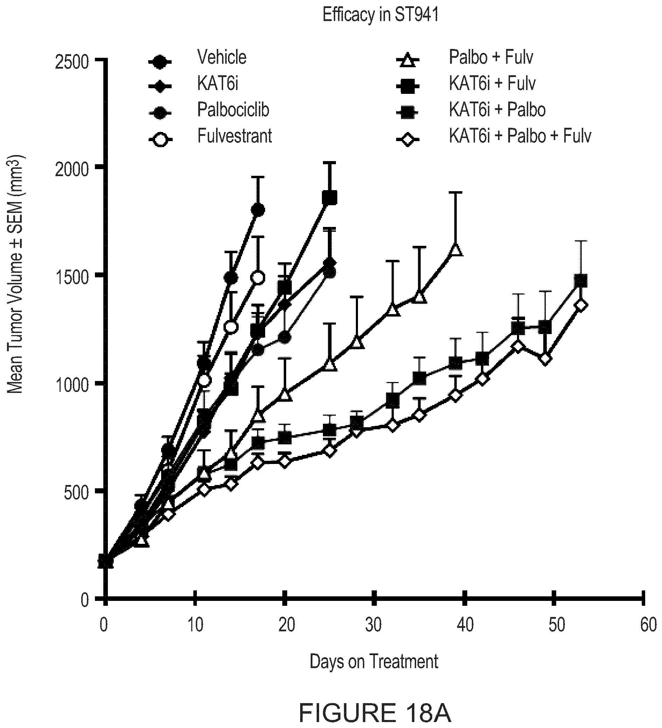

While each of the three agents exhibited only low to moderate efficacy when used as single agents, greater tumor growth inhibition and growth delay was observed in the combination groups **(****Figure 17A****, 18A).** Of the double combo groups, Compound E + palbociclib had the strongest anti-tumor effects and resulted in 75% and 67% TGI in ST340 and ST941, respectively **(Table 9, 12).** Both studies also indicated that Compound E (KAT6i) + fulvestrant was the least effective combination and that fulvestrant did not drive much additional efficacy in either model **(****Figure 17A****, 18A).** However, efficacy was further enhanced when fulvestrant was administered as part of the triple combination of Compound E + palbociclib + fulvestrant, showing strongest anti-tumor efficacy of 78% inhibition in ST340 and 72% inhibition in ST941. Although the triple combo was superior, it did not reach statistical significance when compared to Compound E + palbociclib or palbociclib + fulvestrant **(Table 10, 13).**

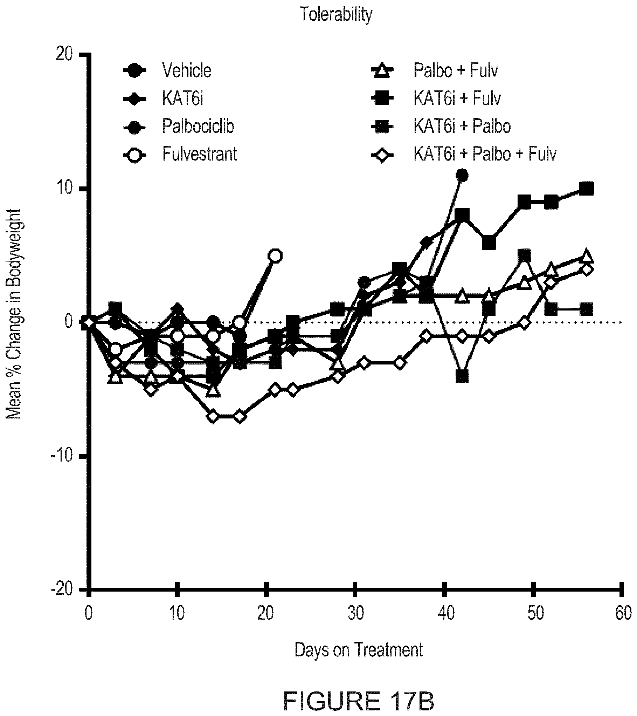

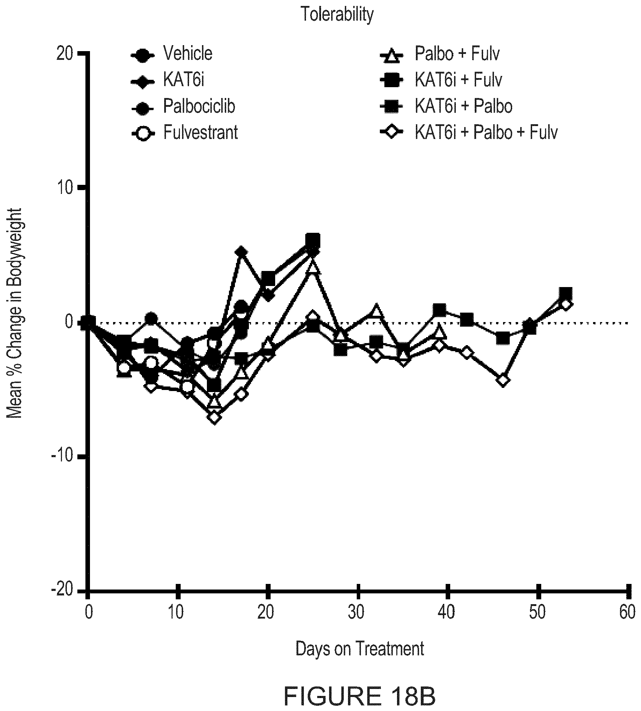

Health and bodyweight monitoring in both studies indicated that mean weight loss did not exceed 10% in any of the treatment groups **(****Figure 17B****,** **18B****, Table 11, 14).** All combinations of Compound E, palbociclib, and fulvestrant were well-tolerated, and drug-related deaths were not observed. Overall, these results demonstrated that combination treatments with the KAT6 inhibitor, Compound E were tolerated, as well as efficacious compared to vehicle and compared to single-agent standard of care in models that were relatively resistant to ER or CDK4/6 inhibition.

**Table 9. PDX ST340 TGI results compared to Vehicle (Group 1)**

| **Group** | **Tₓ** | **Mean ± SEM (day 21)** | **%TGI Delta** | **p-value (ANCOVA)** | **Significant** |
|---|---|---|---|---|---|
| 1 | Vehicle | 1673 ± 236 | -- | -- | -- |
| 2 | Compound E | 882 ± 86 | 55.4% | 0.000282 | Yes |
| 3 | Palbociclib | 1051.4 ± 134 | 43.7% | 0.00466 | Yes |
| 4 | Fulvestrant | 1370 ± 195 | 21.3% | 0.121326 | No |
| 5 | Palbociclib + Fulvestrant | 720 ± 70 | 66.8% | 3.39E-06 | Yes |
| 6 | Compound E + Fulvestrant | 750 ± 76 | 64.8% | 7.40E-06 | Yes |
| 7 | Compound E + Palbociclib | 605 ± 85 | 74.9% | 1.40E-08 | Yes |
| 8 | Compound E + Palbociclib + Fulvestrant | 568 ± 56 | 77.6% | 8.11E-09 | Yes |

**Table 10. PDX ST340 statistical significance of cross-group comparisons**

| **Comparison (day 21)** | | **p-value (ANCOVA)** | **Significant** |
|---|---|---|---|
| Compound E | Compound E + Fulvestrant | 0.301643 | No |
| Compound E | Compound E + Palbociclib | 0.010465 | Yes |
| Compound E | Compound E + Palbociclib + Fulvestrant | 0.007319 | Yes |
| Palbociclib | Palbociclib + Fulvestrant | 0.031959 | Yes |
| Palbociclib | Compound E + Palbociclib | 0.000647 | Yes |
| Palbociclib | Compound E + Palbociclib + Fulvestrant | 0.000421 | Yes |
| Compound E + Palbociclib + Fulvestrant | Palbociclib + Fulvestrant | 0.134797 | No |
| Compound E + Palbociclib + Fulvestrant | Compound E + Fulvestrant | 0.089556 | No |
| Compound E + Palbociclib + Fulvestrant | Compound E + Palbociclib | 0.89567 | No |

**Table 11. PDX ST340 change in bodyweight**

| **Group** | **Treatment** | **Day 21 mean change in bodyweight** | **Maximum mean bodyweight** loss | |
|---|---|---|---|---|
| 1 | Vehicle | +5% | -1% | Day 17 |
| 2 | Compound E | -2% | -4% | Day 3 |
| 3 | Palbociclib | -2% | -3% | Day 17 |
| 4 | Fulvestrant | +5% | -2% | Day 3 |
| 5 | Palbociclib + Fulvestrant | -1% | -5% | Day 14 |
| 6 | Compound E + Fulvestrant | -1% | -4% | Day 14 |
| 7 | Compound E + Palbociclib | -3% | -3% | Day 21 |
| 8 | Compound E + Palbociclib + Fulvestrant | -5% | -7% | Day 17 |

**Table 12. PDX ST941 TGI results compared to Vehicle (Group 1)**

| **Group** | **Treatment** | **Mean ± SEM (day 17)** | **%TGI Delta** | **p-value (ANCOVA)** | **Significant** |
|---|---|---|---|---|---|
| 1 | Vehicle | 1804 ± 151 | -- | -- | -- |
| 2 | Compound E | 1232 ± 129 | 35.2% | 0.079115 | No |
| 3 | Palbociclib | 1156 ± 152 | 39.8% | 0.048799 | Yes |
| 4 | Fulvestrant | 1490 ± 190 | 19.4% | 0.161559 | No |
| 5 | Palbociclib + Fulvestrant | 853 ± 132 | 58.4% | 5.87E-05 | Yes |
| 6 | Compound E + Fulvestrant | 1245 ± 81 | 34.4% | 0.099042 | No |
| 7 | Compound E + Palbociclib | 724 ± 63 | 66.6% | 0.000796 | Yes |
| 8 | Compound E + Palbociclib + Fulvestrant | 632 ± 39 | 72.0% | 0.000279 | Yes |

**Table 13. PDX ST941 statistical significance of cross-group comparisons**

| **Comparison (day 17)** | | **p-value (ANCOVA)** | **Significant** |
|---|---|---|---|
| Compound E | Compound E + Fulvestrant | 0.899396 | No |
| Compound E | Compound E + Palbociclib | 0.06705 | No |
| Compound E | Compound E + Palbociclib + Fulvestrant | 0.031854 | Yes |
| Palbociclib | Palbociclib + Fulvestrant | 0.019082 | Yes |
| Palbociclib | Compound E + Palbociclib | 0.112584 | No |
| Palbociclib | Compound E + Palbociclib + Fulvestrant | 0.056768 | Yes |
| Compound E + Palbociclib + Fulvestrant | Palbociclib + Fulvestrant | 0.64545 | No |
| Compound E + Palbociclib + Fulvestrant | Compound E + Fulvestrant | 0.023433 | Yes |
| Compound E + Palbociclib + Fulvestrant | Compound E + Palbociclib | 0.743358 | No |

**Table 14. PDX ST941 change in bodyweight**

| **Group** | **Treatment** | **Day 17 mean change in bodyweight** | **Maximum mean bodyweight loss** | |
|---|---|---|---|---|
| 1 | Vehicle | +1% | -4% | Day 7 |
| 2 | Compound E | +5% | -4% | Day 11 |
| 3 | Palbociclib | -1% | -3% | Day 14 |
| 4 | Fulvestrant | 0% | -5% | Day 11 |
| 5 | Palbociclib + Fulvestrant | -4% | -6% | Day 14 |
| 6 | Compound E + Fulvestrant | 0% | -5% | Day 14 |
| 7 | Compound E + Palbociclib | -3% | -3% | Day 17 |
| 8 | Compound E + Palbociclib + Fulvestrant | -6% | -7% | Day 14 |

## Claims

1. A lysine acetyltransferase 6 (KAT6) inhibitor for use in a method for treating cancer wherein said method comprises administering to a patient in need thereof an amount of said lysine acetyltransferase 6 (KAT6) inhibitor and
a) an amount of a cyclin-dependent kinase 4 (CDK4) inhibitor; or
b) an amount of an antiestrogen; or
c) an amount of a CDK4 inhibitor and an amount of an antiestrogen;
wherein the amounts together are therapeutically effective in treating cancer;
wherein the cancer is breast cancer;
wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; wherein the antiestrogen is fulvestrant; and
wherein the CDK4 inhibitor is either 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof.

2. A lysine acetyltransferase 6 (KAT6) inhibitor for use in a method of overcoming clinical resistance to endocrine therapy, wherein said method comprises administering to a patient in need thereof an amount of said KAT6 inhibitor and an amount of a CDK4 inhibitor, wherein the amounts together are therapeutically effective in overcoming clinical resistance to endocrine therapy;
wherein the endocrine therapy is used for treating breast cancer;
wherein the KAT6 inhibitor is 2-methoxy-*N*-{4-methoxy-6-[(1*H*-pyrazol-1-yl)methyl]-1,2-benzoxazol-3-yl}benzene-1-sulfonamide, or a pharmaceutically acceptable salt thereof; and
wherein the CDK4 inhibitor is either 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof or palbociclib, or a pharmaceutically acceptable salt thereof.

3. The lysine acetyltransferase 6 (KAT6) inhibitor for use according to claim 1 or claim 2, wherein the CDK4 inhibitor is 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-dideoxy-D-*threo*-pentitol, or a pharmaceutically acceptable salt thereof.

4. The lysine acetyltransferase 6 (KAT6) inhibitor for use according to claim 1 or claim 2, wherein the CDK4 inhibitor is palbociclib, or a pharmaceutically acceptable salt thereof.

5. The lysine acetyltransferase 6 (KAT6) inhibitor for use according to any one of claims 1 to 4, wherein the patient is human.

6. The lysine acetyltransferase 6 (KAT6) inhibitor for use according to any one of claims 1 or 2, wherein the breast cancer is locally advanced or metastatic estrogen receptor positive (ER+) breast cancer.

7. The lysine acetyltransferase 6 (KAT6) inhibitor for use according to any one of claims 1 or 2, wherein the breast cancer is locally advanced or metastatic estrogen receptor positive (ER+), human epidermal growth factor receptor 2 negative (HER2-) breast cancer.

## Patentansprüche

1. Lysin-Acetyltransferase-6 (KAT6)-Inhibitor zur Verwendung in einem Verfahren zur Behandlung von Krebs, wobei das Verfahren umfasst: das Verabreichen einer Menge des Lysin-Acetyltransferase-6 (KAT6)-Inhibitors an einen Patienten, der dessen bedarf, und
a) einer Menge eines Cyclin-abhängigen Kinase-4 (CDK4)-Inhibitors; oder
b) einer Menge eines Antiestrogens; oder
c) einer Menge eines CDK4-Inhibitors und einer Menge eines Antiestrogens;
wobei die Mengen zusammen bei der Behandlung von Krebs therapeutisch wirksam sind;
wobei der Krebs Brustkrebs ist;
wobei der KAT6-Inhibitor 2-Methoxy-*N*-{4-methoxy-6-[(1*H-*pyrazol-1-yl)methyl]-1,2-benzoxazol-3-yl}benzol-1-sulfonamid oder ein pharmazeutisch verträgliches Salz davon ist;
wobei das Antiestrogen Fulvestrant ist; und
wobei der CDK4-Inhibitor entweder 1,5-Anhydro-3-({5-chlor-4-[4-fluor-2-(2-hydroxypropan-2-yl)-1-(propan-2-yl)-1*H-*benzimidazol-6-yl]pyrimidin-2-yl}amino)-2,3-didesoxy-D-threo-pentitol oder ein pharmazeutisch verträgliches Salz davon oder Palbociclib oder ein pharmazeutisch verträgliches Salz davon ist.

2. Lysin-Acetyltransferase-6 (KAT6)-Inhibitor zur Verwendung in einem Verfahren zur Überwindung klinischer Resistenz gegen endokrine Therapie, wobei das Verfahren das Verabreichen einer Menge des KAT6-Inhibitors und einer Menge eines CDK4-Inhibitors an einen Patienten, der dessen bedarf, umfasst, wobei die Mengen zusammen bei der Überwindung klinischer Resistenz gegen endokrine Therapie therapeutisch wirksam sind;
wobei die endokrine Therapie zur Behandlung von Brustkrebs verwendet wird;
wobei der KAT6-Inhibitor 2-Methoxy-*N*-{4-methoxy-6-[(1*H-*pyrazol-1-yl)methyl]-1,2-benzoxazol-3-yl}benzol-1-sulfonamid oder ein pharmazeutisch verträgliches Salz davon ist; und
wobei der CDK4-Inhibitor entweder 1,5-Anhydro-3-({5-chlor-4-[4-fluor-2-(2-hydroxypropan-2-yl)-1-(propan-2-yl)-1*H-*benzimidazol-6-yl]pyrimidin-2-yl}amino)-2,3-didesoxy-D-threo-pentitol oder ein pharmazeutisch verträgliches Salz davon oder Palbociclib oder ein pharmazeutisch verträgliches Salz davon ist.

3. Lysin-Acetyltransferase-6 (KAT6)-Inhibitor zur Verwendung nach Anspruch 1 oder Anspruch 2, wobei der CDK4-Inhibitor 1,5-Anhydro-3-({5-chlor-4-[4-fluor-2-(2-hydroxypropan-2-yl)-1-(propan-2-yl)-1*H*-benzimidazol-6-yl]pyrimidin-2-yl}amino)-2,3-didesoxy-D-*threo*-pentitol oder ein pharmazeutisch verträgliches Salz davon ist.

4. Lysin-Acetyltransferase-6 (KAT6)-Inhibitor zur Verwendung nach Anspruch 1 oder Anspruch 2, wobei der CDK4-Inhibitor Palbociclib oder ein pharmazeutisch verträgliches Salz davon ist.

5. Lysin-Acetyltransferase-6 (KAT6)-Inhibitor zur Verwendung nach einem der Ansprüche 1 bis 4, wobei der Patient ein Mensch ist.

6. Lysin-Acetyltransferase-6 (KAT6)-Inhibitor zur Verwendung nach einem der Ansprüche 1 oder 2, wobei der Brustkrebs lokal fortgeschrittener oder metastatischer Estrogenrezeptorpositiver (ER+) Brustkrebs ist.

7. Lysin-Acetyltransferase-6 (KAT6)-Inhibitor zur Verwendung nach einem der Ansprüche 1 oder 2, wobei der Brustkrebs lokal fortgeschrittener oder metastatischer Estrogenrezeptorpositiver (ER+), menschlicher epidermaler Wachstumsfaktor-Rezeptor-2-negativer (HER2-) Brustkrebs ist.

## Revendications

1. Inhibiteur de lysine acétyltransférase 6 (KAT6) pour utilisation dans un procédé de traitement du cancer, dans lequel ledit procédé comporte l'administration à un patient qui en a besoin d'une quantité dudit inhibiteur de lysine acétyltransférase 6 (KAT6) et de
a) une quantité d'un inhibiteur de kinase 4 dépendante des cyclines (CDK4) ; ou
b) une quantité d'un antiœstrogène ; ou
c) une quantité d'un inhibiteur de CDK4 et une quantité d'un antiœstrogène ;
dans lequel les quantités combinées sont thérapeutiquement efficaces dans le traitement du cancer ;
dans lequel le cancer est un cancer du sein ;
dans lequel l'inhibiteur de KAT6 est le 2-méthoxy-*N*-{4-méthoxy-6-[(1*H*-pyrazol-1-yl)méthyl]-1,2-benzoxazol-3-yl}benzène-1-sulfonamide, ou un sel pharmaceutiquement acceptable de celui-ci ; dans lequel l'antiœstrogène est le fulvestrant ; et
dans lequel l'inhibiteur de CDK4 est soit le 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-didéoxy-D-*thréo*-pentitol, ou un sel pharmaceutiquement acceptable de celui-ci, soit le palbociclib, ou un sel pharmaceutiquement acceptable de celui-ci.

2. Inhibiteur de lysine acétyltransférase 6 (KAT6) pour utilisation dans un procédé permettant de surmonter une résistance clinique à l'endocrinothérapie, dans lequel ledit procédé comporte l'administration à un patient qui en a besoin d'une quantité dudit inhibiteur de KAT6 et d'une quantité d'un inhibiteur de CDK4, dans lequel les quantités combinées sont thérapeutiquement efficaces pour surmonter une résistance clinique à l'endocrinothérapie ;
dans lequel l'endocrinothérapie est utilisée pour traiter le cancer du sein ;
dans lequel l'inhibiteur de KAT6 est le 2-méthoxy-*N*-{4-méthoxy-6-[(1*H*-pyrazol-1-yl)méthyl]-1,2-benzoxazol-3-yl}benzène-1-sulfonamide, ou un sel pharmaceutiquement acceptable de celui-ci ; et
dans lequel l'inhibiteur de CDK4 est soit le 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-didéoxy-D-*thréo*-pentitol, ou un sel pharmaceutiquement acceptable de celui-ci, soit le palbociclib, ou un sel pharmaceutiquement acceptable de celui-ci.

3. Inhibiteur de lysine acétyltransférase 6 (KAT6) pour utilisation selon la revendication 1 ou la revendication 2, dans lequel l'inhibiteur de CDK4 est le 1,5-anhydro-3-({5-chloro-4-[4-fluoro-2-(2-hydroxypropan-2-yl)-1-(propan-2-yl)-1*H*-benzimidazol-6-yl]pyrimidin-2-yl}amino)-2,3-didéoxy-D-thréo-pentitol, ou un sel pharmaceutiquement acceptable de celui-ci.

4. Inhibiteur de lysine acétyltransférase 6 (KAT6) pour utilisation selon la revendication 1 ou la revendication 2, dans lequel l'inhibiteur de CDK4 est le palbociclib, ou un sel pharmaceutiquement acceptable de celui-ci.

5. Inhibiteur de lysine acétyltransférase 6 (KAT6) pour utilisation selon l'une quelconque des revendications 1 à 4, dans lequel le patient est humain.

6. Inhibiteur de lysine acétyltransférase 6 (KAT6) pour utilisation selon l'une quelconque des revendications 1 ou 2, dans lequel le cancer du sein est un cancer du sein localement avancé ou métastatique positif aux récepteurs des œstrogènes (ER+).

7. Inhibiteur de lysine acétyltransférase 6 (KAT6) pour utilisation selon l'une quelconque des revendications 1 ou 2, dans lequel le cancer du sein est un cancer du sein localement avancé ou métastatique positif aux récepteurs des œstrogènes (ER+), négatif aux récepteurs du facteur de croissance épidermique humain 2 (HER2-).
